# EUROPEAN PATENT APPLICATION

(11) **EP 4 414 374 A1**
(43) Date of publication of application: **14.08.2024**
(21) Application number: 22916092.4
(22) Date of filing: 27.12.2022
(51) Int. Cl.: C07K 1/02, C07B 61/00, C07C 227/18, A61K 38/12, A61P 35/00, C07C 229/00, C07K 7/64

(54) **METHOD FOR PRODUCING N-ALKYL AMINO ACID AND PEPTIDE INCLUDING N-ALKYL AMINO ACID**

(30) Priority: 28.12.2021 JP 2021214900; 20.10.2022 JP 2022168608
(71) Applicant: Chugai Seiyaku Kabushiki Kaisha, Kita-ku Tokyo 115-8543 (JP)
(72) Inventor: The designation of the inventor has not yet been filed
(74) Representative: Vossius & Partner Patentanwälte Rechtsanwälte mbB
(86) International application number: PCT/JP2022/048135
(87) International publication number: WO 2023/127869

(57) **Abstract**

Provided is a method for producing an N-monoalkylamino acid or an ester thereof or a peptide containing the N-monoalkylamino acid or an ester thereof, the method comprising an alkylation step of mixing a starting amino acid or an ester thereof or a peptide containing the starting amino acid or an ester thereof, a C₁-C₆ primary alkylating agent or a substituted methyl halide, and a catalyst in a solvent in the presence of hydrogen, wherein the alkylation step is carried out at a pressure of 1 atm or more, and produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.

## Description

### Technical Field

The present invention relates to a method for producing an N-alkylamino acid and a peptide containing the N-alkylamino acid.

### Background Art

In recent years, it has become known that compounds having a molecular weight of more than 500 can contribute to inhibit the interactions at the surface of a target protein, that is, protein-protein interactions, which are difficult to interact by conventional small molecule compounds. These molecules are distinguished from small molecules (having a molecular weight of 500 or less) that have been used as oral drugs, and high molecules (having a molecular weight of more than 100000) used such as antibody pharmaceuticals, and called medium molecular compounds (having a molecular weight of 500 to 2000). Medium molecular compounds are gaining prominence as a new modality that enables drug discovery against tough targets. Among the medium molecular weight compounds, peptide pharmaceuticals have already been on the market in 40 types or more. For example, cyclosporine is used as an immunosuppressive agent (Non Patent Literature 1).

Many of the peptides that are active ingredients in peptide pharmaceuticals are known to contain non-natural amino acids, such as N-alkylamino acids, in their molecules. It is known that peptides having non-natural amino acids, in particular, N-alkylamino acids, have improved metabolic stability and membrane permeability required as active ingredients in pharmaceutical products (Patent Literatures 1 and 2).

To supply a compound as a pharmaceutical product, it is essential to establish for the compound an efficient chemical synthesis method suitable for mass synthesis. In the production of a peptide containing non-natural amino acid in the sequence, especially a peptide containing an N-alkylamino acid, it has been a problem that the yield of the target product is decreased due to the low reactivity of the condensation reaction due to steric hindrance of an alkyl group on the nitrogen atom of the amino group, racemization of the amino acid residue at position α, and the like (Non Patent Literature 2). Meanwhile, for the production of an N-alkylamino acid or a peptide containing an N-alkylamino acid residue by alkylation reaction of the amino group, methods of alkylating an aliphatic primary amino group in the amino acid or the peptide structure under the hydrogen atmosphere using an alkyl aldehyde or alkyl nitrile as an alkylating agent are known (Patent Literature 3, Non Patent Literatures 3 and 4). In addition, methods of converting an aliphatic primary amino group to a secondary amino group by selective alkylation under the hydrogen atmosphere using an alkyl nitrile as an alkylating agent are known (Non Patent Literatures 5 and 6). As an N-alkylation method of a peptide, a method of N-alkylating a primary amine protected by, for example, a 2-nitrobenzenesulfonyl group, with an alkylating agent such as dialkyl sulfate, and then deprotecting it is known (Non Patent Literature 7). A synthetic method of N-alkylating a peptide using a borohydride reagent as a reducing agent is also known (Non Patent Literature 8).

### Citation List

### Patent Literature

[Patent Literature 1] WO 2018/225864
[Patent Literature 2] WO 2020/122182
[Patent Literature 3] WO 2000/015656

### Non Patent Literature

[Non Patent Literature 1] Future Med. Chem., 2009,1,1289-1310.
[Non Patent Literature 2] J. Peptide Res., 2005, 65, 153-166.
[Non Patent Literature 3] J. Med. Chem. 1992, 35, 4195-4204.
[Non Patent Literature 4] J.Org. Chem. 1984, 49, 5269-5271.
[Non Patent Literature 5] Org. Lett. 2004, 6, 4977-4980.
[Non Patent Literature 6] Org. Biomol. Chem. 2012, 10, 293-304.
[Non Patent Literature 7] Acc. Chem. Res. 2008, 41, 1331-1342.
[Non Patent Literature 8] Chem. Sci. 2017, 8, 2717-2722.

### Summary of Invention

### Technical Problem

To efficiently N-alkylate the amino group at the N-terminus of the amino acid or peptide, a synthetic method capable of monoalkylating and converting the aliphatic primary amino group contained in the structure of the amino acid or peptide to a secondary amino group with high selectivity is necessary. Furthermore, the production of a peptide containing an N-alkylamino acid requires a plurality of steps including deprotection of an amino group at the N-terminus, N-alkylation of an amino group at the N-terminus, and then an extension reaction of the amino acid. Thus, an object of the present invention is to provide a method for efficiently producing an N-monoalkylamino acid or a peptide containing an N-monoalkylamino acid residue.

In conventional N-alkylation methods, not only a secondary amine resulting from monoalkylation of the primary amino group of a substrate, but also by-products including dialkylated tertiary amine are often produced. The by-products are sometimes difficult to separate. When both the amino group of the substrate and the alkyl group to be introduced have small bulkiness, it is generally difficult to increase the selectivity of monoalkylation to dialkylation. Non Patent Literatures 3 to 6 and Patent Literature 3 refer to a method of introducing a large sterically hindered alkyl group to an amino group of a large sterically hindered substrate, but not refer to highly selective monoalkylation. In fact, when a method of selectively converting an aliphatic primary amine to a secondary amine under the hydrogen atmosphere using an alkyl nitrile as an alkylating agent was applied to the production of a peptide according to the disclosures of Non Patent Literatures 5 and 6, the reactivity was low due to the influence of the steric hindrance of the amino group, resulting in difficulty to efficiently obtain the target product.

Meanwhile, in the peptide production by liquid-phase methods, an amino acid in which the amino group at the N-terminus is protected by a benzyloxycarbonyl group (also referred to as a Cbz group) is generally used. When an extension reaction of a peptide chain of a sequence containing an N-alkylamino acid is carried out using such a protected amino acid, the reaction conditions such as the liquidity of the solvent or reaction solution may be different in each of the removal step of the Cbz group, the alkylation step of the amino group at the N-terminus, and the extension reaction step of the peptide chain. Furthermore, it requires a plurality of work steps including the post-processing procedure and the isolation procedure of the target product in each step, thus cumbersome. The protecting group and the alkylation reaction of the next step in Non Patent Literature 7 require three steps of introducing a specific protecting group to the amino group at the N-terminus, alkylation, and deprotection, thus the manufacturing method is hardly said to be a practical method. The method of using a borohydride reagent as a reducing agent described in Non Patent Literature 8 merely exemplifies a production of an N-substituted peptide by benzaldehyde having significant steric hindrance. In addition, the hydride reagent such as borohydride reagent is required in the amount equal to or greater than the substrate compound, thus the post-treatment step procedures for the reaction solution containing the excess reagent are cumbersome. When these methods are applied to peptide synthesis, the removal reaction of the protecting group at the N-terminus of the peptide and the alkylation reaction of the amino group at the N-terminus are necessary to carry out each as a separate step due to their difference, thus it is also difficult to carry out the deprotection reaction and the N-alkylation reaction in one-pot.

### Solution to Problem

The present invention relates to a method for producing an N-alkylamino acid and a peptide containing the N-alkylamino acid. The present inventors have aimed to establish a method for efficiently producing an amino acid or an N-alkyl form of a peptide without depending on the bulkiness of the substrate or the alkyl group to be introduced, and have investigated the conditions of the N-alkylation reaction, focusing on alkylating agents, reducing agents, catalysts, and additives. The present inventors have then studied the alkylation by using alkyl nitrile or alkyl aldehyde as the alkylating agent, adding an organic base as an additive in the presence of a transition metal catalyst under the hydrogen atmosphere at normal pressure (1 atm) or more, and have found that the conditions are applicable to the N-alkylation reaction of amino acids as well as the N-alkylation reaction of peptides. Furthermore, the present inventors have found that, when an amino acid or a peptide having a protecting group at the N-terminus that can be removed under hydrogenolysis conditions is applied to the conditions of the present invention, the deprotection reaction and the N-alkylation reaction can be carried out in one-pot. The present inventors have also found that, when an acid is added as an additive in the reaction carried out in one-pot, the N-alkyl product of interest is efficiently obtained.

For example, the present invention provides the following (1) to (35).

(1) A method for producing an N-monoalkylamino acid or an ester thereof or a peptide containing the N-monoalkylamino acid or an ester thereof, the method comprising
   an alkylation step of mixing a starting amino acid or an ester thereof or a peptide containing the starting amino acid or an ester thereof, a C₁-C₆ primary alkylating agent or a substituted methyl halide, and a catalyst in a solvent in the presence of hydrogen,
   wherein the alkylation step is carried out at a pressure of 1 atm or more, and produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.
(1.1) A method for producing an N-monoalkylamino acid or an ester thereof or a peptide containing the N-monoalkylamino acid or an ester thereof, the method comprising:
   an alkylation step of mixing a starting amino acid or an ester thereof or a peptide containing the starting amino acid or an ester thereof, a C₁-C₆ primary alkylating agent or a substituted methyl halide, a hydride reducing agent, and a catalyst in a solvent,
   wherein the alkylation step produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.
(1.2) The method according to (1) or (1.1), wherein the C₁-C₆ primary alkylating agent or the substituted methyl halide is a C₁-C₆ primary alkylating agent.
(2) The method according to any one of (1) to (1.2), wherein the C₁-C₆ primary alkylating agent is C₁-C₅ alkyl nitrile or C₁-C₅ alkyl aldehyde.
(2.1) The method according to (2), wherein the substituted methyl halide is one member selected from the group consisting of methoxymethyl chloride (MOM-Cl), ethoxymethyl chloride (EOM-Cl), 2-methoxyethoxymethyl chloride (NMM-Cl), and 2-(trimethylsilyl)ethoxymethyl chloride (SEM-Cl).
(2.2) The method according to any one of (1.1) to (2.1), wherein the hydride reducing agent is trialkylsilane.
(2.3) The method according to (2.2), wherein the trialkylsilane is triethylsilane.
(3) The method according to any one of (1) to (2.2), wherein the catalyst is a heterogeneous hydrogenation catalyst containing a transition metal.
(4) The method according to (3), wherein the heterogeneous hydrogenation catalyst is a catalyst containing a transition metal selected from the group consisting of Pd, Rh, and Pt.
(5) The method according to (3) or (4), wherein the heterogeneous hydrogenation catalyst is a catalyst selected from the group consisting of Pd-C, Pd(OH)₂-C, Rh-C, and an Adams catalyst.
(6) The method according to any one of (1) to (5), wherein the solvent includes at least one solvent selected from the group consisting of an ether-based solvent, an alcohol-based solvent, and an ester-based solvent.
(7) The method according to any one of (1) to (6), wherein the solvent is selected from the group consisting of an ether-based solvent, an alcohol-based solvent, an ester-based solvent, and a combination thereof.
(8) The method according to any one of (1) to (7), wherein the solvent is an ether-based solvent selected from the group consisting of tetrahydrofuran, 2-methyltetrahydrofuran, dimethyl ether, methyl t-butyl ether, cyclopentyl methyl ether, diisopropyl ether, 4-methyltetrahydropyran, dioxane, and diethyl ether.
(9) The method according to any one of (1) to (7), wherein the solvent is an alcohol-based solvent selected from the group consisting of methanol, ethanol, propanol, butanol, and pentanol.
(10) The method according to any one of (1) to (7), wherein the solvent is an ester-based solvent selected from the group consisting of ethyl acetate, propyl acetate, and butyl acetate.
(11) The method according to any one of (1) to (10), further comprising contacting a reaction mixture in the alkylation step with additional hydrogen.
(12) The method according to any one of (1) to (11), wherein the amino group of the starting amino acid or an ester thereof or a peptide containing the starting amino acid or an ester thereof is attached to a protecting group that can be removed under a hydrogenolysis condition.
(12.1) The method according to (12), comprising the step of removing the protecting group (removal of the protecting group).
(13) The method according to (12) or (12.1), wherein the protecting group is an arylmethyloxycarbonyl group.
(14) The method according to (12) or (13), wherein the removal of the protecting group is carried out in the presence of an additive selected from the group consisting of p-toluenesulfonic acid, methanesulfonic acid, sodium bisulfate, triethylamine hydrochloride, and propylphosphonic acid.
(14.1) The method according to any of (12) or (13), wherein a reaction mixture in the alkylation step further contains a base.
(14.2) The method according to any one of (12) to (14.1), wherein the removal of the protecting group and the alkylation step are carried out in one-pot.
(15) The method according to any one of (1) to (11),
   wherein the amino group of the starting amino acid or an ester thereof is a primary amino group, and
   a reaction mixture in the alkylation step further contains a base.
(16) The method according to (14.1) or (15), wherein the base is a tertiary amine.
(17) The method according to (16), wherein the tertiary amine is selected from the group consisting of 1,8-diazabicyclo[5.4.0]undecene-7, 1,5-diazabicyclo[4.3.0]nonene-5, N-methylmorpholine, 1,4-diazabicyclo[2.2.2]octane, triethylamine, N,N-diisopropylethylamine, pyridine, and collidine.
(18) A method for producing a peptide or an ester thereof, comprising the steps of:
   (a) obtaining an N-monoalkylamino acid or an ester thereof or a peptide containing the N-monoalkylamino acid or an ester thereof in accordance with the method according to any one of (1) to (17); and
   (b) optionally extending one or more amino acids or peptides to the N-monoalkylamino acid or an ester thereof or a peptide containing the N-monoalkylamino acid or an ester thereof by a bond-forming reaction to obtain a peptide or an ester thereof.
(18.1) The method for producing a peptide or an ester thereof according to (18), wherein the steps (a) and (b) are repeated a plurality of times until the desired peptide is obtained.
(18.2) A method for producing a peptide having a cyclic portion composed of at least 4 amino acids or an ester thereof, the method comprising the steps of:
   (c) obtaining a peptide or an ester thereof in accordance with the method according to (18) or (18.1); and
   (d) cyclizing with a group at the C-terminus and a group at the N-terminus of the peptide or an ester thereof to form the cyclic portion.
(18.3) The method according to (18.2), wherein the peptide or an ester thereof in step (d) is a linear peptide or an ester thereof.
(18.4) The method according to (18.3), wherein the linear peptide or an ester thereof is a linear peptide represented by the following formula: 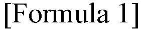 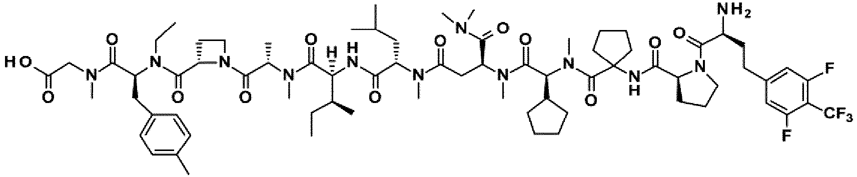 or a salt thereof or a solvate thereof.
(19) The method according to (18.2) or (18.3), wherein the step of forming a cyclic portion is performed by forming a bond between a carboxyl group at the C-terminus and an amino group at the N-terminus of the peptide or an ester thereof.
(20) The method according to any one of (18.2) to (19), wherein
   the cyclic portion is composed of at least 8 amino acids, and
   the peptide having the cyclic portion or an ester thereof is a peptide composed of 8 to 15 amino acids or an ester thereof.
(20.1) A method for producing a peptide containing 5 or more, 6 or more, or 7 or more N-alkylamino acid residues or an ester thereof, the method comprising the method according to any one of (18) to (20).
(20.2) The method according to any one of (18) to (20), wherein the peptide having a cyclic portion or an ester thereof is a peptide having a cyclic portion represented by the following Formula (1): 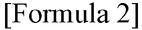 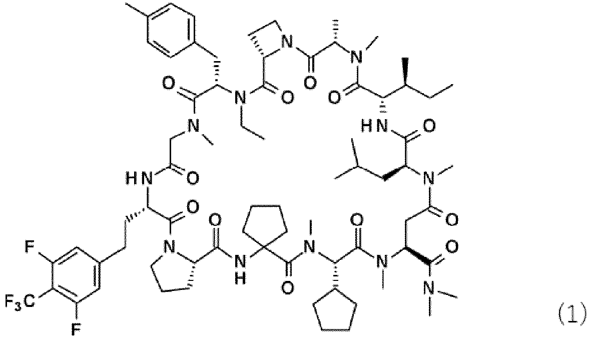 or a salt thereof or a solvate thereof.
(20.3) The method according to (20.2), wherein the peptide having a cyclic portion or a salt thereof or a solvate thereof is a solvate of the peptide having a cyclic portion represented by Formula (1).
(20.4) The method according to (20.2), wherein the peptide having a cyclic portion or a salt thereof or a solvate thereof is a hydrate of the peptide having a cyclic portion represented by Formula (1).
(20.5) The method according to (20.2), wherein the peptide having a cyclic portion or a salt thereof or a solvate thereof is a peptide having a cyclic portion represented by Formula (1).
(20.6) The method according to any one of (18) to (20.5), wherein column chromatography is not used in isolation and/or purification of the peptide having a cyclic portion or a salt thereof or a solvate thereof.
(20.7) The method according to any one of (18) to (20.6), further comprising the step of isolating and/or purifying the peptide having a cyclic portion or a salt thereof or a solvate thereof by crystallization to obtain crystals of the peptide having a cyclic portion or a salt thereof or a solvate thereof.
(20.8) The method according to (20.7), wherein the crystals of the peptide having a cyclic portion or a salt thereof or a solvate thereof is non-solvate crystals or solvate crystals of a peptide having a cyclic portion represented by the following Formula (1): 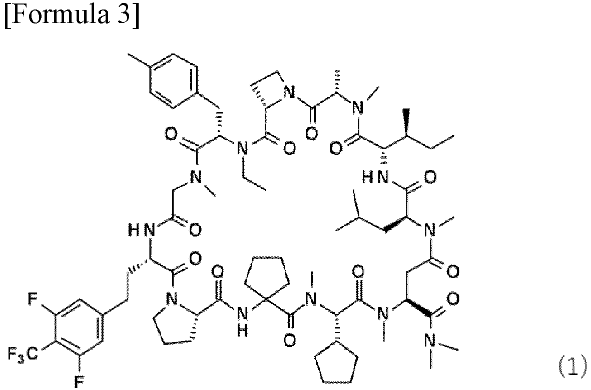
(20.9) The method according to (20.8), wherein the crystals of the peptide having a cyclic portion is solvate crystals.
(20. 10) The method according to (20.9), wherein the solvate crystals of the peptide having a cyclic portion is hydrate crystals.
(21) A method of suppressing a production of a dialkylated compound in an N-monoalkylation reaction of a starting amino acid or an ester thereof or a peptide containing the starting amino acid or an ester thereof, wherein the dialkylated compound is a compound in which an amino group of the starting amino acid or an ester thereof or a peptide containing the starting amino acid or an ester thereof is dialkylated, the method comprising:
   an alkylation step of mixing a starting amino acid or an ester thereof or a peptide containing the starting amino acid or an ester thereof, a C₁-C₆ primary alkylating agent or a substituted methyl halide, and a catalyst in a solvent in the presence of hydrogen,
   wherein the alkylation step is carried out at a pressure of 1 atm or more, and produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.
(21.1) A method of suppressing a production of a dialkylated compound in an N-monoalkylation reaction of a starting amino acid or an ester thereof or a peptide containing the starting amino acid or an ester thereof, wherein the dialkylated compound is a compound in which an amino group of the starting amino acid or an ester thereof or a peptide containing the starting amino acid or an ester thereof is dialkylated, the method comprising:
   an alkylation step of mixing a starting amino acid or an ester thereof or a peptide containing the starting amino acid or an ester thereof, a C₁-C₆ primary alkylating agent or a substituted methyl halide, a hydride reducing agent, and a catalyst in a solvent,
   wherein the alkylation step produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.
(21.2) The method according to (21) or (21.1), wherein the C₁-C₆ primary alkylating agent or the substituted methyl halide is a C₁-C₆ primary alkylating agent.
(22) The method according to any one of (21) to (21.2), further comprising the steps of:
   extending a peptide chain of an N-monoalkylamino acid or an ester thereof or a peptide containing the N-monoalkylamino acid or an ester thereof by a bond-forming reaction; and
   treating the extended peptide with an aqueous acid solution to remove the dialkylated compound.
(23) The method according to any one of (1) to (22), wherein the N-monoalkylamino acid or an ester thereof is a compound represented by Formula C: 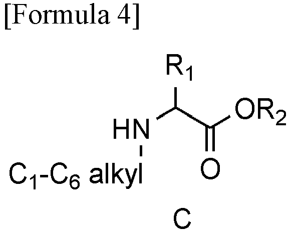 wherein R₁ represents a side chain of amino acid, and R₂ represents a hydrogen atom or a C₁-C₆alkyl group.
(24) The method according to any one of (1) to (11) and (15) to (23), wherein the starting amino acid or an ester thereof is a compound represented by Formula A: 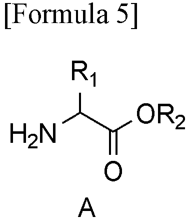 wherein R₁ represents a side chain of amino acid, and R₂ represents a hydrogen atom or a C₁-C₆alkyl group.
(25) The method according to any one of (1) to (14) and (18) to (23), wherein the starting amino acid or an ester thereof is a compound represented by Formula B: 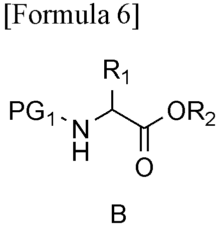 wherein PG₁ is a protecting group of an amino group, R₁ represents a side chain of amino acid, and R₂ represents a hydrogen atom or a C₁-C₆alkyl group.
(26) The method according to any one of (1) to (22), wherein the peptide containing the N-monoalkylamino acid or an ester thereof is a compound represented by Formula F: 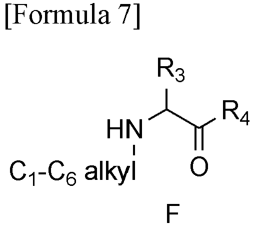 wherein R₃ represents a side chain of amino acid residue and R₄ represents a peptide residue.
(27) The method according to any one of (1) to (11), (15) to (22) and (26), wherein the peptide containing the starting amino acid ((hereinafter also referred to as the "starting peptide") or an ester thereof is a compound represented by Formula D. 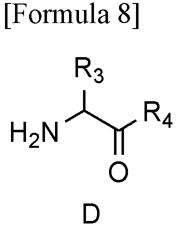 wherein R₃ represents a side chain of amino acid residue, and R₄ represents a peptide residue.
(28) The method according to any one of (1) to (14), (18) to (22), (26) and (27), wherein the peptide containing the starting amino acid is a compound represented by Formula E: 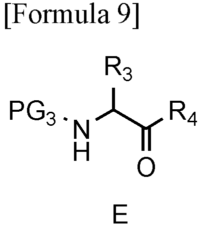 wherein PG₂ is a protecting group of an amino group, R₃ represents a side chain of amino acid residue, and R₄ represents a peptide residue.
(29) The method according to any one of (21) to (26), wherein the C₁-C₆alkyl group of R₂ is a group that is not removed under a hydrogenolysis condition.
(30) The method according to any one of (23) to (25), wherein the C₁-C₆alkyl group of R₂ is selected from a t-butyl group, an n-butyl group, a 1-methylpropyl group, a 2-methylpropyl group, an n-propyl group, an isopropylmethyl group, and an ethyl group.
(31) The method according to any one of (25) and (28) to (30), wherein PG₁ and PG₂ are protecting groups that can be removed under a hydrogenolysis condition.
(32) The method according to any one of (25) and (28) to (31), wherein PG₁ and PG₂ are protecting groups selected from the group consisting of a benzyloxycarbonyl group, a benzyloxymethyl group, and a benzyl group.
(33) The method according to any one of (23) to (32), wherein R₁ and R₃ are not groups that may undergo unintended structural transformation by a condition of the alkylation step.
(34) The method according to any one of (21) to (31), wherein R₁ and R₃ are each independently selected from a hydrogen atom, a C₁-C₆alkyl group, a haloC₁-C₆alkyl group, a C₃-C₆cycloalkyl group, a C₃-C₆cycloalkylC₁-C₆alkyl group, a carboxyC₁-C₆alkyl group, a C₆-C₁₀arylC₁-C₆alkyl group which may have a substituent on the aryl group, a 5- to 10-membered heteroarylC₁-C₆alkyl group which may have a substituent on the heteroaryl group, a 5- to 10-membered heterocyclylC₁-C₆alkyl group which may have a substituent on the heterocyclyl group, a C₃-C₆cycloalkoxyC₁-C₆alkyl group, a haloC₁-C₆alkoxyC₁-C₆alkyl group, a protected aminoC₃-C₆alkyl group, a protected hydroxyC₁-C₆alkyl group, or a C₁-C₆alkoxyC₁-C₆alkyl group.
(35) The method according to any one of (23) to (34), wherein R₁ and R₃ are each independently selected from a hydrogen atom, a C₁-C₆alkyl group, or a C₆-C₁₀arylC₁-C₆alkyl group which may have a substituent on the aryl group.

### Advantageous Effects of Invention

The present invention can produce an N-alkylamino acid and a peptide containing the N-alkylamino acid by a selective N-alkylation reaction. The production method according to one embodiment of the present invention enables reducing costs by simplifying reaction procedures including purification procedures and shortening the processes by one-pot reaction, and enables supplying active ingredients and intermediates thereof as pharmaceuticals inexpensively and in large quantities.

It has also been found that, in particular, the N-alkylation reaction can be applied to the introduction reaction of a primary alkyl group. Furthermore, according to one embodiment of the present invention, the desired N-monoalkylated product can be obtained in one-pot, where the target product has been conventionally obtained through two steps of a removal reaction of the N-terminal protecting group and a subsequent alkylation reaction. In the one-pot reaction of the present invention, the production of diketopiperazine in the deprotection step can be further suppressed by adding an organic acid, enabling obtaining the target product more efficiently.

### Description of Embodiments

Hereinafter, the embodiments for carrying out the present invention are described in detail. However, the present invention is not limited by the embodiments given below.

The "one or more" as used herein means the number of 1 or 2 or more. When the "one or more" is used in a context related to a substituent for a certain group, this term means a number from 1 to the maximum number of substituents accepted by the group. Specific examples of the "one or more" include 1, 2, 3, 4, 5, 6, 7, 8, 9, 10, and/or larger numbers.

As used herein, the "to" representing a range means a range including values of both ends thereof. For example, "A to B" means the range of A or more and B or less.

The term "about" as used herein, when used in combination with a numeric value, means the value range of +10% and -10% of the numeric value.

The term "and/or" as used herein is meant to include every combination of the terms "and" and "or" appropriately combined. Specifically, for example, "A, B, and/or C" includes the following seven variations: (i) A, (ii) B, (iii) C, (iv) A and B, (v) A and C, (vi) B and C, and (vii) A, B, and C.

The "amino acid" as used herein includes a natural amino acid and a non-natural amino acid (sometimes also referred to as an amino acid derivative). The "amino acid" as used herein may mean an amino acid residue. The "natural amino acid" as used herein refers to glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro). Non-natural amino acids (amino acid derivatives) are not particularly limited, and examples thereof include a β-amino acid, a D-type amino acid, an N-substituted amino acid, an α,α-disubstituted amino acid, an amino acid having a side chain different from that of natural amino acids, and a hydroxycarboxylic acid. As the amino acids as used herein, amino acids having any conformation are acceptable. The selection of a side chain of the amino acid is not particularly limited, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, a spiro-bonded cycloalkyl group, and the like. Each of the side chains may have a substituent. The substituent is also not limited, and one or two or more substituents may be freely selected independently from any substituents including, for example, a halogen atom, an O atom, an S atom, an N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group which may be substituted, or oxo, aminocarbonyl, and a halogen atom. In a non-limiting aspect, the amino acid as used herein may be a compound having a carboxyl group and an amino group in the same molecule (even in this case, the amino acid also includes imino acids such as proline and hydroxyproline).

As used herein, the "amino acid residue" constituting the peptide is sometimes referred to simply as an "amino acid".

The "side chain of amino acid" as used herein, in the case of an α-amino acid, means an atomic group bonded to a carbon (a-carbon) to which an amino group and a carboxyl group are bonded. For example, the methyl group of Ala is a side chain of the amino acid. In the case of a β-amino acid, an atomic group attached to an α-carbon and/or a β-carbon can be a side chain of the amino acid, and in the case of a γ-amino acid, an atomic group attached to an α-carbon, a β-carbon, and/or a γ-carbon can be a side chain of the amino acid.

Examples of "halogen" as used herein include a fluorine atom, a chlorine atom, a bromine atom, and an iodine atom.

The "amino acid" as used herein includes all isotopes corresponding to each. The isotope of the "amino acid" is a form in which at least one atom is replaced with an atom having the same atomic number (proton number) and a different mass number (total number of protons and neutrons) at an abundance ratio different from the natural abundance ratio. Examples of the isotope contained in the "amino acid" as used herein include a hydrogen atom, a carbon atom, a nitrogen atom, an oxygen atom, a phosphorus atom, a sulfur atom, a fluorine atom, and a chlorine atom, and they include ²H, ³H; ¹³C, ¹⁴C; ¹⁵N; ¹⁷O, ¹⁸O; ³²P; ³⁵S; ¹⁸F; ³⁶Cl; and the like, respectively. For the compounds as used herein, all the compounds containing any proportions of radioactive or non-radioactive isotopic element are encompassed within the scope of the present invention.

The compound described herein may contain an isotopic atom at a non-natural ratio in one or more atoms that constitute the compounds. The compound in which any atom in the compound is replaced with another isotopic atom having the same atomic number (number of protons) and a different mass number (sum of the numbers of protons and neutrons), thereby the abundance ratio of the isotope is different from the abundance ratio in nature, that is, a compound labeled with an isotopic atom, is also included in the present invention. Examples of the isotopic element contained in the compounds herein include a hydrogen atom, a carbon atom, a nitrogen atom, an oxygen atom, a phosphorus atom, a sulfur atom, a fluorine atom, and a chlorine atom, and they include ²H, ³H; ¹³C, ¹⁴C; ¹⁵N; ¹⁷O, ¹⁸O; ³²P; ³⁵S; ¹⁸F; ³⁶Cl; and the like, respectively. The compound labeled with an isotopic atom is useful as a therapeutic or prophylactic agent, a research reagent (e.g., assay reagent), and a diagnostic agent (e.g., in vivo imaging diagnostic agent). For the compounds as used herein, all the compounds containing any proportions of radioactive or non-radioactive isotopic element are encompassed within the scope of the present invention. The compound labeled with an isotopic atom can be produced in a manner similar to methods for producing unlabeled compounds by using a reagent or a solvent containing a corresponding isotopic atom.

The "N-protected amino acid" as used herein means a natural or non-natural amino acid in which an amino group is protected, and the "N-protected peptide" means a peptide in which an amino group of an amino acid residue at the N-terminus is protected. The peptide may be composed of only natural amino acid residues, only non-natural amino acid residues, or any combination of natural and non-natural amino acid residues.

The "peptide" as used herein is not particularly limited as long as the peptide is a compound to which two or more naturally occurring and/or non-naturally occurring amino acids are linked. The linkages between the amino acid residues may be, for example, linkages of amide bond alone, or may be linkages in which some are linkages of amide bond and the remainder are linkages of bond other than amide bond such as an ester bond, an ether bond, a thioether bond, a sulfoxide bond (-S(=O)-), a sulfone bond (-S(=O)₂-), a disulfide bond, a carbon-carbon bond, or a bond by forming a heterocycle. The groups involved in the linkages of amino acid residues may be groups of the main chain of each amino acid, a group of the main chain of amino acid and a group of a side chain of amino acid, and groups of a side chain of each amino acid. A peptide in which these groups are linked according to the embodiments of the linkages of the amino acid residues above is also included in the "peptide" herein. As used herein, a chain-like "peptide" in which these amino acids are linked is also referred to as a "peptide chain". The number of amino acid residues contained in the "peptide" is preferably 5 to 30 residues, more preferably 8 to 15 residues, and further preferably 9 to 13 residues. The peptide synthesized in the present invention contains preferably at least three N-substituted amino acids, more preferably at least five N-substituted amino acids, in one peptide. These N-substituted amino acids may be present continuously or discontinuously in the peptide. The peptide in the present invention may be linear or cyclic, and is preferably a cyclic peptide compound.

The "main chain of peptide" and the "main chain of cyclic peptide" as used herein means a structure formed by multiple linkages of the "main chain of amino acid" described above by amide bonds.

The compound (including a peptide) described herein can be a salt thereof or a solvate thereof. Examples of the salt of the compound include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salts and potassium salts; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or a base. The solvate as used herein is one in which a compound and a solvent together form a molecular aggregate, and is not particularly limited as long as it is a solvate formed by a solvent acceptable for ingestion along with administration of a medicament. Examples of the solvate include not only solvates formed with a single solvent, such as hydrates, alcohol hydrates (such as ethanolates, methanolates, 1-propanolates, or 2-propanolates), or dimethyl sulfoxide, but also solvates formed with multiple solvents for one molecule of compound, or solvates formed with multiple types of solvents for one molecule of compound. When the solvent is water, the solvate is called as hydrate. The solvate of the compound of the present invention is preferably a hydrate. Specific examples of such a hydrate include mono- to deca-hydrates, preferably mono- to penta-hydrates, and further preferably mono- to tri-hydrates. The solvate of the compound of the present invention includes not only solvates formed with a single solvent such as water, an alcohol (e.g., methanol, ethanol, 1-propanol, and 2-propanol), or dimethylformamide, but also solvates formed with multiple solvents.

When the compound according to the present invention is obtained in a free form, this compound can be converted to a state of its hydrate or solvate in accordance with a routine method. When the compound according to the present invention is obtained in a free form, this compound can be converted to a state of a salt that may be formed by the compound or its hydrate or solvate in accordance with a routine method. Examples of such a compound include hydrates and ethanolates of a compound represented by Formula (1) or a salt thereof. Specifically, examples thereof include, but are not limited to, hemihydrates, monohydrates, dihydrates, trihydrates, tetrahydrates, pentahydrates, hexahydrates, heptahydrates, octahydrates, nonahydrates, decahydrates and monoethanolates of a compound represented by Formula (1), hemihydrates, monohydrates, dihydrates, trihydrates, tetrahydrates, pentahydrates, hexahydrates, heptahydrates, octahydrates, nonahydrates, decahydrates and monoethanolates of sodium salt of a compound represented by Formula (1), and hydrates and ethanolates of hydrochloride of a compound represented by Formula (1). Such a hydrate or a solvate may be produced in a crystal form or a non-crystalline form, and the crystal form is capable of assuming crystal polymorphs. In a method for producing such a hydrate or a solvate, for example, to a compound represented by Formula (1) or a peptide compound described herein, a solvent such as ethanol and/or water is added, and a hydrate or a solvate can be obtained by a routine method involving performing stirring, cooling, concentration, and/or drying, etc.

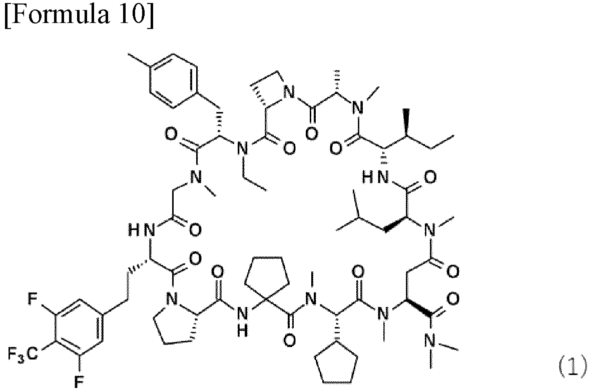

When the compound according to the present invention is obtained as a salt, a hydrate, or a solvate of the compound, this compound can be converted to its free form in accordance with a routine method.

If, in a production of a compound described herein, a defined group undergoes undesired chemical conversion under conditions of the production method, for example, the compounds can be produced by means such as protection or deprotection of the functional group. For the selection and the attachment or removal of the protecting group, reference can be made, for example, to the methods described in "Greene's, "Protective Groups in Organic Synthesis" (5th edition, John Wiley & Sons 2014)", and these methods may be used as appropriate depending on the reaction conditions. It is also possible to change the order of reaction steps, such as steps of introducing a substituent, if necessary.

A first embodiment of the present invention, in one aspect thereof, is a method for producing an N-monoalkylamino acid or an ester thereof, the method comprising an alkylation step of mixing a starting amino acid or an ester thereof, a C₁-C₆ primary alkylating agent or a substituted methyl halide, and a catalyst in a solvent in the presence of hydrogen, wherein the alkylation step is carried out at a pressure of 1 atm or more, and produces an N-monoalkylamino acid or an ester thereof in which a primary alkyl group corresponding to the C₁-C₆ primary alkylating agent or the substituted methyl halide is attached to an amino group of the starting amino acid or an ester thereof.

Another first embodiment of the present invention, in one aspect thereof, is a method for producing an N-monoalkylamino acid or an ester thereof, the method comprising an alkylation step of mixing a starting amino acid or an ester thereof, a C₁-C₆ primary alkylating agent or a substituted methyl halide, a hydride reducing agent, and a catalyst in a solvent, wherein the alkylation step produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.

By the method according to the present embodiment, an N-monoalkylamino acid or an ester, salt or solvate thereof can be efficiently obtained.

The alkylation step according to the present embodiment encompasses a method of selectively N-monoalkylating an amino acid represented by Formula A or an ester thereof (also referred to as starting amino acid A) to obtain an N-monoalkylamino acid represented by Formula C or an ester thereof; and a method of performing a deprotection reaction of an N-protected amino acid represented by Formula B or an ester thereof (also referred to as starting amino acid B) and an N-alkylation reaction in one-pot to obtain an N-monoalkylamino acid represented by Formula C or an ester thereof. Hereinafter, an overview of the method according to the present embodiment is described.

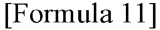

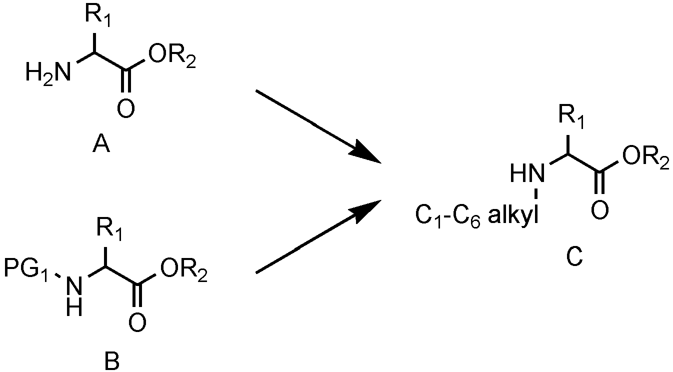

The starting amino acids A and B may be used in the form of a free form, or may be used in the form of a corresponding salt or solvate.

In the chemical formula described above, R₁ represents a side chain of amino acid; PG₁ represents a protecting group of an amino group; and R₂ represents a hydrogen atom or a protecting group of a carboxyl group. The protecting group of the carboxyl group is, for example, a C₁-C₆alkyl group. In the above reaction formula, starting amino acids A and B are described in the form of α-amino acids for convenience, but can be β-amino acids or γ-amino acids. In the reaction formula above, the side chain R₁ of the amino acid preferably does not have a functional group that may undergo unintended structural transformation by alkylation reaction or reduction reaction depending on the conditions of the alkylation step. When R₁ has a functional group that may undergo unintended structural transformation due to the conditions of the alkylation step, the target compound can be produced by introducing a protecting group to the functional group in advance and then carrying out the alkylation step.

R₁ is, for example, selected from a hydrogen atom, a C₁-C₆alkyl group, a haloC₁-C₆alkyl group, a C₃-C₆cycloalkyl group, a C₃-C₆cycloalkylC₁-C₆alkyl group, a carboxyC₁-C₆alkyl group, a C₆-C₁₀arylC₁-C₆alkyl group which may have a substituent on the aryl, a 5- to 10-membered heteroarylC₁-C₆alkyl group which may have a substituent on the heteroaryl, a 5- to 10-membered heterocyclylC₁-C₆alkyl group which may have a substituent on the heterocyclyl, a C₃-C₆cycloalkoxyC₁-C₆alkyl group, a haloC₁-C₆alkoxyC₁-C₆alkyl group, a protected aminoC₃-C₆alkyl group, a protected hydroxyC₁-C₆alkyl group, or a C₁-C₆alkoxyC₁-C₆alkyl group.

The "alkyl" as used herein is a monovalent group derived from an aliphatic hydrocarbon by removing any one hydrogen atom, and has a subset of hydrocarbyl or hydrocarbon group structures that do not contain a hetero atom (which is an atom other than carbon and hydrogen atoms) or an unsaturated carbon-carbon bond, and contain hydrogen and carbon atoms in the backbone. The alkyl includes not only a linear form but also a branched form. Specific examples of C₁-C₆alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

Examples of the amino acids in which a side chain (R₁ or R₃) of an amino acid is a C₁-C₆alkyl group include alanine (Ala), isoleucine (Ile), leucine (Leu), valine (Val), 2-aminobutanoic acid (Abu), norvaline (Nva), norleucine (Nle), and tert-leucine (Tle).

The "haloalkyl" as used herein means a group in which one or more hydrogen of the "alkyl" defined above are replaced with halogen. As haloalkyl, haloC₁-C₆alkyl is preferred, and fluoroC₁-C₆alkyl is more preferred. Specific examples of haloC₁-C₆alkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

Examples of the amino acids in which the side chain of the amino acid (R₁ or R₃) is a haloC₁-C₆alkyl group include 5-difluoronorvaline (Nva(5-F2)) and 2-amino-4-trifluorobutanoic acid (Abu(4-F3)).

The "cycloalkyl" as used herein means a saturated or partially saturated cyclic monovalent aliphatic hydrocarbon group and includes a monocyclic ring, a bicyclo ring, and a spiro ring. As cycloalkyl, C₃-C₆cycloalkyl is preferred. Specific examples of C₃-C₆cycloalkyl include cyclopropyl, cyclobutyl, cyclopentyl, and cyclohexyl.

Examples of the amino acids in which the side chain of the amino acid (R₁ or R₃) is a C₃-C₆cycloalkyl group include α-cyclopropylglycine (Gly(cPr)), α-cyclobutylglycine (Gly(cBu)), α-cyclopentylglycine (Gly(cPent)), and α-cyclohexylglycine (Chg).

The "cycloalkylalkyl" as used herein means a group in which one or more hydrogen of the "alkyl" defined above are replaced with the "cycloalkyl" as defined above. Specific examples of C₃-C₆cycloalkylC₁-C₆alkyl include cyclopropylmethyl, cyclobutylmethyl, cyclopentylmethyl, and cyclohexylmethyl.

The "carboxyalkyl" as used herein means a group in which one or more hydrogen of the "alkyl" defined above are replaced with carboxy. Specific examples of carboxyC₁-C₆alkyl include carboxymethyl.

The "aryl" as used herein means a monovalent aromatic hydrocarbon ring and an aromatic hydrocarbon ring group. As aryl, C₆-C₁₀aryl is preferred. Specific examples of aryl include phenyl and naphthyl (e.g., 1-naphthyl and 2-naphthyl).

The "arylalkyl (aralkyl)" as used herein means a group in which at least one hydrogen atom of the "alkyl" defined above are replaced with the "aryl" as defined above. As arylalkyl, C₆-C₁₀arylC₁-C₆alkyl is preferred. Specific examples of C₆-C₁₀arylC₁-C₆alkyl include benzyl, phenethyl, and 3-phenylpropyl.

Examples of the amino acids in which a side chain of the amino acid (R₁ or R₃) is a C₆-C₁₀arylC₁-C₆alkyl group that may have a substituent on the aryl include phenylalanine (Phe), 4-methylphenylalanine (Phe(4-Me)), and 4-trifluoromethyl-3,5-difluorohomophenylalanine (Hph(4-CF3-35-F2)).

The "heteroaryl" as used herein means an aromatic cyclic monovalent group containing a carbon atom as well as 1 to 5 heteroatoms and an aromatic heterocyclic group. The ring may be a monocyclic ring or a condensed ring with another ring and may be partially saturated. The number of atoms constituting the ring of heteroaryl is preferably 5 to 10 (5- to 10-membered heteroaryl), more preferably 5 to 7 (5- to 7-membered heteroaryl). Specific examples of heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, benzotriazolyl, indolyl, isoindolyl, indazolyl, azaindolyl, quinolyl, isoquinolyl, cinnolinyl, quinazolinyl, quinoxalinyl, benzodioxolyl, indolizinyl, and imidazopyridyl, pyrazolopyridyl, imidazopyridyl, triazolopyridyl, pyrrolopyrazinyl, and flopyridyl.

The "heteroarylalkyl" as used herein means a group in which at least one hydrogen atom of the "alkyl" defined above is replaced with the "heteroaryl" as defined above. As the heteroaryl alkyl, 5- to 10-membered heteroarylC₁-C₆alkyl is preferred, and 5- to 10-membered heteroarylC₁-C₂alkyl is more preferred. Specific examples of 5- to 10-membered heteroarylC₁-C₆alkyl include 3-thienylmethyl, 4-thiazolylmethyl, 2-pyridylmethyl, 3-pyridylmethyl, 4-pyridylmethyl, 2-(2-pyridyl)ethyl, 2-(3-pyridyl)ethyl, 2-(4-pyridyl)ethyl, 2-(6-quinolyl)ethyl, 2-(7-quinolyl)ethyl, 2-(6-indolyl)ethyl, 2-(5-indolyl)ethyl, and 2-(5-benzofuranyl)ethyl.

Examples of the amino acids in which a side chain of the amino acid (R¹ or R³) is a 5- to 10-membered heteroaryl C₁-C₆alkyl group that may have a substituent on the heteroaryl include 2-amino-4-(pyridine-2-yl) -butanoic acid (Abu(4-Pyr)) and 3-(6-trifluoromethylpyridine-3-yl)alanine (Ala(3-Pyr-4-CF3)).

The "heterocyclyl" as used herein means a non-aromatic, cyclic, monovalent group containing 1 to 5 heteroatoms in addition to carbon atoms, and a heterocyclic group. The heterocyclyl may be a saturated heterocyclic ring or have a double and/or triple bond in the ring. A carbon atom in the ring may form carbonyl through oxidation, and the ring may be a monocyclic ring or a condensed ring. In the case of a condensed ring, the condensed ring may be formed with an aromatic ring such as a benzene ring, a pyridine ring, or a pyrimidine ring. The condensed ring may be formed with a saturated cycloaliphatic ring such as a cyclopentane ring or a cyclohexane ring, or a saturated heterocyclic ring such as a tetrahydropyran ring, a dioxane ring, or a pyrrolidine ring.

The number of atoms constituting the ring of heterocyclyl is preferably 4 to 10 (4- to 10-membered heterocyclyl), more preferably 4 to 7 (4- to 7-membered heterocyclyl). Specific examples of heterocyclyl include azetidinyl, oxoazetidinyl, oxiranyl, oxetanyl, azetidinyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, oxopyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, oxazolidone, benzodioxanyl, benzoxazolyl, dioxolanyl, dioxanyl, tetrahydropyrrole [1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, 2-oxaspiro[3.3]heptyl, 6,7-dihydro-pyrrolo[1,2-a]imidazolyl, 4,5,6,7-tetrahydropyrazolo[1,5-a]pyrazinyl, azepanyl, dioxepanyl, and 5,9-dioxaspiro[3.5]nonanyl.

The "heterocyclylalkyl" as used herein means a group in which one or more hydrogen of the "alkyl" defined above are replaced with the "heterocyclyl" as defined above. As heterocyclylalkyl, 5- to 10-membered heterocyclylC₁-C₆alkyl is preferred, 4- to 7-membered heterocyclylC₁-C₆alkyl is more preferred, and 4- to 7-membered heterocyclylC₁-C₂alkyl is further preferred. Specific examples of 5- to 10-membered heterocyclylC₁-C₆alkyl include 2-(tetrahydro-2H-pyran-4-yl)ethyl, 2-(azetidin-3-yl) ethyl, 4-(oxolan-2-ylmethyl)piperazin-1-yl, 2-(1-piperidyl)ethyl, and 3 -(1 -piperidyl)propyl.

The "cycloalkoxy" as used herein means an oxy group to which the "cycloalkyl" as defined above is attached. As cycloalkoxy, C₃-C₈cycloalkoxy is preferred. Specific examples of cycloalkoxy include cyclopropoxy, cyclobutoxy, and cyclopentyloxy.

The "cycloalkoxyalkyl" as used herein means a group in which one or more hydrogen of the "alkyl" defined above are replaced with the "cycloalkoxyl" as defined above. As cycloalkoxyalkyl, C₃-C₈cycloalkoxyC₁-C₆alkyl is preferred, C₃-C₆cycloalkoxyCi-Cealkyl is more preferred, and C₃-C₆cycloalkoxyC₁-C₂alkyl is more preferred. Specific examples of C₃-C₆cycloalkoxyC₁-C₆alkyl include cyclopropoxymethyl and cyclobutoxymethyl.

The "alkoxy" as used herein means an oxy group to which the "alkyl" as defined above is attached. As alkoxy, C₁-C₆alkoxy is preferred. Specific examples of alkoxy include methoxy, ethoxy, 1-propoxy, 2-propoxy, n-butoxy, i-butoxy, s-butoxy, t-butoxy, pentyloxy, and 3-methylbutoxy.

The "alkoxyalkyl" as used herein means a group in which one or more hydrogen of the "alkyl" defined above are replaced with the "alkoxy" as defined above. As alkoxyalkyl, C₁-C₆alkoxyC₁-C₆alkyl is preferred, and C₁-C₆alkoxyC₁-C₂alkyl is more preferred. Specific examples of C₁-C₆alkoxyC₁-C₆alkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxy ethyl, 2-methoxyethyl, and 2-ethoxyethyl.

The "haloalkoxy" as used herein means a group in which one or more hydrogen of the "alkoxy" defined above are replaced with halogen. As haloalkoxy, haloC₁-C₆haloalkoxy is preferred, and fluoroC₁-C₆haloalkoxy is more preferred.

The "haloalkoxyalkyl" as used herein means a group in which one or more hydrogen of the "alkyl" defined above are replaced with the "haloalkoxy" as defined above. As haloalkoxyalkyl, haloC₁-C₆alkoxyC₁-C₆alkyl is preferred. Specific examples of haloC₁-C₆alkoxyC₁-C₆alkyl include difluoromethoxymethyl, trifluoromethoxymethyl, 2,2-difluoroethoxymethyl, 2,2,2-trifluoroethoxymethyl, 3,3-difluoropropoxymethyl, 4,4-difluorobutoxymethyl, and 5,5-difluoropentoxymethyl.

The aryl of C₆-C₁₀arylC₁-C₆alkyl, the heteroaryl of the 5- to 10-membered heteroarylC₁-C₆alkyl, and the heterocyclyl of the 5- to 10-membered heterocyclylC₁-C₆alkyl may further be substituted with a substituent.

As used herein, "may be substituted" means that a group is optionally substituted by any substituent. Furthermore, a substituent may be added to each of the substituents. Such a substituent is not limited and can be one or two or more substituents each independently freely selected from any substituents including a halogen atom, an oxygen atom, a sulfur atom, a nitrogen atom, a boron atom, a silicon atom, or a phosphorus atom.

Examples of the substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

The "aminoalkyl" as used herein means a group in which one or more hydrogen of the "alkyl" defined above are replaced with the "amino" as defined above. As aminoalkyl, aminoC₃-C₆alkyl is preferred. Specific examples of aminoalkyl include aminomethyl, aminoethyl, 4-aminobutyl, methylaminomethyl, dimethylaminomethyl, methylaminoethyl, and dimethylaminoethyl.

The "hydroxyalkyl" as used herein means a group in which one or more hydrogen atoms of the "alkyl" defined above are replaced with hydroxy group. As hydroxyalkyl, hydroxyC₁-C₆alkyl is preferred. Specific examples of hydroxyC₁-C₆alkyl include hydroxymethyl, 1-hydroxyethyl, 2-hydroxyethyl, 2-hydroxy-2-methylpropyl, and 5-hydroxypentyl.

Examples of the "halogen-derived substituent" as used herein include fluoro (-F), chloro (-Cl), bromo (-Br), and iodo (-I).

Examples of the "O-atom-derived substituent" as used herein include hydroxy (-OH), oxy (-OR), carbonyl (-C(=O)-R), carboxy (-CO₂H), oxycarbonyl (-C=O-OR), carbonyloxy (-O-C=O-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino (-NH-SO₂-R), aminosulfonyl (-SO₂-NHR), sulfamoylamino (-NH-SO₂-NHR), thiocarboxy (-C(=O)-SH), and carboxylcarbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Additional examples thereof include groups in which the H atom bonded to the N atom in -C(=O)-NHR is further substituted with alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples thereof include groups in which the H atom bonded to the N atom in -NH-C(=O)-R is further substituted with alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples thereof include groups in which the H atom bonded to the N atom in -NH-C(=O)-OR is further substituted with alkyl, cycloalkyl, alkenyl, alkynyl, aryl, heteroaryl, or aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples thereof include groups in which the H atom bonded to the N atom in -NH-SO₂-R is further substituted with alkyl, cycloalkyl, alkenyl, alkynyl, aryl, heteroaryl, or aralkyl.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples thereof include groups in which the H atom bonded to the N atom in -SO₂-NHR is further substituted with alkyl, cycloalkyl, alkenyl, alkynyl, aryl, heteroaryl, or aralkyl.

Examples of sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in -NH-SO₂-NHR may be substituted by substituents each independently selected from the group consisting of alkyl, cycloalkyl, alkenyl, alkynyl, aryl, heteroaryl, and aralkyl, and these two substituents may form a ring.

Examples of the "nitrogen atom-derived substituent" as used herein include azido (-N₃, also referred to as "azide group"), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH-C(=NH)-NH₂), substituted guanidino (-NR-C(=NR‴)-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

Examples of the tertiary amino (-NR(R')) include an amino group having any two substituents each independently selected from alkyl, cycloalkyl, alkenyl, alkynyl, aryl, heteroaryl, aralkyl, and the like, such as alkyl(aralkyl)amino, and the any two substituents may form a ring.

Examples of the substituted amidino (-C(=NR)-NR'R") include groups in which three substituents R, R', and R" on the N atom are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R, R', R", and R‴ are each independently selected from alkyl, cycloalkyl, alkenyl, alkynyl, aryl, heteroaryl, and aralkyl, and groups in which these substituents form a ring.

Examples of aminocarbonylamino (-NR-C(=O)-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

Examples of the "sulfur atom-derived substituent" as used herein include thiol (-SH), thio (-S-R), sulfinyl (-S(=O)-R), sulfonyl (-S(O)₂-R), sulfo (-SO₃H), and pentafluorosulfanyl (-SF₅).

Examples of thio (-S-R) that can be selected include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-S(=O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of the "boron atom-derived substituent" as used herein include boryl(-BR(R')), dioxyboryl(-B(OR)(OR')), and trifluoroborate salts (-BF₃⁻). Specific examples include a "boron atom-derived substituent" in which the two substituents R and R' are each independently selected from alkyl, cycloalkyl, alkenyl, alkynyl, aryl, heteroaryl, and aralkyl, or a "boron atom-derived substituent" in which these two substituents R and R' are taken together with the atoms to which R and R' are each attached to form a ring, that is a cyclic boryl group.

Preferred examples of the "boron atom-derived substituent" include a cyclic boryl group.

Specific examples of the cyclic boryl group include a pinacolatoboryl group, a neopentanediolatoboryl group, a catecholatoboryl group, and a 9-borabicyclo[3.3.1]nonan-9-yl group.

As used herein, "may be protected" means that a group is optionally protected by any protecting group.

Examples of the "protecting group for amino group" as used herein include a carbamate-type protecting group, an amide-type protecting group, an aryl sulfonamide-type protecting group, an alkyl amine-type protecting group, and an imide-type protecting group. Specific examples thereof include a 9-fluorenylmethoxycarbonyl (Fmoc) group, a t-butoxycarbonyl (Boc) group, an allyloxycarbonyl (Alloc) group, a benzyloxycarbonyl (Cbz) group, a triethylsilyloxycarbonyl (Teoc) group, a trifluoroacetyl group, a pentafluoropropionyl group, a phthaloyl group, a benzenesulfonyl group, a tosyl group, a nosyl group, a dinitronosyl group, a t-butyl group, a trityl group, a cumyl group, a benzylidene group, a 4-methoxybenzylidene group, and a diphenylmethylidene group.

Examples of the "protecting group for hydroxy" as used herein include an alkyl ether type protecting group, an aralkyl ether type protecting group, a silyl ether type protecting group, and a carbonate ester type protecting group. Specific examples of the protecting group for hydroxy include a methoxymethyl group, a benzyloxymethyl group, a tetrahydropyranyl group, a t-butyl group, an allyl group, a 2,2,2-trichloroethyl group, a benzyl group, a 4-methoxybenzyl group, a trimethylsilyl group, a triethylsilyl group, a triisopropylsilyl group, a t-butyldimethylsilyl group, a t-butyldiphenylsilyl group, a methoxycarbonyl group, a 9-fluorenylmethoxycarbonyl (Fmoc) group, and a 2,2,2-trichloroethoxycarbonyl group.

The selection and the attachment or removal of the protecting group can be carried out as appropriate by those skilled in the art. For the selection and the attachment or removal of the protecting group, reference can be made, for example, to the methods described in "Greene's, "Protective Groups in Organic Synthesis" (5th edition, John Wiley & Sons 2014)", and these methods may be employed as appropriate depending on the reaction conditions.

The structural transformation reaction of the compound can be carried out as appropriate by those skilled in the art. For example, reference can be made to the methods described in March's Advanced Organic Chemistry: Reactions, Mechanisms, and Structure (8th ed., John Wiley & Sons, Inc., 2019) and R. C. Laroch, Comprehensive Organic Transformations (3rd ed., John Wiley & Sons, Inc., 2018), and these methods may be employed as appropriate depending on the reaction conditions.

The C₁-C₆ primary alkylating agent may be an alkylating agent having 1 to 6 carbon atoms and in which a formyl or cyano group is attached to a C₁-C₅alkyl group, and preferred examples thereof include C₁-C₅aldehyde or C₁-C₅alkyl nitrile. Specific examples of Ci-Csaldehyde include acetaldehyde, propanal, 1-butanal, 1-pentanal, 3-methylbutanal, 1-hexanal, 4-methylpentanal, and 3,3-dimethylbutanal. Specific examples of C₁-C₅alkyl nitrile include acetonitrile, propionitrile, n-butyronitrile, n-pentyl nitrile (1-cyanopentane), 3-methylbutyronitrile, valeronitrile (1-cyanobutane), and isovaleronitrile (1-cyano-2-methylpropane).

The "substituted methyl halide" as used herein means a methyl halide (halogenated methyl) in which one hydrogen atom on carbon of a methyl halide is replaced with another atom or functional group. Examples of the substituted methyl halide include alkoxymethyl halide, alkoxyalkoxymethyl halide, and trialkylsilylalkoxymethyl halide. Preferably, examples thereof include substituted methyl chloride. Specific examples of the substituted methyl chloride include MOM-Cl (methoxymethyl chloride), EOM-Cl (ethoxymethyl chloride), MEM-Cl (2-methoxyethoxymethyl chloride), and SEM-Cl (2-(trimethylsilyl)ethoxymethyl chloride).

The amount of the C₁-C₆ primary alkylating agent or the substituted methyl halide used may be in the range of 1.0 to 20.0 moles, the range of 1.5 to 15.0 moles, the range of 2.0 to 10.0 moles, or the range of 2.5 to 5.0 moles per 1 mole of the starting amino acid or an ester thereof.

The hydride reducing agent may be any agent that donates a hydride (H⁻) to a substrate and reduces a carbonyl group, a nitrile group, or the like. The hydride reducing agent is preferably a hydride reducing agent containing silicon, more preferably trialkylsilane, and most preferably triethylsilane.

The amount of the hydride reducing agent used may be in the range of 1.0 to 20.0 moles, the range of 1.0 to 15.0 moles, the range of 1.0 to 10.0 moles, or the range of 1.2 to 5.0 moles per 1 mole of the starting amino acid or an ester thereof or the starting peptide or an ester thereof.

The catalyst may be one that promotes the alkylation of the primary amino group and improves the reaction rate. The catalyst is preferably a heterogeneous hydrogenation catalyst containing a transition metal, and more preferably the transition metal is supported on a suitable carrier. The transition metal preferably includes at least one selected from the group consisting of Pd, Rh, and Pt. Specific examples of the catalyst containing a transition metal supported on a carrier include palladium carbon (Pd-C), palladium carbon hydroxide (Pd(OH)₂-C), rhodium carbon (Rh-C), and an Adams catalyst. When the catalyst contains a transition metal supported on a carrier, the handling is easier and the reaction can be carried out under more convenient conditions.

The amount of the catalyst used may be in the range of 0.001 to 0.5 mole, the range of 0.005 to 0.4 mole, the range of 0.01 to 0.4 mole, or the range of 0.03 to 0.1 mole per 1 mole of the starting amino acid or an ester thereof, or the starting peptide or an ester thereof.

The above steps are carried out in a sealed reaction vessel. In the above step, the gas phase in the reaction vessel may be composed only of hydrogen gas or may contain an inert gas to an extent that does not suppress the desired reaction. The pressure (partial pressure) of the hydrogen gas in the reaction vessel may be in the range of 1 atm or more, 1 atm or more and 10 atm or less, 1 atm or more and 7 atm or less, 1 atm or more and 6 atm or less, or 1 atm or more and 5 atm or less. When the reaction is carried out under a pressure of about 1 atm, it is well known that the reaction is performed in the manner in which, for example, a rubber or vinyl balloon is mounted to a reaction vessel and the air in the balloon is replaced with hydrogen gas. In this step, when the reaction solution is stirred vigorously, the contact frequency of the reaction solution with hydrogen gas in the gas phase are increased and the reaction rate may be improved.

Additional hydrogen gas may be contacted with the mixture obtained by bringing the starting amino acid or an ester thereof, a C₁-C₆ primary alkylating agent or a substituted methyl halide, a catalyst, and hydrogen into contact with each other in a solvent in advance. The method of contacting the additional hydrogen gas with the reaction mixture can be performed by adding additional hydrogen to the reaction vessel by a method of feeding and adding hydrogen to the reaction vessel, a method of adding hydrogen to the reaction vessel after degassing the reaction vessel under reduced pressure, a method of performing nitrogen replacement in the reaction vessel after degassing the reaction vessel under reduced pressure, and performing hydrogen replacement in the reaction vessel after degassing the reaction vessel under reduced pressure. The number of times of contacting the additional hydrogen gas can be in the range of 1 to 10 times, 1 to 5 times, 1 to 3 times, depending on the progress of the reaction. The time from the start of the reaction to the contact of additional hydrogen gas can be in the range of 10 minutes to 5 hours, the range of 20 minutes to 3 hours, or the range of 10 minutes to 2 hours. For contacting the additional hydrogen gas, additional reagents may be added to efficiently control the reaction when the reaction vessel is opened. Examples of the additional reagents include a C₁-C₆ primary alkylating agent or a substituted methyl halide, a catalyst, a solvent, an acid, and a base.

A hydride reducing agent can be used as a replacement for hydrogen gas. Depending on the progress of the reaction, an additional hydride reducing agent can be added to the reaction vessel.

The solvent includes at least one solvent selected from the group consisting of an ether-based solvent, an alcohol-based solvent, and an ester-based solvent. The solvent is preferably selected from the group consisting of an ether-based solvent, an alcohol-based solvent, an ester-based solvent, and a combination thereof.

Examples of the ether-based solvent include tetrahydrofuran (THF), 2-methyltetrahydrofuran, dimethoxyethane (DME), methyl t-butyl ether (MTBE), cyclopentylmethyl ether (CPME), diisopropyl ether (IPE), 4-methyltetrahydropyran, dioxane, diethylether, and a combination thereof. Examples of the alcohol-based solvent include methanol, ethanol, propanol, isopropanol, 1-butanol, 2-butanol, 1-pentanol, 2-pentanol, 3-pentanol, and a combination thereof. Examples of the ester-based solvent include ethyl acetate, n-propyl acetate, butyl acetate, and a combination thereof. In this step, the above solvents may be appropriately combined and may be used in combination at any ratio.

The amount of the solvent used may be in the range of 1 to 100 mL, the range of 3 to 75 mL, the range of 5 to 50 mL, or the range of 7 to 25 mL per 1 mole of the starting amino acid or an ester thereof.

When the starting amino acid A is used as the raw material in the alkylation step, it is preferred that a base is added to the reaction solution. The addition of a base to the reaction solution can reduce the proportions of dialkylated compounds and by-products and further improve the monoalkylation selectivity. When the starting amino acid B is used as the raw material, it is preferred that a base is added to the reaction solution in the N-alkylation step after deprotection reaction.

The base may be an organic base or an inorganic base. The base is preferably a tertiary amine, more preferably 1,8-diazabicyclo[5.4.0]undecene-7 (DBU), 1,5-diazabicyclo[4.3.0]nonene-5 (DBN), N-methylmorpholine (NMM), 1,4-diazabicyclo[2.2.2]octane (DABCO), triethylamine (TEA), N,N-diisopropylethylamine (DIPEA), pyridine, and collidine.

The amount of base used may be in the range of 0.01 to 20.0 moles, the range of 0.03 to 15.0 moles, the range of 0.05 to 10.0 moles, or the range of 0.7 to 5.0 moles per 1 mole of the starting amino acid or an ester thereof. When the amount of base used is within the above range, the effect of improving the monoalkylation selectivity is more pronounced.

When the starting amino acid B is used as the raw material in the alkylation step, it is preferred that an acid is added to the reaction solution. The addition of an acid to the reaction solution can reduce the proportion of byproducts, and the target N-monoalkylamino acid or an ester thereof (C) can be obtained more efficiently.

The acid may be any one capable of efficiently removing a protecting group attached to the primary amino group. Examples of the acid include p-toluenesulfonic acid (TsOH), methanesulfonic acid (MsOH), sodium bisulfate, triethylamine hydrochloride, and propyl phosphonic acid. The acid may also be used in the form of a hydrate or any solution.

The amount of acid used may be 0.1 to 10.0 moles, 0.3 to 7.0 moles, 0.5 to 5.0 moles, or 0.7 to 3.0 moles per 1 mole of the starting amino acid or an ester thereof.

The reaction temperature can be in the range of -40°C to near the boiling point of the solvent, or the range of -20°C to 50°C, or the range of 0°C to 30°C.

The reaction time can be in the range of 5 minutes to 72 hours, the range of 10 minutes to 48 hours, or the range of 10 minutes to 24 hours.

After the alkylation step, the obtained N-monoalkylamino acid or an ester thereof can also be converted to the corresponding salt or solvate in a manner well known to those skilled in the art.

A second embodiment of the present invention, in one aspect, is a method for producing a peptide containing an N-monoalkylamino acid residue or an ester thereof, the method comprising an alkylation step of mixing a peptide containing a starting amino acid residue or an ester thereof, a C₁-C₆ primary alkylating agent or a substituted methyl halide, and a catalyst in a solvent in the presence of hydrogen, wherein the alkylation step is carried out at a pressure of 1 atm or more, and produces a peptide containing an N-monoalkylamino acid residue or an ester thereof in which a primary alkyl group corresponding to the C₁-C₆ primary alkylating agent or a substituted methyl halide is attached to an amino group of the starting amino acid residue.

Another second embodiment of the present invention is, in one stage thereof, a method for producing a peptide containing an N-monoalkylamino acid or an ester thereof, the method comprising an alkylation step of mixing a peptide containing a starting amino acid or an ester thereof, a C₁-C₆ primary alkylating agent or a substituted methyl halide, a hydride reducing agent, and a catalyst in a solvent, wherein the alkylation step produces a peptide containing an N-monoalkylamino acid or an ester thereof in which a primary alkyl group corresponding to the C₁-C₆ primary alkylating agent or the substituted methyl halide is attached to an amino group of the starting amino acid residue.

The alkylation step according to the present embodiment encompasses: a method of selectively N-monoalkylating a peptide represented by Formula D or an ester thereof (also referred to as starting peptide D) to obtain a peptide containing an N-monoalkylamino acid residue represented by Formula F or an ester thereof, and a method of performing a deprotection reaction of an N-protected peptide represented by Formula E or an ester thereof (also referred to as starting peptide E) and an N-alkylation reaction in one-pot to obtain a peptide represented by Formula F or an ester thereof. Hereinafter, an overview of the method according to the present embodiment is described.

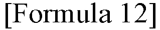

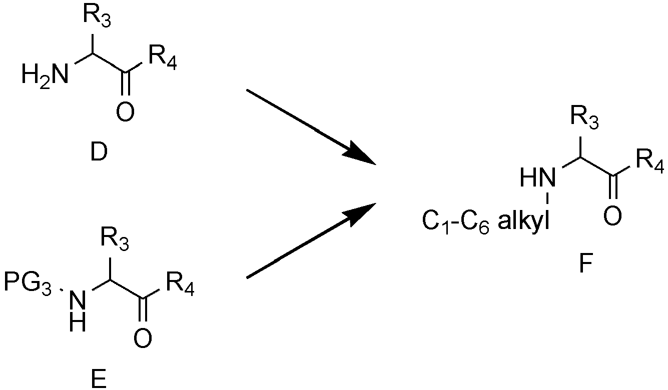

In the above chemical formula, R₃ represents a side chain of amino acid residue at the N-terminus of a peptide, PG₂ represents a protecting group of an amino group, and R₄ represents a peptide chain attached to an N-terminal amino acid residue. In the above reaction formula, starting peptides D and E are described in the form of α-amino acids for convenience, but can be β-amino acids or γ-amino acids. In the reaction formula above, the side chain R₃ of amino acid residue at the N-terminus and the peptide chain R₄ preferably do not have a functional group that can undergo unintended structural transformation by alkylation reaction or reduction reaction due to the conditions of the alkylation step. When R₃ and/or R₄ have a functional group that may undergo unintended structural transformation due to the conditions of the alkylation step, the target compound can be produced by introducing a protecting group to the functional group in advance and then carrying out the alkylation step.

R₃ is, for example, selected from a hydrogen atom, a C₁-C₆alkyl group, a haloC₁-C₆alkyl group, a C₃-C₆cycloalkyl group, a C₃-C₆cycloalkylC₁-C₆alkyl group, a carboxyC₁-C₆alkyl group, a C₆-C₁₀arylC₁-C₆alkyl group which may have a substituent on the aryl, a 5- to 10-membered heteroarylC₁-C₆alkyl group which may have a substituent on the heteroaryl, a 5- to 10-membered heterocyclylC₁-C₆alkyl group which may have a substituent on the heterocyclyl, a C₃-C₆cycloalkoxyC₁-C₆alkyl group, a haloC₁-C₆alkoxyC₁-C₆alkyl group, a protected aminoC₃-C₆alkyl group, a protected hydroxyC₁-C₆alkyl group, or a C₁-C₆alkoxyC₁-C₆alkyl group.

R₄ may be a peptide chain of two or more amino acid residues which may contain an N-alkylamino acid residue. The side chain of the amino acid residue contained in R₄ may be an amino acid having a side chain that does not have a functional group that may undergo unintended structural transformation by an alkylation reaction or a reduction reaction due to the conditions of the alkylation step. When the side chain of the amino acid residue contained in R₄ has a functional group that may undergo unintended structural transformation by an alkylation reaction or a reduction reaction due to the conditions of the alkylation step, the side chain may be an amino acid having a side chain in which a protecting group has been introduced in advance to the functional group. Such a side chain is, for example, selected from a hydrogen atom, a C₁-C₆alkyl group, a haloC₁-C₆alkyl group, a C₃-C₆cycloalkyl group, a C₃-C₆cycloalkylC₁-C₆alkyl group, a carboxyC₁-C₆alkyl group, a C₆-C₁₀arylC₁-C₆alkyl group which may have a substituent on the aryl, a 5- to 10-membered heteroarylC₁-C₆alkyl group which may have a substituent on the heteroaryl, a 5- to 10-membered heterocyclylC₁-C₆alkyl group which may have a substituent on the heterocyclyl, a C₃-C₆cycloalkoxyC₁-C₆alkyl group, a haloC₁-C₆alkoxyC₁-C₆alkyl group, a protected aminoC₃-C₆alkyl group, a protected hydroxyC₁-C₆alkyl group, or a C₁-C₆alkoxyC₁-C₆alkyl group.

The reaction conditions of the alkylation step in the second embodiment can be referred to the reaction conditions described in the first embodiment by replacing the term "starting amino acid" with the term "peptide containing a starting amino acid" (also referred to as the "starting peptide" herein).

A third embodiment of the present invention is a method for producing a peptide or an ester thereof, comprising the step of using the N-monoalkylamino acid or an ester thereof obtained in the first embodiment (see Formula C), or the peptide or an ester thereof containing an N-monoalkylamino acid residue obtained in the second embodiment (see Formula F), as a starting material, and optionally extending one or more amino acids by a bond-forming reaction (e.g., a peptide bond-forming reaction), to obtain a desired peptide or an ester thereof. The embodiment can also include a method for producing a peptide having a cyclic portion composed of at least 4 amino acids or an ester thereof, which additionally comprises a step of cyclizing with the group at the C-terminus and the group at the N-terminus of the peptide (cyclization precursor peptide, or linear peptide) or an ester thereof obtained by the production method described above to form a cyclic portion.

In the step of extending the main chain of the peptide, when the N-monoalkylamino acid or an ester thereof obtained in the first embodiment (see Formula C) is used as the starting material, it is necessary to extend the main chain of the peptide to form a cyclic portion. However, when the peptide having an N-monoalkylamino acid residue or an ester thereof obtained in the second embodiment (see Formula F) is used as the starting material, it is not necessary to extend the main chain of the peptide. Depending on the chemical structure of the peptide having the cyclic portion of interest, those skilled in the art can appropriately select whether to carry out this step.

By the method according to the present embodiment, a peptide having a cyclic portion composed of at least 4 amino acids or an ester thereof can be produced. The method according to the present embodiment is more suitable for producing a peptide composed of 8 to 15 amino acids or an ester thereof, having a cyclic portion composed of at least 8 amino acids. The peptide or an ester thereof obtained in this embodiment may contain 5 or more, 6 or more, or 7 or more N-alkylamino acid residues.

For the extension of peptide chains by bond-forming reaction or peptide bond-forming reaction, reference can be made, for example, to Biopolym. Pept. Sci. 2000, 55, 227-250, or W.M. Hussein et al., Peptide Synthesis Methods and Protocols (Humana Press, 2020), and the reaction can be performed in a manner well known to those skilled in the art.

The step of forming a cyclic portion is a step of reacting a C-terminal group of the main chain of the peptide in the cyclization precursor peptide obtained above or the peptide having the N-monoalkylamino acid residue obtained in the second embodiment or an ester thereof with an N-terminal group thereof to form a cyclic portion. The C-terminal group and the N-terminal group may be those in combination capable of forming an organic bond with each other.

Preferred combinations are combinations in which the C-terminal group is a carboxy group and the N-terminal group is an amino group. In this case, the step of forming the cyclic portion can be a condensation reaction (peptide bond-forming reaction). For conditions of the condensation reaction, conditions well known to those skilled in the art can be employed, and examples thereof include stirring in a solvent in the presence of a condensing agent. The positions of the carboxyl group, the amino group, and the like used for cyclization are not particularly limited as long as the positions allow the groups to be cyclized, and may be on the main chain or on the side chain.

The condensing agent may be any one capable of binding a carboxy group and an amino group to form a peptide bond. Specific examples of the condensing agent include a carbodiimide-based condensing agent such as N,N'-dicyclohexylcarbodiimide (DCC) or N-(3-dimethylaminopropyl)-N'-ethylcarbodiimide (EDC), a phosphate-azide-based condensing agent such as diphenylphosphate azide (DPPA), a BOP reagent, a phosphonium-based condensing agent such as PyBOP reagent, an uronium-based condensing agent such as TBTU, HBTU, TATU, TATU, or HATU, 4-(4,6-dimethoxy-1,3,5-triazin-2-yl)-4-methylmorpholinium tetrafluoroborate (DMT-MM), or T3P (propylphosphonic anhydride).

In the condensation reaction, an additive may be further added to the reaction mixture. Examples of the additive include 1-hydroxybenzotriazole (HOBt), 1-hydroxy-7-azabenzotriazole (HOAt), 3-hydroxy-1,2,3-benzotriazin-4(3H)-one (HOOBt), and ethyl(hydroxyimino) cyanoacetate (Oxyma).

A fourth embodiment of the present invention is a method of suppressing a production of a dialkylated compound in a production method of an N-monoalkylamino acid or an ester thereof (see Formula C) or a peptide containing an N-monoalkylamino acid or an ester thereof (see Formula F) wherein the dialkylated compound is a compound in which an amino group of the starting amino acid or an ester thereof or a peptide containing the starting amino acid or an ester thereof is dialkylated, the method comprising an alkylation step of mixing the starting amino acid A or B or the starting peptide D or E, a C₁-C₆ primary alkylating agent or a substituted methyl halide, and a catalyst in a solvent in the presence of hydrogen, wherein the alkylation step is carried out at a pressure of 1 atm or more, and produces an N-monoalkylamino acid or an ester thereof (see Formula C) or a peptide containing the N-monoalkylamino acid or an ester thereof (see Formula F) in which a primary alkyl group corresponding to the C₁-C₆ primary alkylating agent or the substituted methyl halide is attached to an amino group of the starting amino acid A or B.

The definition described in the first or second embodiment can be referred to in this embodiment. According to the alkylation step described above, the production of dialkylated compounds can be suppressed.

Furthermore, the obtained dialkylated compound can be easily removed by additionally carrying out a bond-forming reaction using the N-monoalkylamino acid or an ester thereof obtained in the alkylation step (see Formula C) or the peptide containing the N-monoalkylamino acid or an ester thereof (see Formula F) as a starting material and extending the main chain of the peptide, and then carrying out a step of treating the obtained peptide with an acidic aqueous solution.

In one aspect, the linear peptide is a linear peptide represented by the formula: 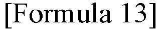 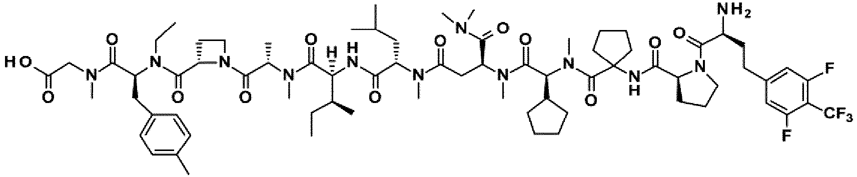 or a salt thereof or a solvate thereof.

In one aspect, the peptide having a cyclic portion which is produced by the method of the present invention is a peptide having a cyclic portion represented by the following Formula (1): 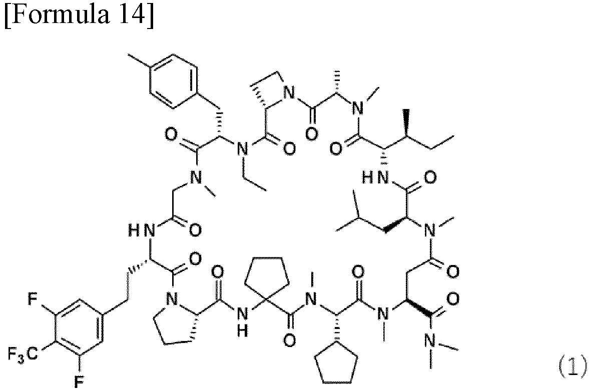 or a salt thereof or a solvate thereof. The peptide having a cyclic portion represented by Formula (1) is preferably a solvate, more preferably a hydrate, DMSO-hydrate, acetone-hydrate, or DMSO-solvate, and further preferably a hydrate. As also described in International Publication No. WO 2021/090855, the peptide having a cyclic portion represented by Formula (1) is useful as a KRAS inhibitor and may be used for various diseases related to KRAS, for example, cancers related to KRAS.

In one aspect, it is preferred that column chromatography is not used in isolation and/or purification of the peptide having a cyclic portion or a salt thereof or a solvate thereof which is produced by the method of the present invention. This crystallization can also be applied to a peptide comprising an N-monoalkylamino acid or an ester thereof.

The peptide having a cyclic portion or a salt thereof or a solvate thereof which is produced by the method of the present invention can be crystallized by crystallization and thereby isolated and/or purified, instead of column chromatography. Specifically, crystals of the peptide having a cyclic portion or a salt thereof or a solvate thereof can be obtained, for example, by subjecting a reaction solution after condensation reaction to a separation procedure, concentrating and/or filtering, if necessary, the organic layer, then adding a solvent suitable for crystallization to the obtained residue, optionally adding seed crystals, and stirring, if necessary, the mixture. The solvent to be added for crystallization is not particularly limited as long as the solvent allows the peptide having a cyclic portion to form crystals, though a solvent that allows a procedure of reducing the solubility of the peptide having a cyclic portion to be performed for a solution of the dissolved peptide having a cyclic portion is preferred. For example, when crystallization can be performed by reducing the solubility of the peptide having a cyclic portion by an addition of a poor solvent or a cooling of the solution, examples of the solvent include solvents that permit such a procedure. When crystals of the peptide having a cyclic portion can be obtained by keeping the suspended state of crude crystals of the peptide having a cyclic portion for any period of time, a solvent that permits such a procedure can be used in crystallization. Specifically, examples of the solvent to be added for crystallization include acetone, water, DMSO, acetonitrile, and ethanol, and combinations thereof.

In one aspect, the crystals of the peptide having a cyclic portion or a salt thereof or a solvate thereof which is produced by the method of the present invention can be non-solvate crystals, solvate crystals, crystals of a salt, or solvate crystals of a salt of the peptide having a cyclic portion represented by Formula (1). In one aspect, the non-solvate crystals (solvent-free crystals) may refer to crystals that are not solvate crystals or hydrate crystals. The crystals of the peptide having a cyclic portion represented by Formula (1) or a salt thereof or a solvate thereof are preferably solvate crystals, and more preferably hydrate crystals.

### [Examples]

Hereinafter, the present invention is described in more detail with Examples. The abbreviations used in Examples are to be understood in the generic sense in the field of organic chemistry, and examples of which are given below.
Bn: benzyl
Boc: t-butoxycarbonyl
Cbz: benzyloxycarbonyl
CPME: cyclopentyl methyl ether
DABCO: 1,4-diazabicyclo[2.2.2]octane
DBN: 1,5-diazabicyclo[4.3.0]nonene-5
DBU: 1,8-diazabicyclo[5.4.0]undecene-7
DCHA: dicyclohexylamine
DIPEA: N,N-diisopropylethylamine
DKP: diketopiperazine
DMA: dimethylacetamide
DMI: 1,3-dimethyl-2-imidazolidinone
DMSO: dimethyl sulfoxide
EOM-Cl: ethoxymethyl chloride
HATU: O-(7-azabenzotriazol-1-yl)-N,N,N',N'-tetram ethyluronium hexafluorophosphate
¹H-NMR spectrum: proton nuclear magnetic resonance spectrum
HPLC: high-performance liquid chromatography
i-: iso
LCMS: liquid chromatography and mass spectrum
MEM-Cl: 2-methoxyethoxymethyl chloride
MOM-Cl: methoxymethyl chloride
MTBE: methyl tert-butyl ether
n-: normal
NMM: N-methylmorpholine
PFP: pentafluorophenyl
s-: secondary
SEM-Cl: 2-(trimethylsilyl)ethoxymethyl chloride
tBu: tertiary butyl
TEA: triethylamine
Teoc: 2-(trimethylsilyl)ethoxycarbonyl
t-: tertiary
THF: tetrahydrofuran
TMSCI: chlorotrimethylsilane
TsOH·H₂O: paratoluenesulfonic acid monohydrate
Sar: sarcosine

The ¹H-NMR spectrum was measured using a nuclear magnetic resonance device JNM-ECZ 500 (manufactured by JEOL Ltd.), the chemical shift of tetramethylsilane used as an internal standard substance was set to 0 ppm, and a deuterium lock signal from a sample solvent was consulted. The chemical shift of a signal from a compound to be analyzed was expressed in ppm. Abbreviations for splitting of a signal were expressed as follows: s = singlet, brs = broad singlet, d = doublet, t = triplet, q = quartet, dd = double doublet, and m = multiplet. The splitting width of a signal was expressed as a J value (Hz). The integral value of signal was calculated on the basis of a ratio of the area intensity of each signal.

For HPLC analysis, measurement was performed at a wavelength of 220 nm for the detection of compound 4 and at a wavelength of 210 nm for the detection of the other compounds with a PDA detector using H-Class System manufactured by Waters Corporation. The reactivity, selectivity, and purity of each substrate used in Examples were evaluated by the analytical method shown in Table 1 below. For LCMS analysis, SQD2 was used for the detection of compounds 3, 4, 11 and 12, and a QDa detector was used for the detection of the other compounds.

**[Table 1]**

| Measurement method | Column | Measurement conditions |
|---|---|---|
| HPLC Method A | Sigma Aldrich Ascentis Express C18 (5 cm x 4.6 mm, 2.7 µm) | Eluent A: 0.05% TFAin water |
| | | Eluent B: 0.05% TFA in MeCN |
| | | Gradient(%B): 5%(0 min) → 100%(6.0 min) → 100%(7.0 min) → 5%(7.01 min) -> 5%(9.0 min) |
| | | Flow rate:0.8 mL/min |
| | | Column temperature: 35°C |
| | | Detection wavelength:210 nm |
| HPLC Method B | Sigma Aldrich Ascentis Express C18 (5 cm x 2.1 mm, 2.7 µm) | Eluent A: 0.05% TFAin water |
| | | Eluent B: 0.05% TFA in MeCN |
| | | Gradient(%B): 5%(0 min) → 100%(5.0 min) → 5%(5.1 min) → 5%(7.0 min) |
| | | Flow rate:0.5 mL/min |
| | | Column temperature:35°C |
| | | Detection wavelength:210 nm |
| HPLC Method C | OSAKA SODA CAPCELL CORE ADME (5 cm x 2.1 mm, 2.7 µm) | Eluent A: 0.05% TFAin water |
| | | Eluent B: 0.05% TFA in MeCN |
| | | Gradient(%B): 5%(0 min) → 100%(5.0 min) → 5%(5.1 min) → 5%(7.0 min) |
| | | Flow rate:0.5 mL/min |
| | | Column temperature:35°C |
| | | Detection wavelength:210 nm |
| HPLC Method D | OSAKA SODA CAPCELL CORE ADME (5 cm x 2.1 mm, 2.7 µm) | Eluent A: 0.05% TFAin water |
| | | Eluent B: 0.05% TFA in MeCN |
| | | Gradient(%B): 5%(0 min) → 100%(10.0 min) → 5%(10.1 min) → 5%(12.0 min) |
| | | Flow rate:0.5 mL/min |
| | | Column temperature:35°C |
| | | Detection wavelength:210 nm |
| HPLC Method E | Sigma Aldrich Ascentis Express RP-Amide (5 cm x 3.0 mm, 2.7 µm) | Eluent A: 0.05% TFA in water |
| | | Eluent B: 0.05% TFA in MeCN |
| | | Gradient(%B): 5%(0 min) → 95%(10.0 min) → 95%(12.0 min) → 5%(12.1 min) → 5%(15.0 min) |
| | | Flow rate:0.7 mL/min |
| | | Column temperature: 30°C |
| | | Detection wavelength:210 nm |
| HPLC Method F | Sigma Aldrich Ascentis Express RP-Amide (5 cm x 3.0 mm, 2.7 µm) | Eluent A: 0.05% TFA in water |
| | | Eluent B: 0.05% TFA in MeCN |
| | | Gradient(%B): 5%(0 min) → 95%(20.0 min) → 95%(24.0 mm) → 5%(24.1 mm) → 5%(30.0 min) |
| | | Flow rate: 1.0 mL/min |
| | | Column temperature:30°C |
| | | Detection wavelength:210 nm |
| HPLC Method G | Waters ACQUITY UPLC CSH C18 (10 cm x 2.1 mm, 1.7 µm) | Eluent A: 0.05% TFAin water |
| | | Eluent B: 0.05% TFA in MeCN |
| | | Gradient(%B): 20%(0 min) → 100%(10.0 min) → 100%(13.5 min) → 20%(13.6 min) → 20%(18.0 min) |
| | | Flow rate:0.3 mL/min |
| | | Column temperature:50°C |
| | | Detection wavelength:210 nm |
| HPLC Method H | Waters ACQUITY UPLC CSH C18 (15 cm x 2.1 mm, 1.7 µm) | Eluent A: 0.05% TFAin water |
| | | Eluent B: 0.05% TFA in MeCN |
| | | Gradient(%B): 20%(0 min) → 100%(24.0 min) → 100%(29 min) → 20%(29.1 min) → 20%(34.0 min) |
| | | Flow rate:0.3 mL/min |
| | | Column temperature:50°C |
| | | Detection wavelength:220 nm |

The melting points of crystals were measured by thermal analysis carried out under the following conditions.

### (Measurement conditions 1)

Measurement apparatus: TGA/DSC3+ (manufactured by Mettler Toledo International Inc.)
Temperature increase rate: 10°C/min
Atmosphere: dry nitrogen

### (Synthesis of raw material 1) Preparation of H-Phe-OtBu

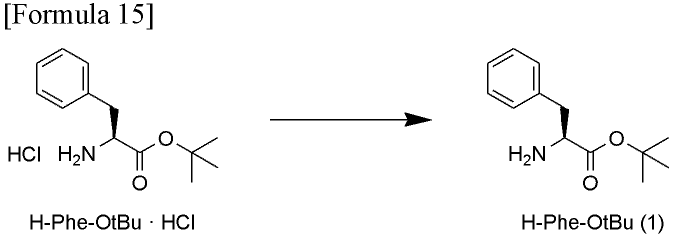

In a separatory funnel, t-butyl L-phenylalaninate hydrochloride (15.05 g, 58.2 mmol) was suspended in 2-methyltetrahydrofuran (200 mL), and 5% aqueous sodium carbonate solution (150 mL) was added, and they were mixed. The solids were gradually dissolved. To the mixture, 5% saline (about 10 mL) was added. The aqueous layer was drained, and the organic layer was washed again with 5% aqueous sodium carbonate (150 mL). The aqueous layer was drained, and the obtained organic layer was dried over anhydrous sodium sulfate. After sodium sulfate was filtered off, the mixture was concentrated under reduced pressure until there was no weight change, and t-butyl L-phenylalanine (hereinafter also referred to as compound 1) was obtained (12.43 g, yield 97%).
HPLC purity: 100%
Measurement method: HPLC Method A
Retention time: 2.72 minutes
¹H-NMR (500 MHz, DMSO-d₆) δ:1.32 (9H, s), 1.66 (2H, bs), 2.79 (2H, m), 3.44 (1H, t, J = 6.9Hz), 7.19-7.21 (3H, m), 7.25-7.28 (2H, m) Mass spectrometry: m/z166.17([M-tBu + H]⁺)

### (Example 1) Evaluation of effect of adding base to monomethylation reaction of H-Phe-OtBu

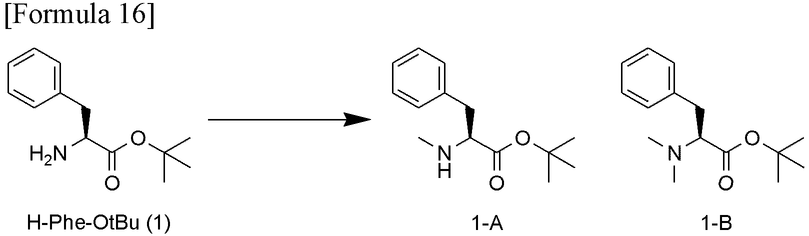

To a flask with a stirrer, 5% palladium carbon (50% wet, 96 mg, 0.023 mmol, 5 mol% metal Pd basis) and a methanol solution of compound 1 (20 mL/g substrate, 2.0 mL, 0.452 mmol) were added. To the obtained mixture, 37% aqueous formaldehyde (33.4 µL, 0.452 mmol, 1.0 eq.) was added, and a base was added. While stirring, the gas phase in the flask was replaced with nitrogen and replaced again with hydrogen. Sampling was performed 3 and 6 hours after replacement with hydrogen, and the ratio of each compound was confirmed by HPLC analysis. The results are shown in Table 2.

**[Table 2]**

| No. | Reaction conditions | | Analysis result (%) | | |
|---|---|---|---|---|---|
| | Base (eq.) | Time (h) | Compound 1 | Compound 1-A | Compound 1-B |
| 1 | None | 3 | 25 | 61.2 | 13.8 |
| 2 | Pyridine (1 eq.) | 3 | 33.7 | 57 | 9.3 |
| 3 | 2,4,6-Collidine (1 eq.) | 3 | 22.5 | 62.6 | 14.9 |
| 4 | NMM (1 eq.) | 3 | 20.4 | 68.9 | 10.7 |
| 5 | TEA (1 eq.) | 3 | 26.7 | 69.6 | 3.7 |
| 6 | DABCO (1 eq.) | 3 | 21.2 | 55.1 | 23.6 |
| 7 | DBU (0.1 eq.) | 3 | 28.6 | 70.1 | 1.3 |
| | | 6 | 25.5 | 72.9 | 1.7 |
| 8 | DBN (0.1 eq.) | 3 | 30.9 | 68.1 | 1.1 |

Measurement method: HPLC Method A
Retention time: 2.82 minutes (compound 1), 2.93 minutes (compound 1-A), 3.05 minutes (compound 1-B)
Mass spectrometry: m/z 236.31 (compound 1-A, [M + H]⁺), 250.30
(compound 1-B, [M + H]⁺)

### (Example 2) Monomethylation reaction of H-Phe-OtBu

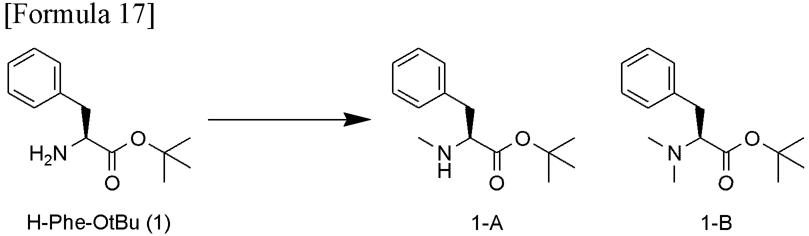

To a flask with a stirrer, 5% palladium carbon (50% wet, 96 mg, 0.023 mmol, 5 mol% metal Pd basis) and a methanol solution of compound 1 (20 mL/g substrate, 2.0 mL, 0.452 mmol) were added. To the obtained mixture, 37% aqueous formaldehyde (67 µL, 0.904 mmol, 2.0 eq.) was added. While stirring, the gas phase in the flask was replaced with nitrogen and replaced again with hydrogen. Sampling was performed 3 and 11 hours after replacement with hydrogen, and the ratio of each compound was confirmed by HPLC analysis. The reaction was performed in the same manner except that DBU (6.7 µL, 0.045 mmol, 0.1 eq.) was added before replacement with nitrogen. The results are shown in Table 3.

**[Table 3]**

| No. | Reaction conditions | | Analysis result (%) | | |
|---|---|---|---|---|---|
| | Base (eq.) | Time (h) | Compound 1 | Compound 1-A | Compound 1-B |
| 1 | None | 3 | 3 | 17.1 | 80 |
| 2 | DBU (1 eq.) | 11 | 4.3 | 88.6 | 6.2 |

Measurement method: HPLC Method A
Retention time: 2.44 minutes (compound 1), 2.54 minutes (compound 1-A), 2.66 minutes (compound 1-B)
Mass spectrometry: m/z 236.31 (compound 1-A, [M + H]⁺), 250.32 (compound 1-B, [M + H]⁺)

### (Example 3) Monoethylation reaction of H-Phe-OtBu with acetaldehyde

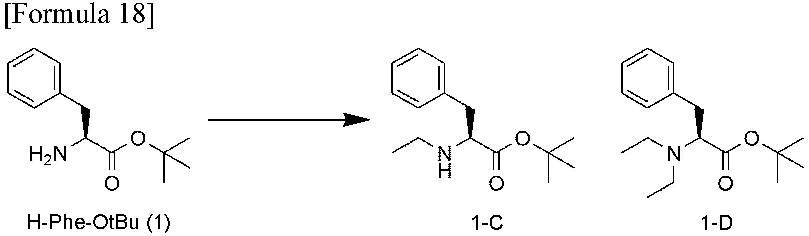

To a flask with a stirrer, 5% palladium carbon (50% wet, 96 mg, 0.023 mmol, 5 mol% metal Pd basis) and an ethanol solution of compound 1 (15 mL/g substrate, 1.5 mL, 0.452 mmol) were added. To the obtained mixture, acetaldehyde (51 µL, 0.904 mmol, 2.0 eq.) was added. While stirring, the gas phase in the flask was replaced with nitrogen and replaced again with hydrogen. Sampling was performed 3 hours after replacement with hydrogen, and the ratio of each compound and the amount of impurities were confirmed by HPLC analysis. The reaction was performed in the same manner except that DBU (6.7 µL, 0.045 mmol, 0.1 eq.) was added before replacement with nitrogen. The results are shown in Table 4.

**[Table 4]**

| No. | Reaction conditions | | Analysis result (%) | | | |
|---|---|---|---|---|---|---|
| | Base (eq.) | Time (h) | Compound 1 | Compound 1-C | Compound 1-D | Impurities |
| 1 | None | 3 | 0.1 | 17.5 | 64.8 | 17.6 |
| 2 | DBU (0.1 eq.) | 3 | 0.1 | 95.6 | 2.9 | 1.4 |

Measurement method: HPLC Method A
Retention time: 2.43 minutes (compound 1), 2.63 minutes (compound 1-C), 2.85 minutes (compound 1-D)
Mass spectrometry: m/z 250.31 (compound 1-C, [M + H]⁺), 278.33 (compound 1-D, [M + H]⁺)

### (Example 4) Monoethylation reaction of H-Phe-OtBu with acetonitrile

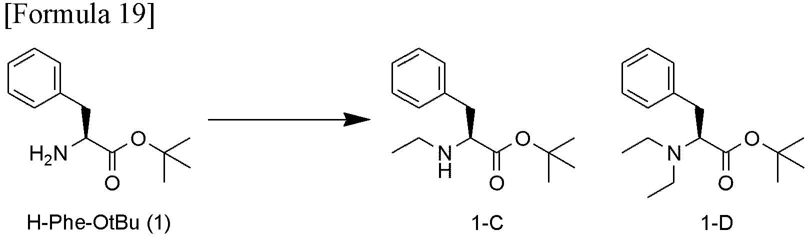

To a flask with a stirrer, 5% palladium carbon (50% wet, 96 mg, 0.023 mmol, 5 mol% metal Pd basis) and an ethanol solution of compound 1 (15 mL/g substrate, 1.5 mL, 0.452 mmol) were added. To the obtained mixture, acetonitrile (236 µL, 4.52 mmol, 10.0 eq.) was added. While stirring, the gas phase in the flask was replaced with nitrogen and replaced again with hydrogen, and the mixture was stirred at 30°C. Sampling was performed 5 and 11 hours after replacement with hydrogen, and the ratio of each compound was confirmed by HPLC analysis. The reaction was performed in the same manner except that DBU (6.7 µL, 0.045 mmol, 0.1 eq.) was added before replacement with nitrogen. The results are shown in Table 5.

**[Table 5]**

| No. | Reaction conditions | | Analysis result (%) | | |
|---|---|---|---|---|---|
| | Base (eq.) | Time (h) | Compound 1 | Compound 1-C | Compound 1-D |
| 1 | None | 5 | 0.2 | 84.8 | 15.1 |
| 2 | DBU (0.1 eq.) | 11 | 2.7 | 96.3 | 1 |

Measurement method: HPLC Method A
Retention time: 2.61 minutes (compound 1), 2.83 minutes (compound 1-C), 3.06 minutes (compound 1-D)
Mass spectrometry: m/z 250.09 (compound 1-C, [M + H]⁺), 278.06 (compound 1-D, [M + H]⁺)

### (Example 5) Monopropylation reaction of H-Phe-OtBu with propanal

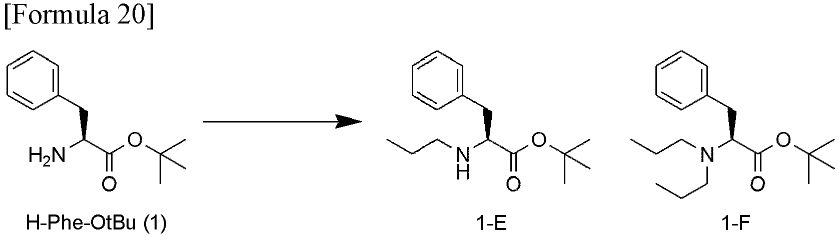

To a flask with a stirrer, 5% palladium carbon (50% wet, 96 mg, 0.023 mmol, 5 mol% metal Pd basis) and an ethanol solution of compound 1 (15 mL/g substrate, 1.5 mL, 0.452 mmol) were added. To the obtained mixture, propanal (65 µL, 0.904 mmol, 2.0 eq.) was added. While stirring, the gas phase in the flask was replaced with nitrogen and replaced again with hydrogen. Sampling was performed 3 and 6 hours after replacement with hydrogen, and the ratio of each compound and the amount of impurities were confirmed by HPLC analysis. The reaction was performed in the same manner except that DBU (6.7 µL, 0.045 mmol, 0.1 eq.) was added before replacement with nitrogen. The results are shown in Table 6.

**[Table 6]**

| No. | Reaction conditions | | Analysis result (%) | | | |
|---|---|---|---|---|---|---|
| | Base (eq.) | Time (h) | Compound 1 | Compound 1-E | Compound 1-F | Impurities |
| 1 | None | 3 | 0.1 | 39 | 42 | 18.9 |
| 2 | DBU (0.1 eq.) | 6 | 0.3 | 95.3 | 1.5 | 2.9 |

Measurement method: HPLC Method A
Retention time: 2.42 minutes (compound 1), 2.82 minutes (compound 1-E), 3.27 minutes (compound 1-F)
Mass spectrometry: m/z 264.36 (compound 1-E, [M + H]⁺), 306.38 (compound 1-F, [M + H]⁺)

### (Example 6) Monopropylation reaction of H-Phe-OtBu with propionitrile

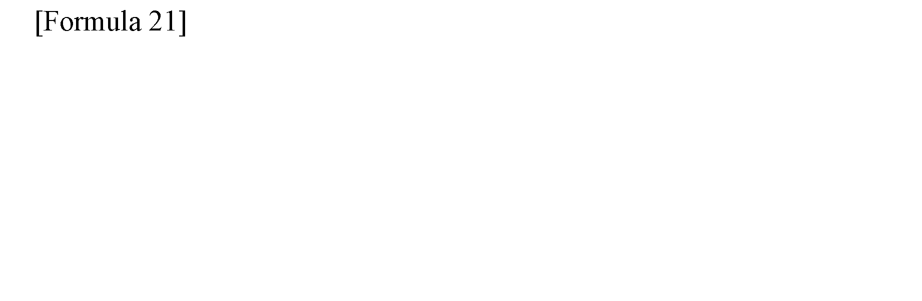

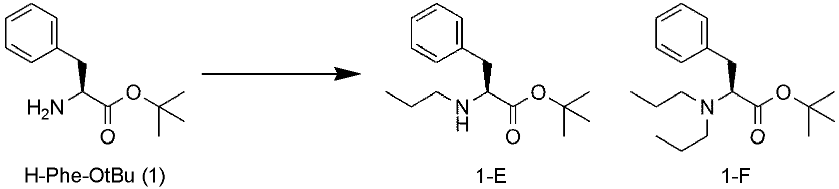

To a flask with a stirrer, 5% palladium carbon (50% wet, 96 mg, 0.023 mmol, 5 mol% metal Pd basis) and an ethanol solution of compound 1 (15 mL/g substrate, 1.5 mL, 0.452 mmol) were added. To the obtained mixture, propionitrile (322 µL, 4.52 mmol, 10.0 eq.) was added. While stirring, the gas phase in the flask was replaced with nitrogen and replaced again with hydrogen. Sampling was performed 8 hours after replacement with hydrogen, and the ratio of each compound was confirmed by HPLC analysis. The results are shown in Table 7.

**[Table 7]**

| Analysis result (%) | | |
|---|---|---|
| Compound 1 | Compound 1-E | Compound 1-F |
| 1.1 | 96.8 | 2.1 |

Measurement method: HPLC Method A
Retention time: 2.69 minutes (compound 1), 3.11 minutes (compound 1-E), 3.58 minutes (compound 1-F)
Mass spectrometry: m/z 264.29 (compound 1-E, [M + H]⁺), 306.31 (compound 1-F, [M + H]⁺)

### (Example 7) Monobutylation reaction of H-Phe-OtBu with butanal

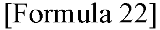

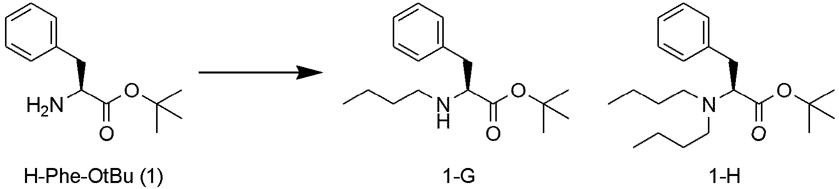

To a flask with a stirrer, 5% palladium carbon (50% wet, 96 mg, 0.023 mmol, 5 mol% metal Pd basis) and an ethanol solution of compound 1 (15 mL/g substrate, 1.5 mL, 0.452 mmol) were added. To the obtained mixture, butanal (81 µL, 0.904 mmol, 2.0 eq.) was added. While stirring, the gas phase in the flask was replaced with nitrogen and replaced again with hydrogen. Sampling was performed 3 and 6 hours after replacement with hydrogen, and the ratio of each compound and the amount of impurities were confirmed by HPLC analysis. The reaction was performed in the same manner except that DBU (6.7 µL, 0.045 mmol, 0.1 eq.) was added before replacement with nitrogen. The results are shown in Table 8.

**[Table 8]**

| No. | Reaction conditions | | Analysis result (%) | | | |
|---|---|---|---|---|---|---|
| | Base (eq.) | Time (h) | Compound 1 | Compound 1-G | Compound 1-H | Impurities |
| 1 | None | 3 | Not detected | 29 | 58.9 | 12.1 |
| 2 | DBU (0.1 eq.) | 6 | 0.2 | 95.2 | 1.9 | 2.7 |

Measurement method: HPLC Method A
Retention time: 2.42 minutes (compound 1), 3.04 minutes (compound 1-G), 3.68 minutes (compound 1-H)
Mass spectrometry: m/z 278.35 (compound 1-G, [M + H]⁺), 334.43 (compound 1-H, [M + H]⁺)

### (Example 8) Monobutylation reaction of H-Phe-OtBu with butylonitrile

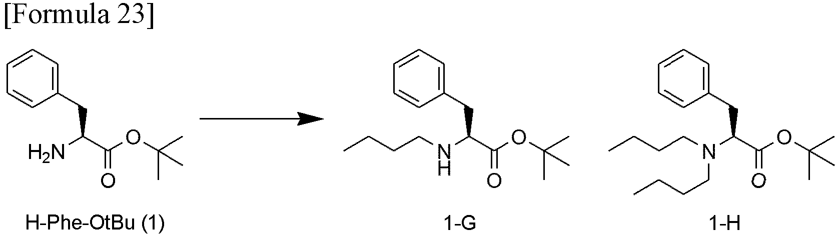

To a flask with a stirrer, 5% palladium carbon (50% wet, 96 mg, 0.023 mmol, 5 mol% metal Pd basis) and an ethanol solution of compound 1 (15 mL/g substrate, 1.5 mL, 0.452 mmol) were added. To the obtained mixture, butylonitrile (393 µL, 4.52 mmol, 10.0 eq.) was added. While stirring, the gas phase in the flask was replaced with nitrogen and replaced again with hydrogen. Sampling was performed 5 hours after replacement with hydrogen, and the ratio of each compound was confirmed by HPLC analysis. The results are shown in Table 9.

**[Table 9]**

| Analysis result (%) | | |
|---|---|---|
| Compound 1 | Compound 1-G | Compound 1-H |
| 2.2 | 96 | 1 |

Measurement method: HPLC Method A
Retention time: 2.67 minutes (compound 1), 3.32 minutes (compound 1-G), 3.99 minutes (compound 1-H)
Mass spectrometry: m/z 278.30 (compound 1-G, [M + H]⁺), 334.39 (compound 1-F, [M + H]⁺)

### (Example 9) Monopropylation reaction of H-Val-MeAsp(OtBu)-NMe₂ with propanal

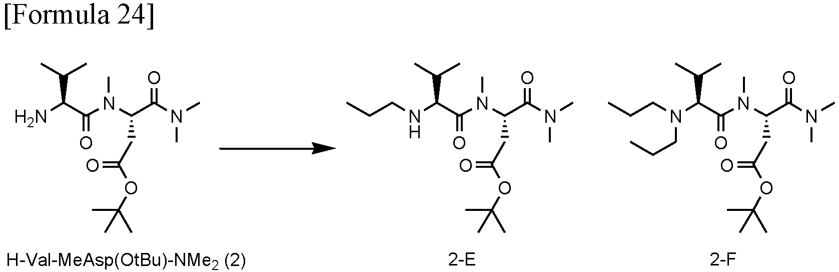

To a vial with a stirrer, 5% palladium carbon (50% wet, 48 mg, 0.011 mmol, 5 mol% metal Pd basis) and an ethanol solution of compound 2 (10 mL/g substrate, 0.75 mL, 0.228 mmol) were added. To the obtained mixture, propanal (82 µL, 1.138 mmol, 5.0 eq.) was added. The vial was placed in a pressure-resistant vessel, and while stirring the reaction solution in the vial, the gas phase in the pressure-resistant vessel was replaced with nitrogen and replaced again with hydrogen. Sampling was performed 6 hours after replacement with hydrogen, and the ratio of each compound and the amount of impurities were confirmed by HPLC analysis. The reaction was performed in the same manner except that TEA (63.5 µL, 0.455 mmol, 2.0 eq.) was added before replacement with nitrogen. The results are shown in Table 10.

**[Table 10]**

| No. | Reaction conditions | Analysis result (%) | | | |
|---|---|---|---|---|---|
| | Base (eq.) | Compound 2 | Compound 2-E | Compound 2-F | Impurities |
| 1 | None | 1.4 | 65.1 | 19 | 14.5 |
| 2 | TEA (2 eq.) | 0.4 | 85.5 | 8.5 | 5.6 |

Measurement method: HPLC Method A
Retention time: 2.06 minutes (compound 2), 2.41 minutes (compound 2-E), 2.77 minutes (compound 2-F)
Mass spectrometry: m/z 372.68 (compound 2-E, [M + H]⁺), 414.65 (compound 2-F, [M + H]⁺)

### (Example 10) Monobutylation reaction of H-Val-MeAsp(OtBu)-NMe₂ with butanal

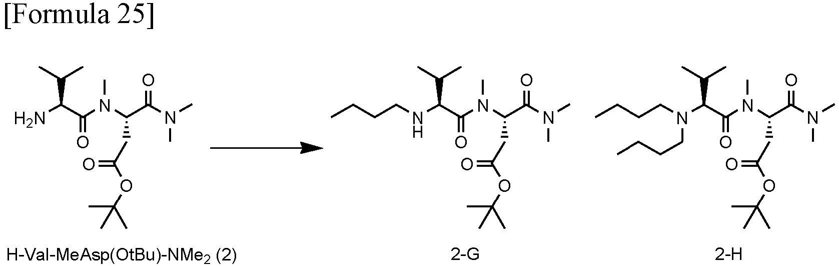

To a vial with a stirrer, 5% palladium carbon (50% wet, 48 mg, 0.011 mmol, 5 mol% metal Pd basis) and an ethanol solution of compound 2 (10 mL/g substrate, 0.75 mL, 0.228 mmol) were added. To the obtained mixture, butanal (103 1 µL, 1.138 mmol, 5.0 eq.) was added. The vial was then placed in a pressure-resistant vessel, and while stirring the vial in the pressure-resistant vessel, the gas phase in the pressure-resistant vessel was replaced with nitrogen and replaced again with hydrogen. Sampling was performed 6 hours after replacement with hydrogen, and the ratio of each compound and the amount of impurities were confirmed by HPLC analysis. The reaction was performed in the same manner except that TEA (63.5 µL, 0.455 mmol, 2.0 eq.) was added before replacement with nitrogen. The results are shown in Table 11.

**[Table 11]**

| No. | Reaction conditions | Analysis result (%) | | | |
|---|---|---|---|---|---|
| | Base (eq.) | Compound 2 | Compound 2-G | Compound 2-H | Impurities |
| 1 | None | 2.1 | 60.3 | 27.6 | 10 |
| 2 | TEA (2 eq.) | 2.3 | 86.2 | 8 | 3.5 |

Measurement method: HPLC Method A
Retention time: 2.06 minutes (compound 2), 2.64 minutes (compound 2-G), 3.22 minutes (compound 2-H)
Mass spectrometry: m/z 386.71 (compound 2-G, [M + H]⁺), 442.87 (compound-H, [M + H]⁺)

### (Example 11) One-pot co-implementation of Cbz-Phe-OtBu deprotection and monoethylation reaction

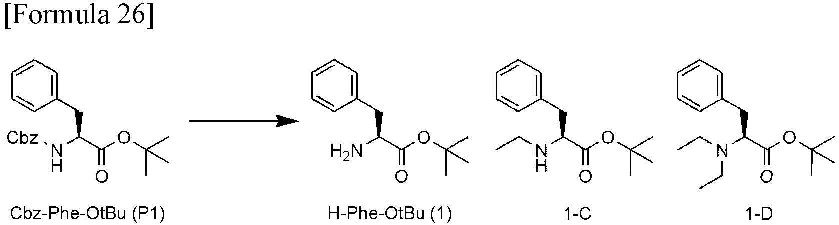

To a flask or vial with a stirrer, 5% palladium carbon (50% wet, 120 mg, 0.028 mmol, 5 mol% metal Pd basis) and p-toluenesulfonic acid (107 mg, 0.563 mmol, 1.0 eq.) were added. To the obtained mixture, a solution of compound P1 in THF (10 mL/g substrate, 2.0 mL, 0.563 mmol) and acetonitrile (294 µL, 5.63 mmol, 10.0 eq.) were sequentially added. While stirring, the gas phase in the flask was replaced with nitrogen and replaced again with hydrogen, and the mixture was stirred at 30°C. When the hydrogen pressure was 1 atm, hydrogen was supplied with a balloon filled with hydrogen. Under conditions that the hydrogen pressure was 3.5 atm or 5.5 atm, the vial containing the reaction solution was placed in a pressure-resistant vessel, and the gas in the pressure-resistant vessel was replaced with nitrogen and then replaced with hydrogen. Sampling was performed 10 hours after replacement with hydrogen, and the ratio of each compound was confirmed by HPLC analysis. The results are shown in Table 12.

**[Table 12]**

| No. | Reaction conditions | Analysis result (%) | | | |
|---|---|---|---|---|---|
| | Hydrogen pressure | Compound P1 | Compound 1 | Compound 1-C | Compound 1-D |
| 1 | 1 atm (balloon) | Not detected | 0.2 | 84.1 | 15.7 |
| 2 | 3.5 atm | Not detected | Not detected | 90.1 | 9.9 |
| 3 | 5.5 atm | Not detected | 0.3 | 92.9 | 6.8 |

Measurement method: HPLC Method A
Retention time: 2.61 minutes (compound 1), 2.83 minutes (compound 1-C), 3.06 minutes (compound 1-D)
Mass spectrometry: m/z 250.09 (compound 1-C, [M + H]⁺), 278.06 (compound 1-D, [M + H]⁺)

### (Synthesis of raw material 2) Synthesis of Cbz-Asp(OtBu)-NMe₂

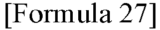

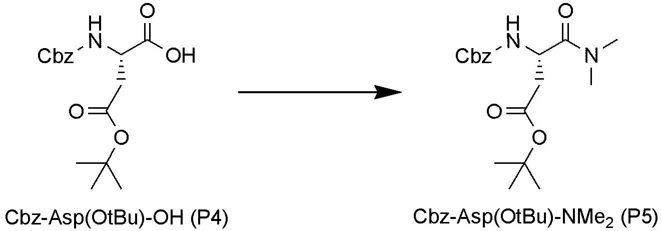

To a 200 mL flask with nitrogen replacement, compound P4 (3.55 g, 10.4 mmol) and 2-methyl tetrahydrofuran (32 mL, 30 eq.) were added. To the obtained mixture, a solution of dimethylamine in THF (2.0 M, 7.8 mL, 15.6 mmol, 1.5 eq.) and DIPEA (6.2 mL, 36.4 mmol, 3.5 eq.) were sequentially added, while cooling the flask with an ice bath. Then, a solution of propylphosphonic acid anhydride in 2-methyltetrahydrofuran (1.6 M, 16.3 mL, 26.0 mmol, 2.5 eq.) was added dropwise to the mixture. The internal temperature during the dropwise addition was 15.0 to 26.0°C. After completion of the dropwise addition, the mixture was stirred at room temperature for 1.5 hours. Sampling was then performed, and the completion of the reaction was confirmed by HPLC analysis. While cooling the flask with an ice bath, a 5% aqueous sodium hydrogen sulfate solution (20 mL) was slowly added thereto. After stirring for 15 minutes, the entire reaction solution was transferred to a separatory funnel, and after standing still, the aqueous layer was removed. To the organic layer, a 5% aqueous sodium bisulfate solution (20 mL) was added. After shaken well, the mixture was stood still, and then the aqueous layer was removed. A 5% aqueous potassium carbonate solution (20 mL) was added to the organic layer. After shaken well, the mixture was stood still, and then the aqueous layer was removed. The washing with 5% aqueous potassium carbonate solution (20 mL) was repeated one more time. The obtained organic layer was concentrated under reduced pressure to obtain 3.9 g of the crude product. The obtained crude product was purified by silica gel column chromatography to obtain compound P5 (3.48 g, 95% yield).
HPLC purity: 100%
Measurement method: HPLC Method A
Retention time: 3.83 minutes
Mass spectrometry: m/z 295.21 ([M-tBu + H]⁺)

### (Example 12) One-pot implementation of Cbz-Asp(OtBu)-NMe₂ deprotection and monopropylation reaction

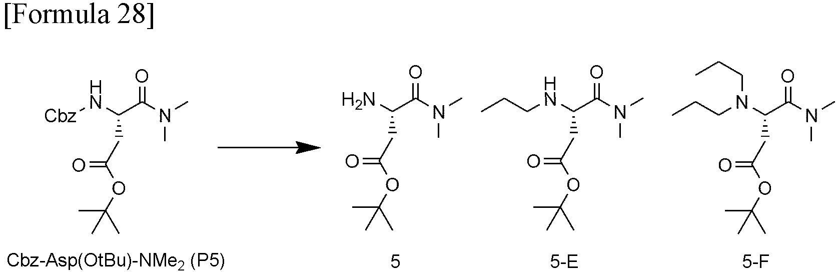

To a flask with a stirrer, 5% palladium carbon (50% wet, 60 mg, 0.014 mmol, 5 mol% metal Pd basis) and an ethanol solution of compound P5 (15 mL/g substrate, 1.5 mL, 0.285 mmol) were added. While stirring, nitrogen replacement was performed, then hydrogen replacement was performed, and deprotection reaction was performed. After 3 hours, the mixture was degassed under reduced pressure, and propanal (41 µL, 0.571 mmol, 2.0 eq.) was added. While stirring, the gas phase in the flask was replaced with nitrogen and replaced again with hydrogen. Sampling was performed 4 hours after replacement with hydrogen, and the ratio of each compound and the amount of impurities were confirmed by HPLC analysis. The reaction was performed in the same manner except that DBU (4.3 µL, 0.029 mmol, 0.1 eq.) was added immediately before adding propanal. The results are shown in Table 13.

**[Table 13]**

| No. | Reaction conditions | Analysis result (%) | | | | |
|---|---|---|---|---|---|---|
| | Base (eq.) | Compound P5 | Compound 5 | Compound 5-E | Compound 5-F | Impurities |
| 1 | None | Not detected | Not detected | 34.6 | 57 | 8.4 |
| 2 | DBU (0.1 eq) | Not detected | Not detected | 90.2 | 8.5 | 1.3 |

Measurement method: HPLC Method A
Retention time: 3.57 minutes (compound P5), 1.69 minutes (compound 5), 2.04 minutes (compound 5-E), 2.49 minutes (compound 5-F)
Mass spectrometry: m/z 295.23 (compound P5, [M-tBu + H]⁺), 259.32 (compound 5-E, [M + H]⁺), 301.35 (compound 5-F, [M + H]⁺)

### (Synthesis of raw material 3) Synthesis of Cbz-Phe(4-Me)-Sar-OtBu

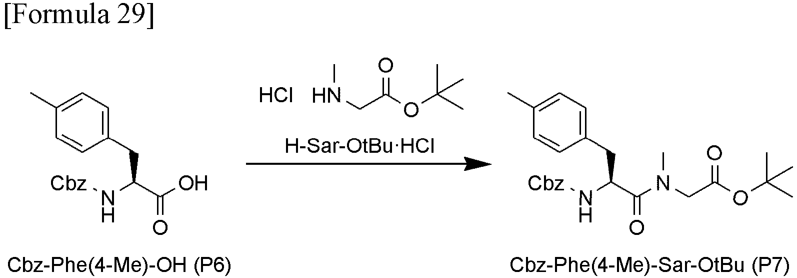

To a 2 L flask, compound P6 (30.36 g, 96 mmol) and t-butyl sarcosinate hydrochloride (20.87 g, 115 mmol, 1.2 eq.) were added, and the gas phase in the flask was replaced with nitrogen. 2-methyltetrahydrofuran (290 mL, 30 eq.) was added, and the mixture was cooled until the external temperature reached 15°C, and DIPEA (88 mL, 517 mmol, 5.4 eq.) was added dropwise from a dropping funnel. A solution of propylphosphonic acid anhydride in 2-methyltetrahydrofuran (1.6 M, 132 mL, 211 mmol, 2.2 eq.) was added dropwise for 1 hour and 20 minutes from a dropping funnel. The internal temperature during the dropwise addition was 15.0 to 17.4°C. Sampling was performed 1 hour after the completion of the dropwise addition, and the completion of the reaction was confirmed by HPLC analysis. A 5% aqueous sodium carbonate solution (180 mL) was added slowly from a dropping funnel. The internal temperature during the dropwise addition was maintained at 30.3°C or lower. After completion of the dropwise addition, the external temperature was set to 23°C. The mixture was stirred for 15 minutes or more, then allowed to stand still, and then the aqueous layer was removed. To the organic layer, a 5% aqueous sodium bisulfate solution (180 mL) was added. The mixture was stirred for 10 minutes or more, then allowed to stand still, and then the aqueous layer was removed. The washing with 5% aqueous sodium bisulfate solution (180 mL) was repeated one more time. To the organic layer, a 5% aqueous sodium carbonate solution (180 mL) was added. The mixture was stirred for 10 minutes or more, then allowed to stand still, and then the aqueous layer was removed. The obtained organic layer was concentrated under reduced pressure conditions to obtain 44.93 g of the crude product. A part of the obtained crude product was purified by silica gel column chromatography to obtain compound P7.
HPLC purity: 100%
Measurement method: HPLC Method B
Retention time: 3.80 minutes
Mass spectrometry: m/z 385.34 ([M-tBu + H]⁺)

### (Example 13) One-pot implementation of Cbz-Phe(4-Me)-Sar-OtBu deprotection and monoethylation reaction: suppression effect of DKP formation by addition of TsOH

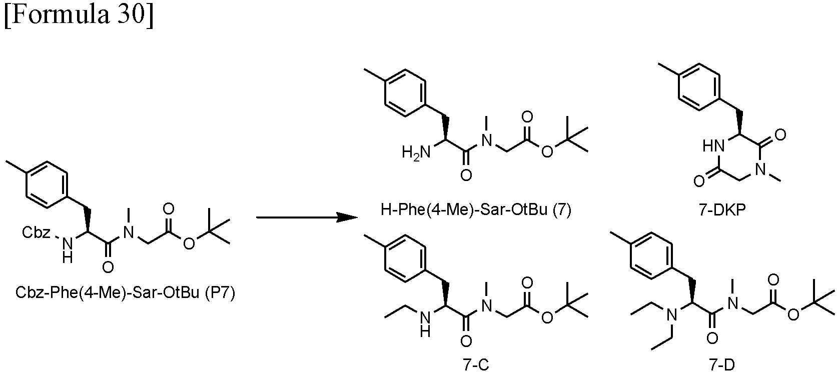

To a flask with a stirrer, 5% palladium carbon (50% wet, 48 mg, 0.011 mmol, 5 mol% metal Pd basis) and TsOH·H₂O (43 mg, 0.227 mmol, 1.0 eq.) were added. To the obtained mixture, a solution of compound P7 in 2-methyltetrahydrofuran (10 mL/g substrate, 1.0 mL, 0.227 mmol) and acetonitrile (119 µL, 2.27 mmol, 10.0 eq.) were sequentially added. While stirring, the gas phase in the flask was replaced with nitrogen and replaced again with hydrogen, and the mixture was stirred at 25°C. Sampling was performed 6 or 10.5 hours after replacement with hydrogen, and the ratio of each compound was confirmed by HPLC analysis. The reaction was performed in the same manner except that TsOH·H₂O (43 mg, 0.227 mmol, 1.0 eq.) was added before replacement with nitrogen. The results are shown in Table 14.

**[Table 14]**

| No. | Reaction conditions | | Analysis result (%) | | | | |
|---|---|---|---|---|---|---|---|
| | Acid (eq.) | Time | Compou nd P7 | Compou nd 7-DKP | Compou nd 7 | Compou nd 7-C | Compou nd 7-D |
| 1 | None | 6 | Not detected | 13.8 | Not detected | 82.9 | 3.3 |
| 2 | TsOH (1.0 eq.) | 10.5 | Not detected | 2 | Not detected | 91.1 | 6.9 |

Measurement method: HPLC Method B
Retention time: 1.66 minutes (compound 7-DKP), 2.23 minutes (compound 7), 2.32 minutes (compound 7-C), 2.46 minutes (compound 7-D)
Mass spectrometry: m/z 233.26 (compound 7-DKP, [M + H]⁺), 307.26 (compound 7, [M + H]⁺), 335.34 (compound 7-C, [M + H]⁺), 363.38 (compound 7-D, [M + H]⁺)

### (Example 14) One-pot implementation of Cbz-Phe(4-Me)-Sar-OtBu deprotection and monoethylation reaction

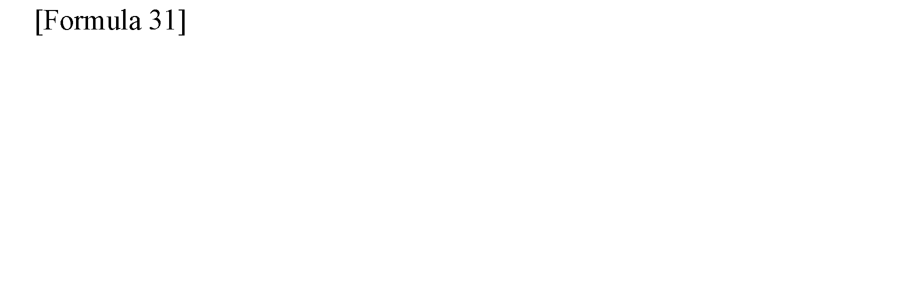

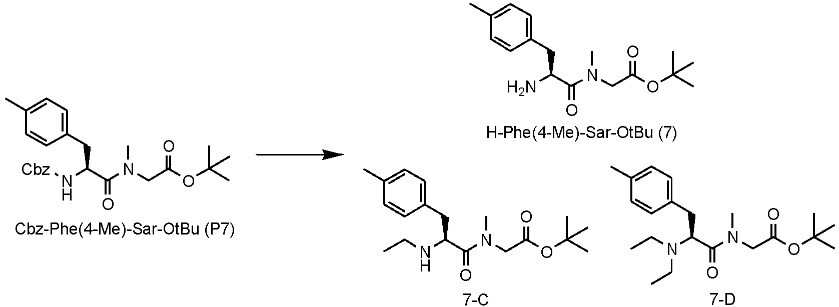

To a flask or pressure-resistant reaction vessel with a stirrer, 5% palladium carbon (50% wet, 97 mg, 0.023 mmol, 5 mol% metal Pd basis) and p-toluenesulfonic acid (86 mg, 0.454 mmol, 1.0 eq.) were added. A solution of compound P7 in 2-methyltetrahydrofuran (10 mL/g substrate, 2.0 mL, 0.454 mmol) and acetonitrile (237 µL, 4.54 mmol, 10.0 eq.) were sequentially added. While stirring, the gas phase in the flask was replaced with nitrogen and replaced again with hydrogen, and the reaction was performed at 30°C. When the hydrogen pressure was 1 atm, hydrogen was supplied with a balloon filled with hydrogen. When the hydrogen pressure was 5.5 atm, a vial containing the reaction solution was placed in a pressure-resistant vessel, and the gas in the pressure-resistant vessel was replaced with nitrogen and then replaced with hydrogen. Sampling was performed 10 hours after replacement with hydrogen, and the ratio of each compound was confirmed by HPLC analysis. The results are shown in Table 15.

**[Table 15]**

| No. | Reaction conditions | Analysis result (%) | | | |
|---|---|---|---|---|---|
| | Hydrogen pressure | Compound P7 | Compound 7 | Compound 7-C | Compound 7-D |
| 1 | 1 atm (balloon) | Not detected | 0.4 | 87.6 | 11.1 |
| 2 | 5.5 atm | Not detected | Not detected | 94.1 | 5.8 |

Measurement method: HPLC Method B
Retention time: 2.23 minutes (compound 7), 2.32 minutes (compound 7-C), 2.46 minutes (compound 7-D)
Mass spectrometry: m/z 307.26 (compound 7, [M + H]⁺), 335.34 (compound 7-C, [M + H]⁺), 363.38 (compound 7-D, [M + H]⁺)

### (Example 15) Synthesis of Cbz-Aze-EtPhe(4-Me)-Sar-OtBu

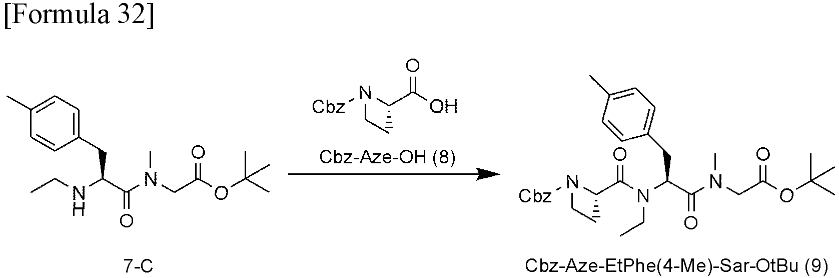

Compound 7-C (3.00 g, 8.97 mmol, including 1.6% Compound 7-D) was dissolved in 2-methyltetrahydrofuran (27.0 mL, 30 eq.) in a 200 mL flask. To the mixture, compound 8 (3.17 g, 13.45 mmol, 1.5 eq.) was added, and nitrogen replacement was performed. The mixture was cooled until the external temperature reached 15°C, and DIPEA (9.0 mL, 53.8 mmol, 6.0 eq.) was added with a syringe. A solution of propylphosphonic acid anhydride in 2-methyltetrahydrofuran (1.6 M, 20 mL, 31.4 mmol, 3.5 eq.) was added dropwise for 17 minutes from a dropping funnel. The internal temperature during the dropwise addition was 15.0 to 18.0°C. Sampling was performed 5 hours after the completion of the dropwise addition, and the completion of the reaction was confirmed by HPLC analysis. A 5% aqueous sodium carbonate solution (27 mL) was added slowly from a dropping funnel. The internal temperature during the dropwise addition was maintained at 28.0°C or lower. After completion of the dropwise addition, the external temperature was set to 25°C. The mixture was stirred for 10 minutes, then allowed to stand still, and then the aqueous layer was removed. Cyclohexane (15 mL) and a 5% aqueous sodium bisulfate solution (30 mL) were added, and the mixture was stirred for 10 minutes or more, then allowed to stand still, and then the aqueous layer was removed. The washing with 5% aqueous sodium bisulfate solution (30 mL) was repeated one more time. A 5% aqueous sodium carbonate solution (30 mL) was added, and the mixture was stirred for 10 minutes or more, then allowed to stand still, and then the aqueous layer was removed. It was confirmed by HPLC analysis that compound 7-D contained in the raw material was completely removed by the washing. The analysis results before the reaction and after the washing are shown in Table 16.

**[Table 16]**

| No. | Analysis point | Analysis result (%) | | |
|---|---|---|---|---|
| | | Compound P7 | Compound 7 | Compound 7-C |
| 1 | Before reaction | 97.7 | 1.6 | Not detected |
| 2 | After washing | Not detected | Not detected | 99.1 |

Measurement method: HPLC Method B
Retention time: 2.47 minutes (compound 7-C), 2.61 minutes (compound 7-D), 3.84 minutes (compound 9)
Mass spectrometry: m/z 407.36 (compound 9, [M-Sar + H]⁺)

### (Synthesis of raw material 4) 50 gram-scale synthesis of Cbz-Phe(4-Me)-Sar-OtBu

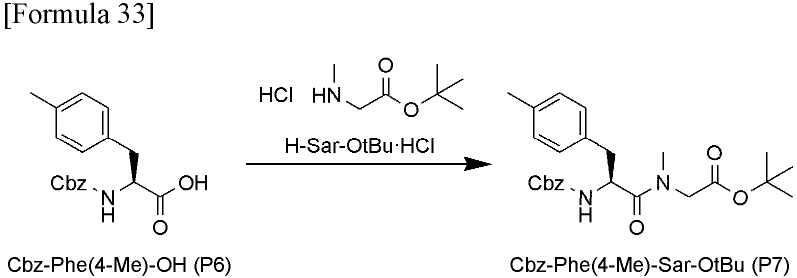

To a 2 L flask, compound P6 (50.03 g, 160 mmol) and t-butyl sarcosinate hydrochloride (34.50 g, 191 mmol, 1.2 eq.) were added, and nitrogen replacement was performed. 2-methyltetrahydrofuran (485 mL, 30 eq.) was added, and the mixture was cooled until the external temperature reached 15°C, and DIPEA (147 mL, 862 mmol, 5.4 eq.) was added dropwise from a dropping funnel. A solution of propylphosphonic acid anhydride in 2-methyltetrahydrofuran (1.6 M, 219 mL, 2.2 eq.) was added dropwise for 1 hour and 20 minutes from a dropping funnel. The internal temperature during the dropwise addition was 15.5 to 18.5°C. Sampling was performed 1 hour after the completion of the dropwise addition, and the completion of the reaction was confirmed by HPLC analysis. A 5% aqueous sodium carbonate solution (300 mL) was added slowly from a dropping funnel. The internal temperature during the dropwise addition was maintained at 22.8°C or lower. After completion of the dropwise addition, the external temperature was set to 23°C. The mixture was stirred for 15 minutes or more, then allowed to stand still, and then the aqueous layer was removed. A 5% aqueous sodium bisulfate solution (300 mL) was added, and the mixture was stirred for 10 minutes or more, then allowed to stand still, and then the aqueous layer was removed. The washing with 5% aqueous sodium bisulfate solution (300 mL) was repeated two more times. A 5% aqueous sodium carbonate solution (300 mL) was added, and the mixture was stirred for 10 minutes or more, then allowed to stand still, and then the aqueous layer was removed. A 5% aqueous sodium chloride solution (300 mL) was added, and the mixture was stirred for 10 minutes or more, then allowed to stand still, and then the aqueous layer was removed. The washing with 5% aqueous sodium chloride solution (300 mL) was repeated one more time. The obtained organic layer was concentrated under reduced pressure conditions. To a solution of the obtained compound P7 (175.41 g), 2-methyltetrahydrofuran (229 mL) was added, and a solution at a concentration of 0.189 g/g was prepared.
HPLC purity: 99.37%
Measurement method: HPLC Method B
Retention time: 3.78 minutes
Mass spectrometry: m/z 385.32([M-tBu + H]⁺)

### (Example 16) 30 gram-scale one-pot implementation of Cbz-Phe(4-Me)-Sar-OtBu deprotection and monoethylation reaction

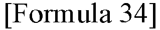

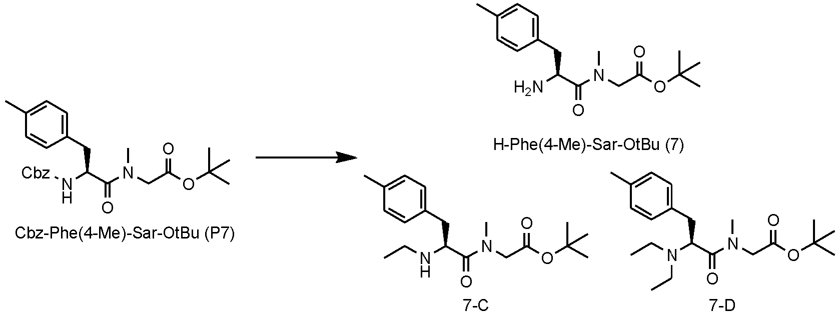

The inside air of a 1 L pressure-resistant reaction vessel with stirring blades was replaced with nitrogen, and 5% palladium carbon (50% wet, 20.30 g, 4.77 mmol, 7 mol% metal Pd basis) and TsOH·H₂O (12.95 g, 68.1 mmol, 1.0 eq.) were added thereto. A solution of compound P7 in 2-methyltetrahydrofuran (concentration 0.189 g/g, 159.0 g solution, 30.0 g substrate, 68.1 mmol) was added, and 2-methyltetrahydrofuran (150 mL) and acetonitrile (35.6 mL, 681 mmol, 10.0 eq.) were sequentially added. While stirring, nitrogen replacement was performed and then hydrogen replacement was performed, then a reaction was performed at 30°C. The vessel internal pressure during the reaction was maintained at 2 to 4 atm by hydrogen supply. Degassing was performed for 1 minute at a depressurization degree of 150 to 300 torr at 1 hour, 2 hours, and 3 hours after the start of reaction. 5% palladium carbon (50% wet, 8.70 g, 2.04 mmol, 3 mol% metal Pd basis) was added at the time point of 5 hours reaction time. Nitrogen replacement was performed at the time point of 11 hours reaction time, and the mixture was allowed to stand still and stored under the nitrogen atmosphere for 12 hours. After storage, the reaction was resumed by hydrogen replacement, and after 7 hours after the resumption, the reaction was terminated by nitrogen replacement. The palladium carbon was filtered off by suction under reduced pressure, and the filtered palladium carbon was washed three times with 2-methyl tetrahydrofuran (90 mL). The filtrate and wash solution were mixed, and the mixture was washed twice with 5% aqueous sodium carbonate solution (150 mL) using a separatory funnel. The organic layer after washing was concentrated under reduced pressure to yield compound 7-C, which was a target product. The yield was 93% over 2 steps from compound P6. The analysis results during and after the reaction are shown in Table 17.

**[Table 17]**

| No. | Analysis point | Analysis result (%) | | | |
|---|---|---|---|---|---|
| | | Compound P7 | Compound 7 | Compound 7-C | Compound 7-D |
| 1 | Reaction 5 h | Not detected | 69.2 | 29.8 | 0.3 |
| 2 | Reaction 11 h | Not detected | 0.5 | 97.4 | 1.7 |
| 3 | Reaction 18 h | Not detected | 0.1 | 97.3 | 1.7 |

Measurement method: HPLC Method B
Retention time: 2.23 minutes (compound 7), 2.32 minutes (compound 7-C), 2.46 minutes (compound 7-D)
Mass spectrometry: m/z 307.26 (compound 7, [M + H]⁺), 335.34 (compound 7-C, [M + H]⁺), 363.38 (compound 7-D, [M + H]⁺)

### (Example 17) Evaluation of methylation reagent in monomethylation reaction of H-Phe-OtBu

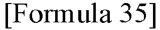

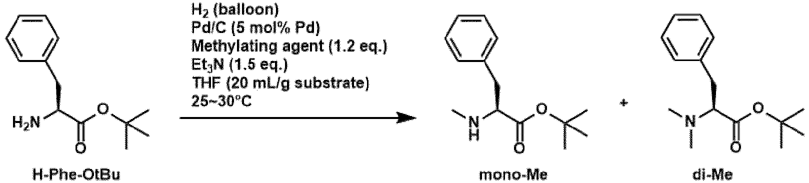

To a flask with a stirrer, 5% palladium carbon (50% wet, 72 mg, 0.017 mmol, 5 mol% on Pd metal basis) and a tetrahydrofuran solution of H-Phe-OtBu (20 mL/g substrate, 1.5 mL, 0.339 mmol) were added. TEA (71 µL, 0.508 mmol, 1.5 eq.) and a methylation reagent (0.407 mmol, 1.2 eq.) were sequentially added. While stirring, nitrogen replacement was performed, then hydrogen replacement was performed, and reaction was performed. The ratios of the raw material, a monomethylated compound, and a dimethylated compound were confirmed by HPLC analysis.

**[Table 18]**

| Reaction conditions | | Analysis result (%) | | |
|---|---|---|---|---|
| Reagent | Temperature (°C) | H-Phe-OtBu | mono-Me | di-Me |
| MOM-Cl | 25 | 15.7 | 82.6 | 1.8 |
| EOM-Cl | 25 | 5.4 | 91.5 | 3.1 |
| MEM-Cl | 25 | 11.1 | 87.2 | 1.8 |
| SEM-Cl | 25 | 11.4 | 87.6 | 1.1 |
| | 30 | 3.8 | 94.7 | 1.5 |

Measurement method: HPLC Method A
Retention time: H-Phe-OtBu: 2.75 min, mono-Me: 2.84 min, di-Me: 2.98 min
Mass spectrometry: H-Phe-OtBu: m/z 166.47 ([M-tBu+H]⁺), mono-Me: m/z 236.59 ([M+H]⁺), di-Me: m/z 250.61 ([M+H]⁺)

### (Example 18) Monomethylation reaction using SEM-Cl of H-Phe-OtBu as methylation reagent: 1 gram scale

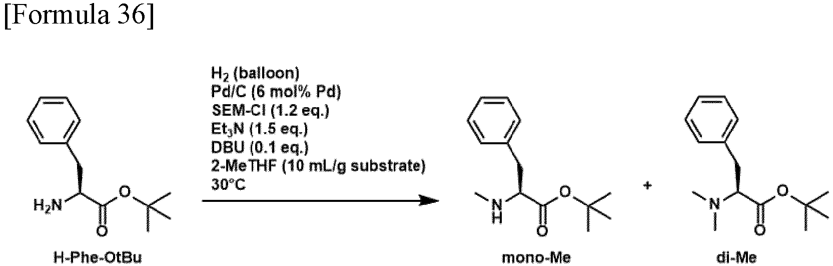

To a flask with a stirrer, 5% palladium carbon (50% wet, 1.154 g, 0.271 mmol, 6 mol% on Pd metal basis) and a 2-methyltetrahydrofuran solution of H-Phe-OtBu (10 mL/g substrate, 10.0 mL, 4.52 mmol) were added. TEA (0.945 mL, 6.78 mmol, 1.5 eq.) and DBU (68 µL, 0.452 mmol, 0.1 eq.) were sequentially added, and SEM-Cl (0.96 mL, 5.42 mmol, 1.2 eq.) was finally added. While stirring, nitrogen replacement was performed, then hydrogen replacement was performed, and reaction was performed at 30°C. Sampling was performed 8 hours later, and the ratios of the raw material, a monomethylated compound, and a dimethylated compound were confirmed by HPLC analysis.

**[Table 19]**

| Reaction conditions | | Analysis result (%) | | |
|---|---|---|---|---|
| Reagent (eq.) | Time (h) | H-Phe-OtBu | mono-Me | di-Me |
| SEM-Cl(1.2) | 8 | 2.4 | 95.8 | 1.8 |

Measurement method: HPLC Method A
Retention time: H-Phe-OtBu: 2.82 min, mono-Me: 2.90 min, di-Me: 3.06 min
Mass spectrometry: H-Phe-OtBu: m/z 166.42 ([M-tBu+H]⁺), mono-Me: m/z 236.53 ([M+H]⁺), di-Me: m/z 250.55 ([M+H]⁺)

### (Example 19) Monomethylation reaction of H-Phe-OtBu: Comparative experiment using aqueous formaldehyde as methylation reagent

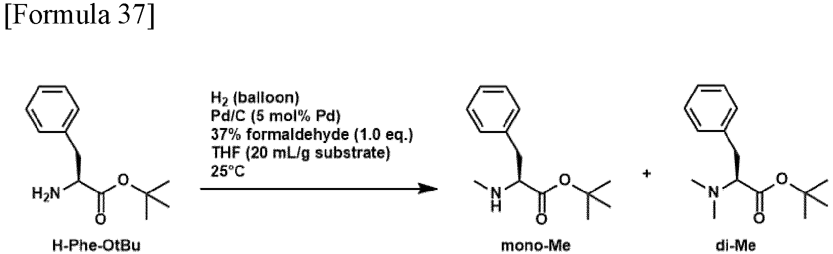

To a flask with a stirrer, H-Phe-OtBu (75 mg, 0.339 mmol) was added, and tetrahydrofuran (1.5 mL) was added. 5% palladium carbon (50% wet, 72 mg, 0.017 mmol, 5 mol% on Pd metal basis) and 37% aqueous formaldehyde (25.1 µL, 0.339 mmol, 1.0 eq.) were sequentially added. While stirring, nitrogen replacement was performed, then hydrogen replacement was performed, and reaction was performed at 25°C. Sampling was performed 6 hours later, and the ratios of the raw material, a monomethylated compound, and a dimethylated compound were confirmed by HPLC analysis.

**[Table 20]**

| Reaction conditions | | Analysis result (%) | | |
|---|---|---|---|---|
| Reagent (eq.) | Time (h) | H-Phe-OtBu | mono-Me | di-Me |
| 37% aqueous formaldehyde (1.0) | 6 | 21.4 | 63.9 | 14.8 |

Measurement method: HPLC Method A
Retention time: H-Phe-OtBu: 2.75 min, mono-Me: 2.85 min, di-Me: 2.98 min
Mass spectrometry: H-Phe-OtBu: m/z 166.47 ([M-tBu+H]⁺), mono-Me: m/z 236.59 ([M+H]⁺), di-Me: m/z 250.61 ([M+H]⁺)

### (Example 20) Evaluation of reducing agent other than hydrogen gas in monomethylation reaction of H-Phe-OtBu

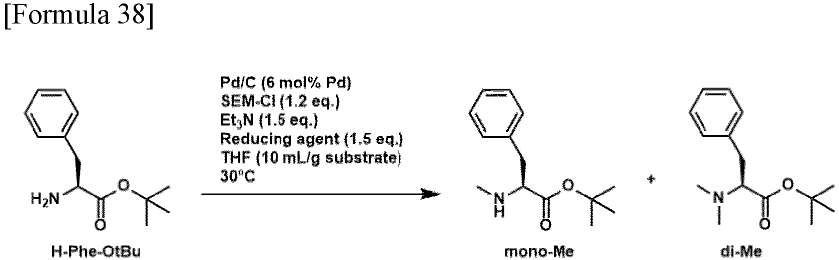

To a flask with a stirrer, 5% palladium carbon (50% wet, 231 mg, 0.054 mmol, 6 mol% on Pd metal basis) and a tetrahydrofuran solution of H-Phe-OtBu (10 mL/g substrate, 2.0 mL, 0.904 mmol) were added. TEA (189 µL, 1.356 mmol, 1.5 eq.) was added. SEM-Cl (192 µL, 1.085 mmol, 1.2 eq.) and a reducing agent (1.356 mmol, 1.5 eq.) were sequentially added. While stirring, nitrogen replacement was performed, and reaction was performed at 30°C. Sampling was performed 5 hours later, and the ratios of the raw material, a monomethylated compound, and a dimethylated compound and the amount of impurities were confirmed by HPLC analysis.

**[Table 21]**

| Reaction conditions | Analysis result (%) | | |
|---|---|---|---|
| Reducing agent | H-Phe-OtBu | mono-Me | di-Me |
| Triethylsilane | 2.2 | 94.1 | 3.7 |
| Triphenylsilane | 52.7 | 45.9 | 1.3 |
| Picoline borane | 67.1 | 32.2 | 0.8 |
| 1-Methyl-1,4-cyclohexadiene | 77.7 | nd | nd |

Measurement method: HPLC Method A
Retention time: H-Phe-OtBu: 2.80 min, mono-Me: 2.88 min, di-Me: 3.03 min
Mass spectrometry: H-Phe-OtBu: m/z 166.36 ([M-tBu+H]⁺), mono-Me: m/z 236.53 ([M+H]⁺), di-Me: m/z 250.55 ([M+H]⁺)

### (Example 21) One-pot implementation of deprotection of Cbz-Val-MeAsp(OtBu)-NMe₂ and monomethylation reaction

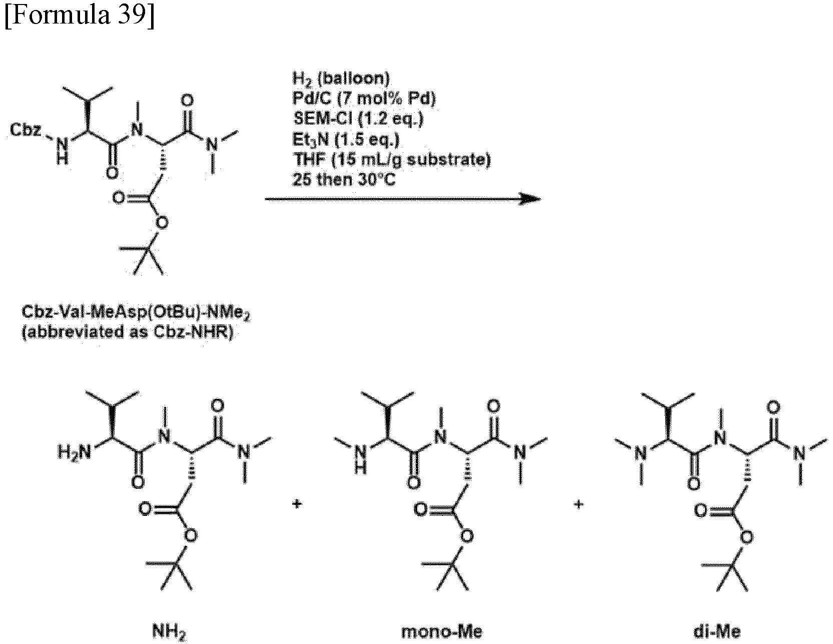

To a flask with a stirrer, 5% palladium carbon (50% wet, 46 mg, 0.004 mmol, 2 mol% on Pd metal basis) and a tetrahydrofuran solution of Cbz-Val-MeAsp(OtBu)-NMe₂ (15 mL/g substrate, 1.5 mL, 0.216 mmol) were added. While stirring, nitrogen replacement was performed, then hydrogen replacement was performed, and de-Cbz reaction was performed at 25°C. After 1 hour, the mixture was degassed under reduced pressure, and 5% palladium carbon (50% wet, 46 mg, 0.011 mmol, 5 mol% on Pd metal basis), TEA (45 µL, 0.324 mmol, 1.5 eq.), and SEM-Cl (46 µL, 0.259 mmol, 1.2 eq.) were sequentially added. While stirring, nitrogen replacement was performed, then hydrogen replacement was performed, and methylation reaction was performed at 30°C. Sampling was performed 8.5 hours later and 10 hours later, and the ratios of the raw material, a NH₂ compound, a monomethylated compound, and a dimethylated compound were confirmed by HPLC analysis.

**[Table 22]**

| Reaction conditions | Analysis result (%) | | | |
|---|---|---|---|---|
| Time (h) | Cbz-NHR | NH₂ | mono-Me | di-Me |
| 8.5 | nd | 5.9 | 90.7 | 0.5 |
| 10 | nd | 4.0 | 92.4 | 0.6 |

Measurement method: HPLC Method A
Retention time: NH₂: 2.34 min, mono-Me: 2.39 min, di-Me: 2.48 min
Mass spectrometry: NH₂: m/z 330.75 ([M+H]⁺), mono-Me: m/z 344.94 ([M+H]⁺), di-Me: m/z 358.80 ([M+H]⁺)

### (Example 22) One-pot implementation of deprotection of Cbz-Ala-EtPhe(4-Me)-Sar-OtBu and monomethylation reaction

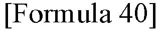

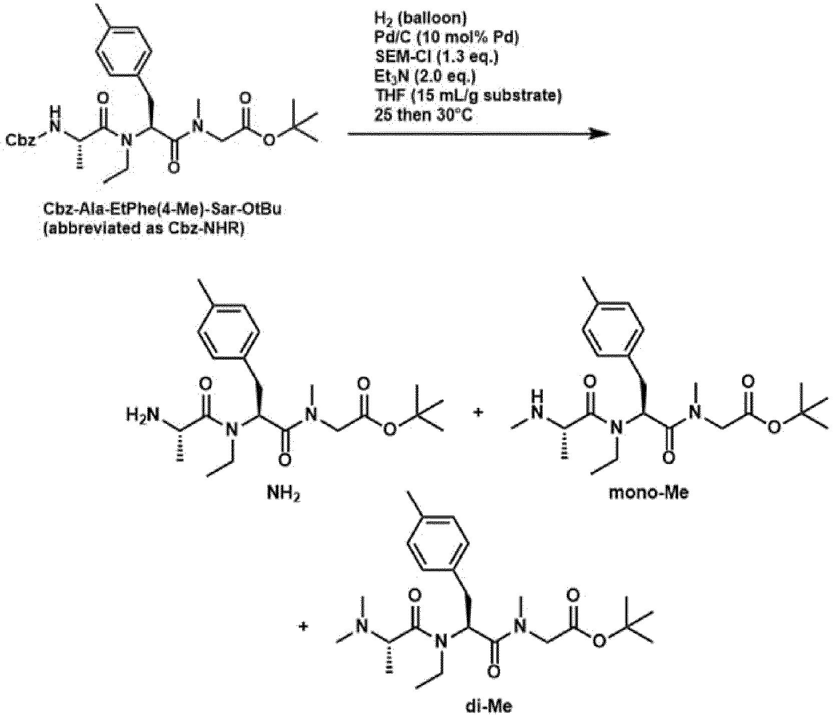

To a flask with a stirrer, 5% palladium carbon (50% wet, 21 mg, 0.005 mmol, 2 mol% on Pd metal basis) and a tetrahydrofuran solution of Cbz-Ala-EtPhe(4-Me)-Sar-OtBu (15 mL/g substrate, 2.0 mL, 0.250 mmol) were added. While stirring, nitrogen replacement was performed, then hydrogen replacement was performed, and de-Cbz reaction was performed at 25°C. After 1 hour, the mixture was degassed under reduced pressure, and 5% palladium carbon (50% wet, 85 mg, 0.020 mmol, 8 mol% on Pd metal basis), TEA (70 µL, 0.500 mmol, 2.0 eq.), and SEM-Cl (58 µL, 0.325 mmol, 1.3 eq.) were sequentially added. While stirring, nitrogen replacement was performed, then hydrogen replacement was performed, and methylation reaction was performed at 30°C. Sampling was performed 8 hours later and 10 hours later, and the ratios of the raw material, a NH₂ compound, a monomethylated compound, and a dimethylated compound were confirmed by HPLC analysis.

**[Table 23]**

| Reaction conditions | Analysis result (%) | | | |
|---|---|---|---|---|
| Time (h) | Cbz-NHR | NH₂ | mono-Me | di-Me |
| 8 | nd | nd | 95.5 | 0.8 |
| 10 | nd | nd | 95.0 | 1.0 |

Measurement method: HPLC Method A
Retention time: mono-Me: 3.33 min, di-Me: 3.40 min
Mass spectrometry: mono-Me: m/z 420.93 ([M+H]⁺), di-Me: m/z 434.89 ([M+H]⁺)

### (Example 23) One-pot implementation of deprotection of Cbz-Ala-EtPhe(4-Me)-Sar-OtBu and monomethylation reaction: Comparative experiment using aqueous formaldehyde as methylation reagent

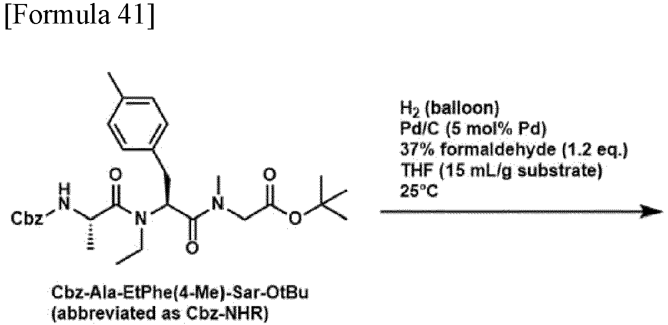

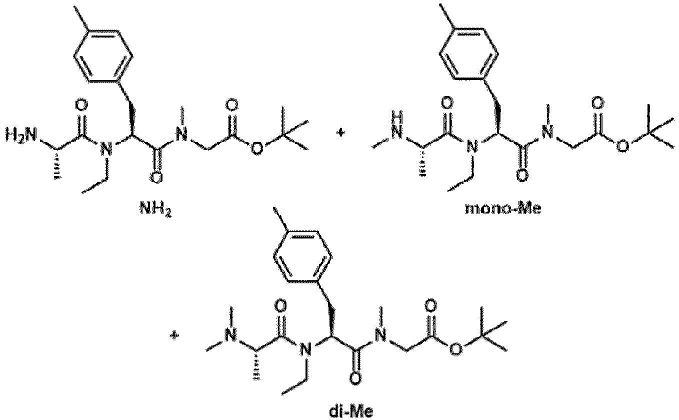

To a flask with a stirrer, 5% palladium carbon (50% wet, 53 mg, 0.013 mmol, 5 mol% on Pd metal basis) and a tetrahydrofuran solution of Cbz-Ala-EtPhe(4-Me)-Sar-OtBu (15 mL/g substrate, 2.0 mL, 0.250 mmol) were added. While stirring, nitrogen replacement was performed, then hydrogen replacement was performed, and de-Cbz reaction was performed at 25°C. After 1 hour, the mixture was degassed under reduced pressure, and 37% aqueous formaldehyde (22.2 µL, 0.300 mmol, 1.2 eq.) was added. While stirring, nitrogen replacement was performed, then hydrogen replacement was performed, and methylation reaction was performed at 25°C. Sampling was performed 7 hours later, and the ratios of the raw material, a NH₂ compound, a monomethylated compound, and a dimethylated compound were confirmed by HPLC analysis.

**[Table 24]**

| Reaction conditions | Analysis result (%) | | | |
|---|---|---|---|---|
| Time (h) | Cbz-NHR | NH₂ | mono-Me | di-Me |
| 7 | nd | nd | 95.4 | 2.6 |

Measurement method: HPLC Method A
Retention time: mono-Me: 3.34 min, di-Me: 3.41 min
Mass spectrometry: mono-Me: m/z 420.93 ([M+H]⁺), di-Me: m/z 434.84 ([M+H]⁺)

### (Example 24) One-pot implementation of deprotection of Cbz-Hph(3,5-F₂-4-CF₃)-Pro-cLeu-MeGcp-MeAsp(OtBu)-NMe₂ and monomethylation reaction

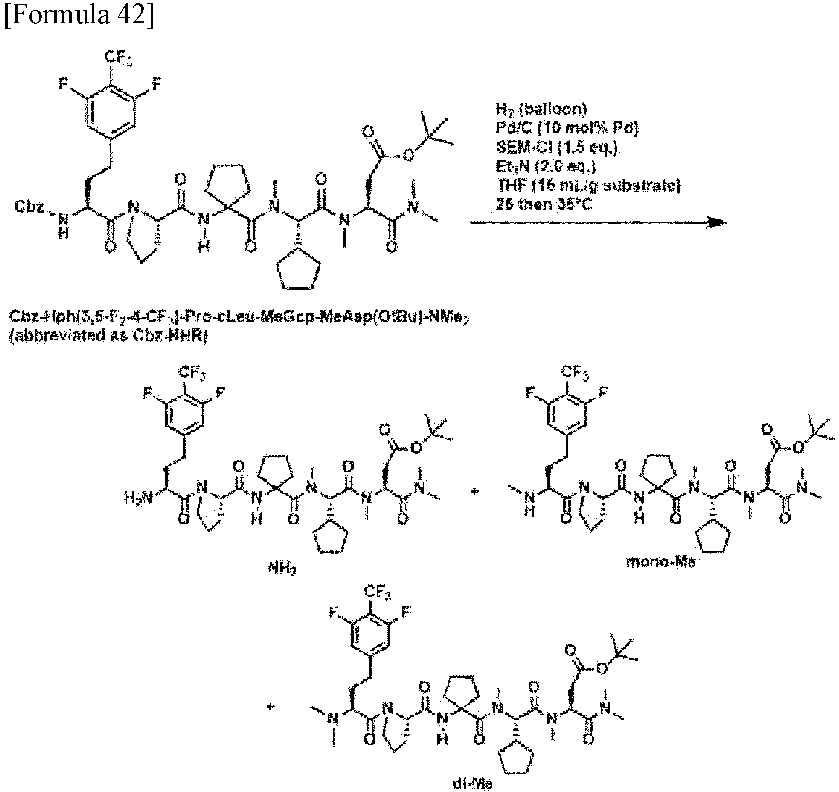

To a flask with a stirrer, 5% palladium carbon (50% wet, 12 mg, 0.003 mmol, 2 mol% on Pd metal basis) and a tetrahydrofuran solution of Cbz-Hph(3,5-F₂-4-CF₃)-Pro-cLeu-MeGcp-MeAsp(OtBu)-NMe₂ (15 mL/g substrate, 2.0 mL, 0.138 mmol) were added. While stirring, nitrogen replacement was performed, then hydrogen replacement was performed, and de-Cbz reaction was performed at 25°C. After 1 hour, the mixture was degassed under reduced pressure, and 5% palladium carbon (50% wet, 47 mg, 0.011 mmol, 8 mol% on Pd metal basis), TEA (39 µL, 0.276 mmol, 2.0 eq.), and SEM-CL (39 µL, 0.276 mmol, 1.5 eq.) were sequentially added. While stirring, nitrogen replacement was performed, then hydrogen replacement was performed, and methylation reaction was performed at 35°C. Sampling was performed 8 hours later, and the ratios of the raw material, a NH₂ compound, a monomethylated compound, and a dimethylated compound were confirmed by HPLC analysis.

**[Table 25]**

| Reaction conditions | Analysis result (%) | | | |
|---|---|---|---|---|
| Time (h) | Cbz-NHR | NH₂ | mono-Me | di-Me |
| 8 | nd | 0.1 | 97.0 | 2.2 |

Measurement method: HPLC Method A
Retention time: NH₂: 4.22 min, mono-Me: 4.28 min, di-Me: 4.40 min
Mass spectrometry: NH₂: m/z 843.93 ([M+H]⁺), mono-Me: m/z 858.35 ([M+H]⁺), di-Me: m/z 872.20 ([M+H]⁺)

### (Example 25) One-pot implementation of deprotection of Cbz-Hph(3,5-F₂-4-CF₃)-Pro-cLeu-MeGcp-MeAsp(OtBu)-NMe₂ and monomethylation reaction: Comparative experiment using aqueous formaldehyde as methylation reagent

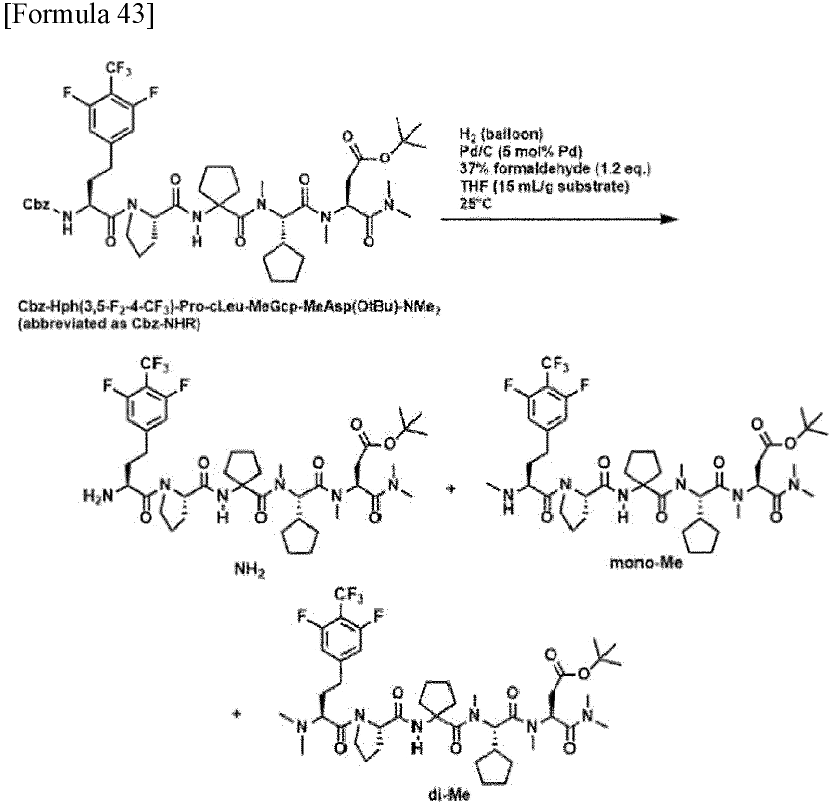

To a flask with a stirrer, 5% palladium carbon (50% wet, 29 mg, 0.007 mmol, 5 mol% on Pd metal basis) and a tetrahydrofuran solution of Cbz-Hph(3,5-F₂-4-CF₃)-Pro-cLeu-MeGcp-MeAsp(OtBu)-NMe₂ (15 mL/g substrate, 2.0 mL, 0.138 mmol) were added. While stirring, nitrogen replacement was performed, then hydrogen replacement was performed, and de-Cbz reaction was performed at 25°C. After 1 hour, the mixture was degassed under reduced pressure, and 37% aqueous formaldehyde (12.3 µL, 0.166 mmol, 1.2 eq.) was added. While stirring, nitrogen replacement was performed, then hydrogen replacement was performed, and methylation reaction was performed at 25°C. Sampling was performed 7 hours later, and the ratios of the raw material, a NH₂ compound, a monomethylated compound, and a dimethylated compound were confirmed by HPLC analysis.

**[Table 26]**

| Reaction conditions | Analysis result (%) | | | |
|---|---|---|---|---|
| Time (h) | Cbz-NHR | NH₂ | mono-Me | di-Me |
| 7 | 2.3 | 7.2 | 57.2 | 33.0 |

Measurement method: HPLC Method A
Retention time: Cbz-NHR: 6.00 min, NH₂: 4.21 min, mono-Me: 4.29 min, di-Me: 4.38 min
Mass spectrometry: Cbz-NHR: m/z 1000.23 ([M+Na]⁺), NH₂: m/z 844.16 ([M+H]⁺), mono-Me: m/z 858.35 ([M+H]⁺), di-Me: m/z 872.43 ([M+H]⁺)

### (Example 26) Monopropylation of H-Phe-OtBu: Example using triethylsilane as reducing agent

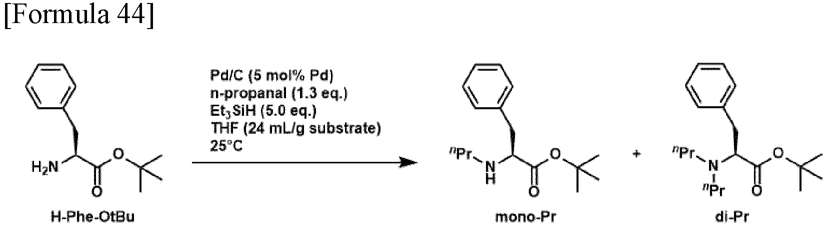

To a flask with a stirrer, H-Phe-OtBu (84 mg, 0.380 mmol) was added, and tetrahydrofuran (2.0 mL) was added. 5% palladium carbon (50% wet, 82 mg, 0.015 mmol, 5 mol% on Pd metal basis) was added. n-Propanal (36 µL, 0.494 mmol, 1.3 eq.) and triethylsilane (303 µL, 1.899 mmol, 5.0 eq.) were sequentially added, then while stirring, nitrogen replacement was performed, and reaction was performed at 25°C. Sampling was performed 2.5 hours later, and the ratios of the raw material, a NH₂ compound, a monopropylated compound, and a dipropylated compound were confirmed by HPLC analysis.

**[Table 27]**

| Reaction conditions | Analysis result (%) | | |
|---|---|---|---|
| Time (h) | H-Phe-OtBu | mono-Pr | di-Pr |
| 2.5 | 0.2 | 92.0 | 5.8 |

Measurement method: HPLC Method A
Retention time: H-Phe-OtBu: 2.70 min, mono-Pr: 3.12 min, di-Pr: 3.61 min
Mass spectrometry: H-Phe-OtBu: m/z 166.47 ([M-tBu+H]⁺), mono-Pr: m/z 264.74 ([M+H]⁺), di-Pr: m/z 306.81 ([M+H]⁺)

### (Example 27) One-pot implementation of deprotection of Cbz-Ala-EtPhe(4-Me)-Sar-OtBu and monopropylation reaction

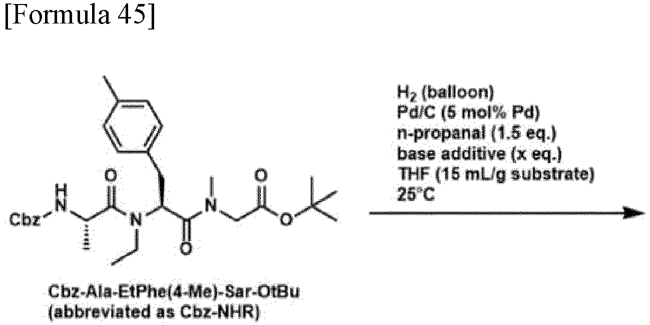

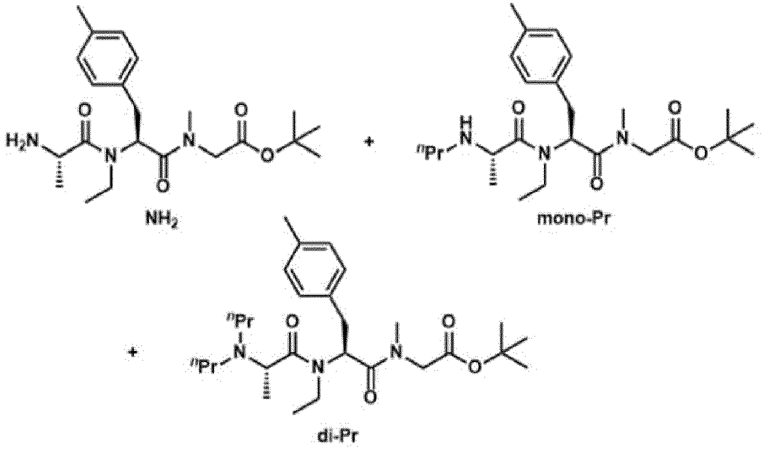

To a flask with a stirrer, 5% palladium carbon (50% wet, 53 mg, 0.013 mmol, 5 mol% on Pd metal basis) and a tetrahydrofuran solution of Cbz-Ala-EtPhe(4-Me)-Sar-OtBu (15 mL/g substrate, 2.0 mL, 0.250 mmol) were added. While stirring, nitrogen replacement was performed, then hydrogen replacement was performed, and de-Cbz reaction was performed. After 1 hour, the mixture was degassed under reduced pressure, and n-propanal (27 µL, 0.375 mmol, 1.5 eq.) was added. While stirring, nitrogen replacement was performed, then hydrogen replacement was performed, and propylation reaction was performed. Sampling was performed 5 hours later, and the ratios of the raw material, a NH₂ compound, a monopropylated compound, and a dipropylated compound and the amount of impurities were confirmed by HPLC analysis. Two experiments depending on the presence and absence of addition of DBU (3.7 µL, 0.025 mmol, 0.1 eq.) immediately before the n-propanal addition were carried out, and the results were compared.

**[Table 28]**

| Reaction conditions | | | Analysis result (%) | | | |
|---|---|---|---|---|---|---|
| Base | Equivalent (eq.) | Time (h) | Cbz-NHR | NH₂ | mono-Pr | di-Pr |
| none | NA | 5 | nd | nd | 74.6 | 23.5 |
| DBU | 0.1 | 5 | nd | nd | 96.9 | 1.5 |

Measurement method: HPLC Method A
Retention time: NH₂: 3.30 min, mono-Pr: 3.59 min, di-Pr: 3.95 min
Mass spectrometry: NH₂: m/z 406.85 ([M+H]⁺), mono-Pr: m/z 449.03 ([M+H]⁺), di-Pr: m/z 490.92 ([M+H]⁺)

### (Example 28) One-pot implementation of deprotection of Cbz-Hph(3,5-F₂-4-CF₃)-Pro-cLeu-MeGcp-MeAsp(OtBu)-NMe₂ and monopropylation reaction

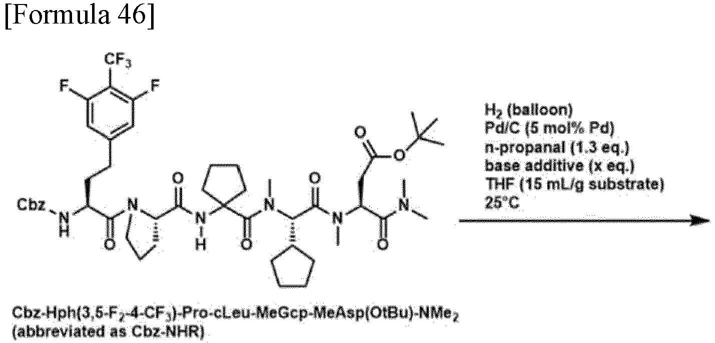

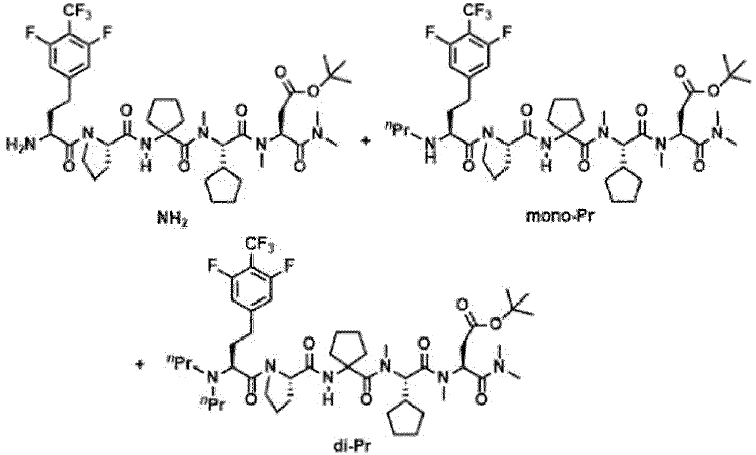

To a flask with a stirrer, 5% palladium carbon (50% wet, 29 mg, 0.014 mmol, 5 mol% on Pd metal basis) and a tetrahydrofuran solution of Cbz-Hph(3,5-F₂-4-CF₃)-Pro-cLeu-MeGcp-MeAsp(OtBu)-NMe₂ (15 mL/g substrate, 2.0 mL, 0.138 mmol) were added. While stirring, nitrogen replacement was performed, then hydrogen replacement was performed, and de-Cbz reaction was performed. After 1 hour, the mixture was degassed under reduced pressure, and n-propanal (13 µL, 0.180 mmol, 1.3 eq.) was added. While stirring, nitrogen replacement was performed, then hydrogen replacement was performed, and propylation reaction was performed. Sampling was performed 7 hours later, and the ratios of the raw material, a NH₂ compound, a monopropylated compound, and a dipropylated compound and the amount of impurities were confirmed by HPLC analysis. Two experiments depending on the presence and absence of addition of DBU (2.1 µL, 0.014 mmol, 0.1 eq.) immediately before the n-propanal addition were carried out, and the results were compared.

**[Table 29]**

| Reaction conditions | | | Analysis result (%) | | | |
|---|---|---|---|---|---|---|
| Base | Equivalent (eq.) | Time (h) | Cbz-NHR | NH₂ | mono-Pr | di-Pr |
| none | NA | 7 | nd | nd | 94.8 | 3.0 |
| DBU | 0.1 | 7 | nd | nd | 99.3 | 0.8 |

Measurement method: HPLC Method A
Retention time: mono-Pr: 4.47 min, di-Pr: 4.79 min
Mass spectrometry: mono-Pr: m/z 886.34 ([M+H]⁺), di-Pr: m/z 928.41 ([M+H]⁺)

### (Example 29) One-pot implementation of deprotection of Cbz-Val-MeAsp(OtBu)-NMe₂ and monobutylation reaction

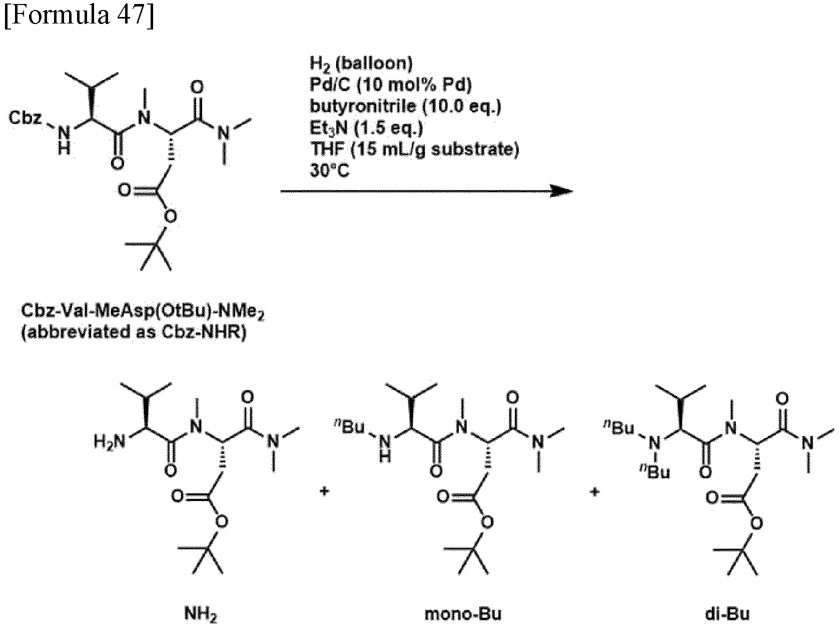

To a flask with a stirrer, 5% palladium carbon (50% wet, 124 mg, 0.029 mmol, 10 mol% on Pd metal basis) and a tetrahydrofuran solution of Cbz-Val-MeAsp(OtBu)-NMe₂ (15 mL/g substrate, 2.0 mL, 0.291 mmol) were added. Butyronitrile (253 µL, 2.91 mmol, 10.0 eq.) was added, then while stirring, nitrogen replacement was performed, then hydrogen replacement was performed, and reaction was performed at 30°C. Sampling was performed 8 hours later, and the ratios of the raw material, a NH₂ compound, a monobutylated compound, and a dibutylated compound were confirmed by HPLC analysis.

**[Table 30]**

| Reaction conditions | Analysis result (%) | | | |
|---|---|---|---|---|
| Time (h) | Cbz-NHR | NH₂ | mono-Bu | di-Bu |
| 8 | nd | 3.3 | 96.0 | 0.1 |

Measurement method: HPLC Method A
Retention time: NH₂: 2.28 min, mono-Bu: 2.86 min, di-Bu: 3.49 min
Mass spectrometry: NH₂: m/z 330.75 ([M+H]⁺), mono-Bu: m/z 386.90 ([M+H]⁺), di-Bu: m/z 442.99 ([M+H]⁺)

### (Example 30) One-pot implementation of deprotection of Cbz-Ala-EtPhe(4-Me)-Sar-OtBu and monobutylation reaction

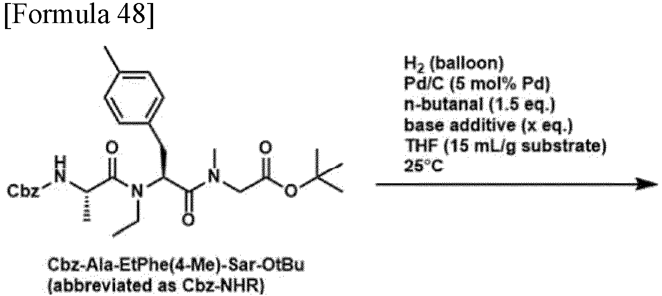

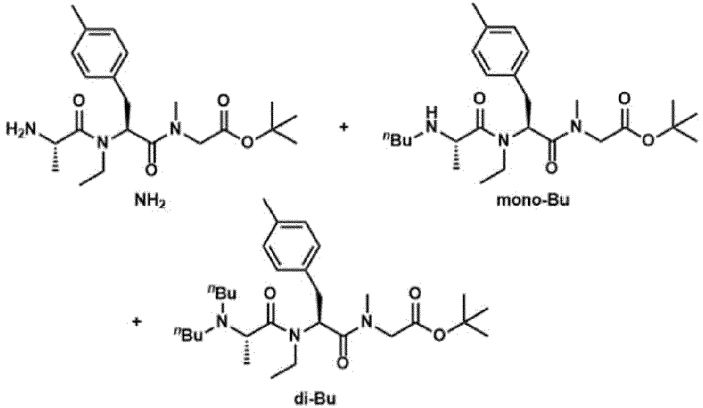

To a flask with a stirrer, 5% palladium carbon (50% wet, 53 mg, 0.013 mmol, 5 mol% on Pd metal basis) and a tetrahydrofuran solution of Cbz-Ala-EtPhe(4-Me)-Sar-OtBu (15 mL/g substrate, 2.0 mL, 0.250 mmol) were added. While stirring, nitrogen replacement was performed, then hydrogen replacement was performed, and de-Cbz reaction was performed. After 1 hour, the mixture was degassed under reduced pressure, and n-butanal (33 µL, 0.375 mmol, 1.5 eq.) was added. While stirring, nitrogen replacement was performed, then hydrogen replacement was performed, and butylation reaction was started. Sampling was performed 5 hours later, and the ratios of the raw material, a NH₂ compound, a monobutylated compound, and a dibutylated compound and the amount of impurities were confirmed by HPLC analysis. Two experiments depending on the presence and absence of addition of DBU (3.7 µL, 0.025 mmol, 0.1 eq.) immediately before the n-butanal addition were carried out, and the results were compared.

**[Table 31]**

| Reaction conditions | | | Analysis result (%) | | | |
|---|---|---|---|---|---|---|
| Base | Equivalent (eq.) | Time (h) | Cbz-NHR | NH₂ | mono-Bu | di-Bu |
| none | NA | 5 | nd | 0.1 | 69.1 | 29.6 |
| DBU | 0.1 | 5 | nd | 0.4 | 97.9 | 0.5 |

Measurement method: HPLC Method A
Retention time: NH₂: 3.30 min, mono-Bu: 3.80 min, di-Bu: 4.35 min
Mass spectrometry: NH₂: m/z 406.85 ([M+H]⁺), mono-Bu: m/z 463.11 ([M+H]⁺), di-Bu: m/z 519.03 ([M+H]⁺)

### (Example 31) One-pot implementation of deprotection of Cbz-Hph(3,5-F₂-4-CF₃)-Pro-cLeu-MeGcp-MeAsp(OtBu)-NMe₂ and monobutylation reaction

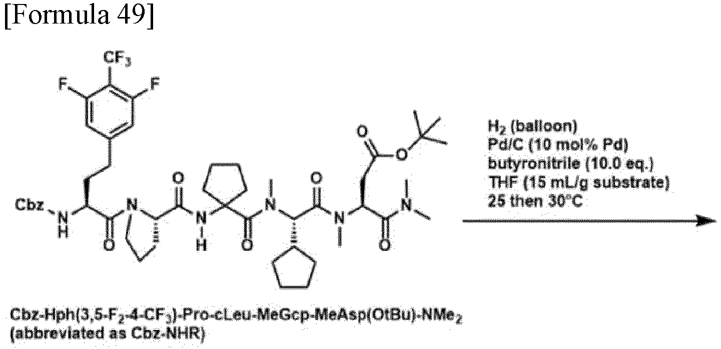

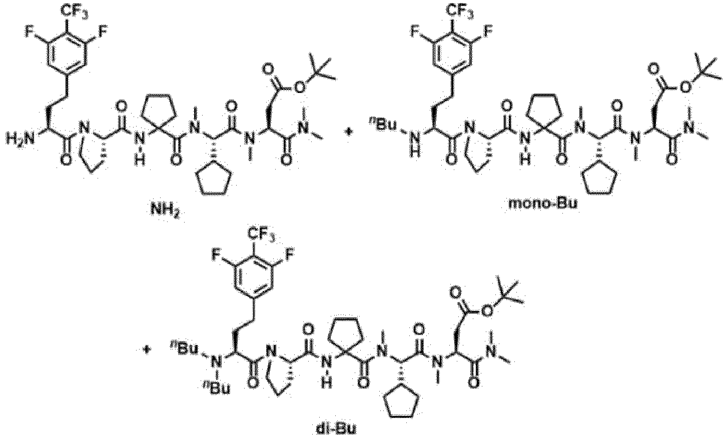

To a flask with a stirrer, 5% palladium carbon (50% wet, 12 mg, 0.003 mmol, 2 mol% on Pd metal basis) and a tetrahydrofuran solution of Cbz-Hph(3,5-F₂-4-CF₃)-Pro-cLeu-MeGcp-MeAsp(OtBu)-NMe₂ (15 mL/g substrate, 2.0 mL, 0.138 mmol) were added. While stirring, nitrogen replacement was performed, then hydrogen replacement was performed, and de-Cbz reaction was performed at 25°C. After 1 hour, the mixture was degassed under reduced pressure, and 5% palladium carbon (50% wet, 47 mg, 0.011 mmol, 8 mol% on Pd metal basis) and butyronitrile (120 µL, 1.382 mmol, 10.0 eq.) were sequentially added. While stirring, nitrogen replacement was performed, then hydrogen replacement was performed, and butylation reaction was performed at 30°C. Sampling was performed 10 hours later, and the ratios of the raw material, a NH₂ compound, a monobutylated compound, and a dibutylated compound were confirmed by HPLC analysis.

**[Table 32]**

| | | | | |
|---|---|---|---|---|
| | | | | |

| Reaction conditions | Analysis result (%) | | | |
|---|---|---|---|---|
| Time (h) | Cbz-NHR | NH₂ | mono-Bu | di-Bu |
| 10 | nd | nd | 99.6 | nd |

Measurement method: HPLC Method A
Retention time: mono-Bu: 4.56 min
Mass spectrometry: mono-Bu: m/z 900.31 ([M+H]⁺)

### (Example 32) One-pot implementation of deprotection of Cbz-Val-MeAsp(OtBu)-NMe₂ and monohexylation reaction

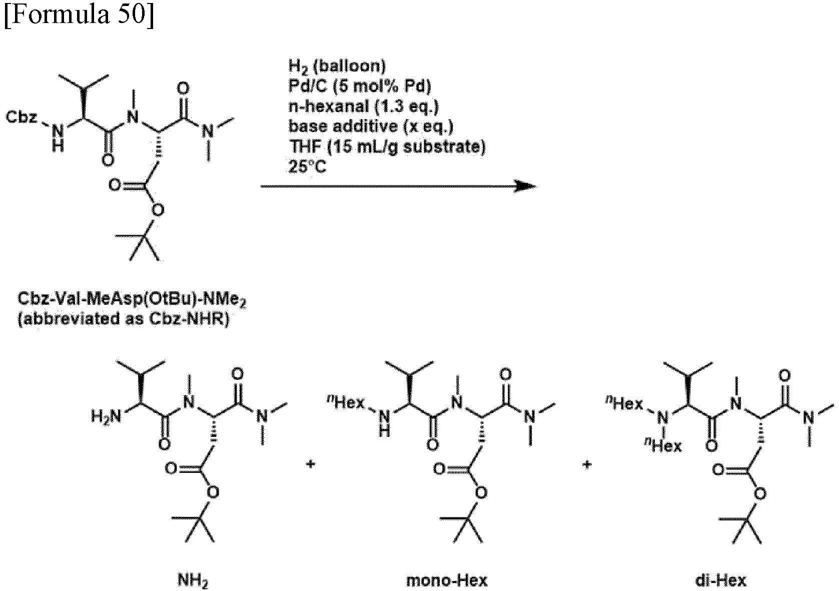

To a flask with a stirrer, 5% palladium carbon (50% wet, 62 mg, 0.015 mmol, 5 mol% on Pd metal basis) and a tetrahydrofuran solution of Cbz-Val-MeAsp(OtBu)-NMe₂ (15 mL/g substrate, 2.0 mL, 0.291 mmol) were added. While stirring, nitrogen replacement was performed, then hydrogen replacement was performed, and de-Cbz reaction was performed. After 1 hour, the mixture was degassed under reduced pressure, and n-hexanal (46 µL, 0.379 mmol, 1.3 eq.) was added. While stirring, nitrogen replacement was performed, then hydrogen replacement was performed, and hexylation reaction was performed at 25°C. Sampling was performed 6 hours later, and the ratios of the raw material, a NH₂ compound, a monohexylated compound, and a dihexylated compound and the amount of impurities were confirmed by HPLC analysis. Two experiments depending on the presence and absence of addition of DBU (4.4 µL, 0.029 mmol, 0.1 eq.) immediately before the n-hexanal addition were carried out, and the results were compared.

**[Table 33]**

| Reaction conditions | | | Analysis result (%) | | | |
|---|---|---|---|---|---|---|
| Base | Equivalent (eq.) | Time (h) | Cbz-NHR | NH₂ | mono-Hex | di-Hex |
| none | NA | 6 | nd | nd | 75.7 | 22.7 |
| DBU | 0.1 | 6 | nd | 22.2 | 76.8 | nd |

Measurement method: HPLC Method A
Retention time: NH₂: 2.32 min, mono-Hex: 3.42 min, di-Hex: 4.47 min
Mass spectrometry: NH₂: m/z ([M+H]⁺), mono-Hex: m/z 415.11 ([M+H]⁺), di-Hex: m/z499.02 ([M+H]⁺)

### (Example 33) One-pot implementation of deprotection of Cbz-Val-MeAsp(OtBu)-NMe₂ and monohexylation reaction: Example using triethylsilane as reducing agent

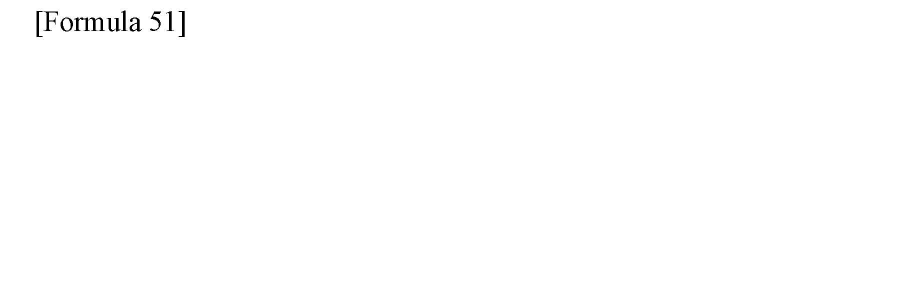

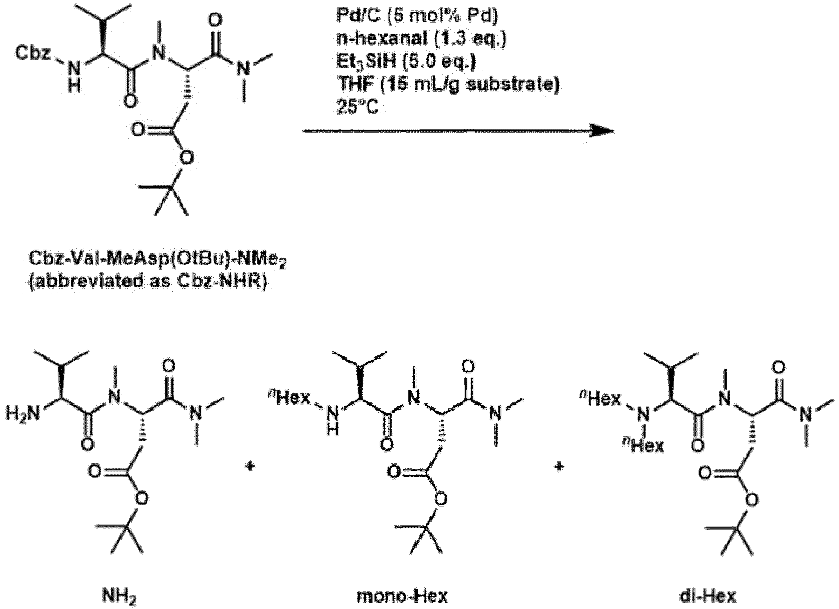

To a flask with a stirrer, 5% palladium carbon (50% wet, 62 mg, 0.015 mmol, 5 mol% on Pd metal basis) and a tetrahydrofuran solution of Cbz-Val-MeAsp(OtBu)-NMe₂ (15 mL/g substrate, 2.0 mL, 0.291 mmol) were added. n-Hexanal (46 µL, 0.379 mmol, 1.3 eq.) and triethylsilane (233 µL, 1.456 mmol, 5.0 eq.) were sequentially added, then while stirring, nitrogen replacement was performed, and reaction was performed at 25°C. Sampling was performed 7 hours later, and the ratios of the raw material, a NH₂ compound, a monohexylated compound, and a dihexylated compound and the amount of impurities were confirmed by HPLC analysis.

**[Table 34]**

| Reaction conditions | Analysis result (%) | | | |
|---|---|---|---|---|
| Time (h) | Cbz-NHR | NH₂ | mono-Hex | di-Hex |
| 7 | nd | nd | 95.5 | 4.1 |

Measurement method: HPLC Method A
Retention time: mono-Hex: 3.44 min, di-Hex: 4.47 min
Mass spectrometry: mono-Hex: m/z 415.00 ([M+H]⁺), di-Hex: m/z 499.02 ([M+H]⁺)

### (Example 34) One-pot implementation of deprotection of Cbz-Hph(3,5-F₂-4-CF₃)-Pro-cLeu-MeGcp-MeAsp(OtBu)-NMe₂ and monohexylation reaction

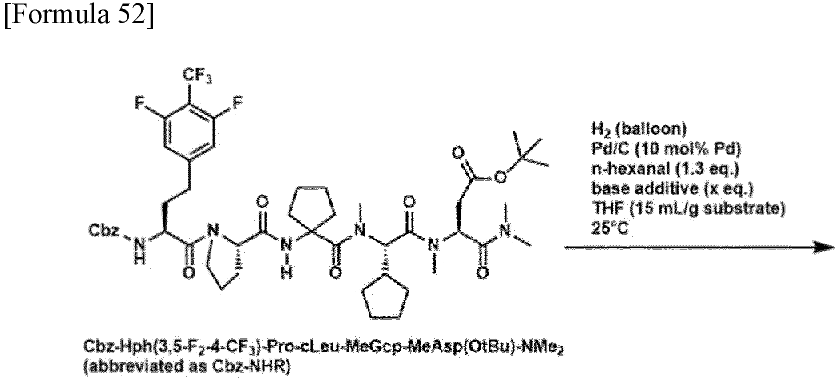

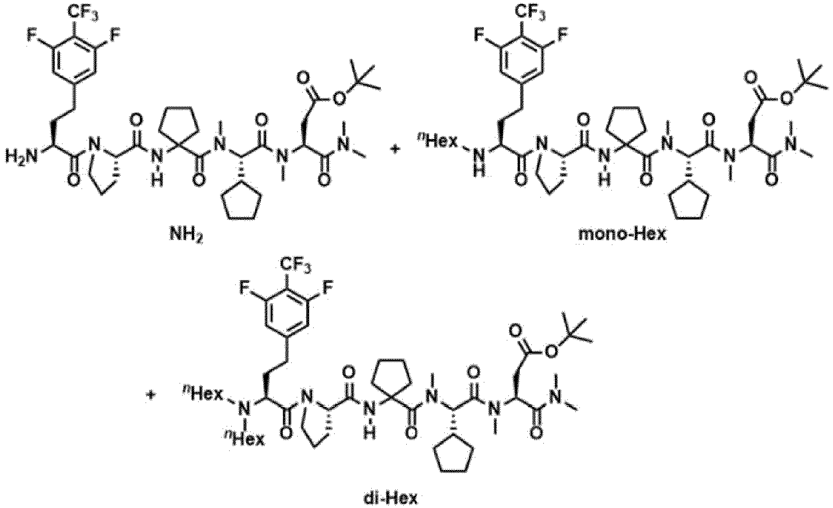

To a flask with a stirrer, 5% palladium carbon (50% wet, 29 mg, 0.014 mmol, 5 mol% on Pd metal basis) and a tetrahydrofuran solution of Cbz-Hph(3,5-F₂-4-CF₃)-Pro-cLeu-MeGcp-MeAsp(OtBu)-NMe₂ (15 mL/g substrate, 2.0 mL, 0.138 mmol) were added. While stirring, nitrogen replacement was performed, then hydrogen replacement was performed, and de-Cbz reaction was performed. After 1 hour, the mixture was degassed under reduced pressure, and n-hexanal (22 µL, 0.180 mmol, 1.3 eq.) was added. While stirring, nitrogen replacement was performed, then hydrogen replacement was performed, and hexylation reaction was performed at 25°C. Sampling was performed 6 hours later, and the ratios of the raw material, a NH₂ compound, a monohexylated compound, and a dihexylated compound and the amount of impurities were confirmed by HPLC analysis. Two experiments depending on the presence and absence of addition of DBU (2.1 µL, 0.014 mmol, 0.1 eq.) immediately before the n-hexanal addition were carried out, and the results were compared.

**[Table 35]**

| Reaction conditions | | | Analysis result (%) | | | |
|---|---|---|---|---|---|---|
| Base | Equivalent (eq.) | Time (h) | Cbz-NHR | NH₂ | mono-Hex | di-Hex |
| none | NA | 6 | nd | nd | 86.4 | 10.0 |
| DBU | 0.1 | 6 | nd | nd | 98.3 | 1.4 |

Measurement method: HPLC Method A
Retention time: mono-Hex: 4.87 min, di-Hex: 5.54 min
Mass spectrometry: mono-Hex: m/z 928.41 ([M+H]⁺), di-Hex: m/z 1012.54 ([M+H]⁺)

### (Example 35) Monoethylation reaction of H-Phe(4-Me)-OH

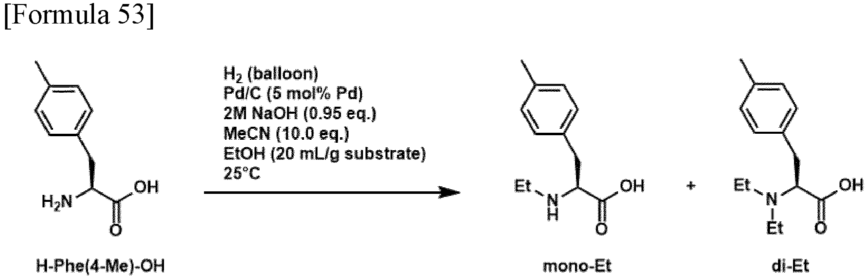

To a flask with a stirrer, H-Phe(4-Me)-OH (100 mg, 0.558 mmol) was added, and ethanol (2.0 mL) was added. To a solution obtained by dissolving the substrate by the addition of a 2 M aqueous NaOH solution (0.265 mL, 0.530 mmol, 0.95 eq.), 5% palladium carbon (50% wet, 119 mg, 0.028 mmol, 5 mol% on Pd metal basis) and acetonitrile (0.291 mL, 5.58 mmol, 10.0 eq.) were added. While stirring, nitrogen replacement was performed, then hydrogen replacement was performed, and ethylation reaction was performed at 30°C. Sampling was performed 7 hours later, and the ratios of the raw material, a NH₂ compound, a monoethylated compound, and a diethylated compound were confirmed by HPLC analysis.

**[Table 36]**

| Reaction conditions | Analysis result (%) | | |
|---|---|---|---|
| Time (h) | H-Phe(4-Me)-OH | mono-Et | di-Et |
| 7 | nd | 80.9 | 18.1 |

Measurement method: HPLC Method A
Retention time: mono-Et: 2.00 min, di-Et: 2.29 min
Mass spectrometry: mono-Et: m/z 208.77 ([M+H]⁺), di-Et: m/z 236.76 ([M+H]⁺)

### (Example 36) Monopropylation reaction of H-Phe(4-Me)-OH

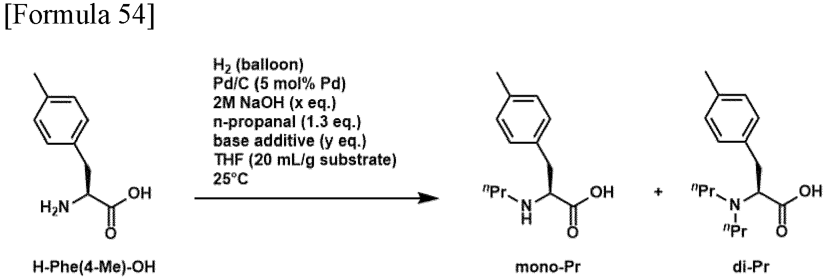

To a flask with a stirrer, H-Phe(4-Me)-OH (100 mg, 0.558 mmol) was added, and tetrahydrofuran (2.0 mL) was added. To a solution obtained by dissolving the substrate by the addition of a 2 M aqueous NaOH solution (x eq., see Table 37), 5% palladium carbon (50% wet, 119 mg, 0.028 mmol, 5 mol% on Pd metal basis) was added. A basic additive (y eq., see Table 37) and n-propanal (52 µL, 0.725 mmol, 1.3 eq.) were added. While stirring, nitrogen replacement was performed, then hydrogen replacement was performed, and propylation reaction was performed at 25°C. The ratios of the raw material, a NH₂ compound, a monoethylated compound, and a diethylated compound were confirmed by HPLC analysis. The equivalent of NaOH and the effect of addition of Et₃N (78 µL, 1.0 eq., 0.558 mmol) or DBU (8.3 µL, 0.056 mmol, 0.1 eq.) were evaluated.

**[Table 37]**

| Reaction conditions | | Analysis result (%) | | |
|---|---|---|---|---|
| NaOH (x eq.) | Basic additive (y eq.) | H-Phe(4-Me)-OH | mono-Pr | di-Pr |
| 0.95 | none | 20.6 | 34.4 | 40.8 |
| 1.05 | none | 12.7 | 68.8 | 8.9 |
| 0.95 | Et₃N (1.0) | 10.9 | 54.1 | 29.6 |
| 0.95 | DBU (0.1) | 2.8 | 81.7 | 7.6 |

Measurement method: HPLC Method A
Retention time: H-Phe (4-Me)-OH: 1.88 min, mono-Pr: 2.23 min, di-Pr: 3.00 min
Mass spectrometry: H-Phe (4-Me)-OH: m/z 180.50 ([M+H]⁺), mono-Pr: m/z 222.68 ([M+H]⁺), di-Pr: m/z 264.63 ([M+H]⁺)

### (Synthesis of raw material 5) Synthesis of Cbz-Val-MeAsp(OtBu)-NMe₂

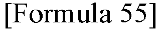

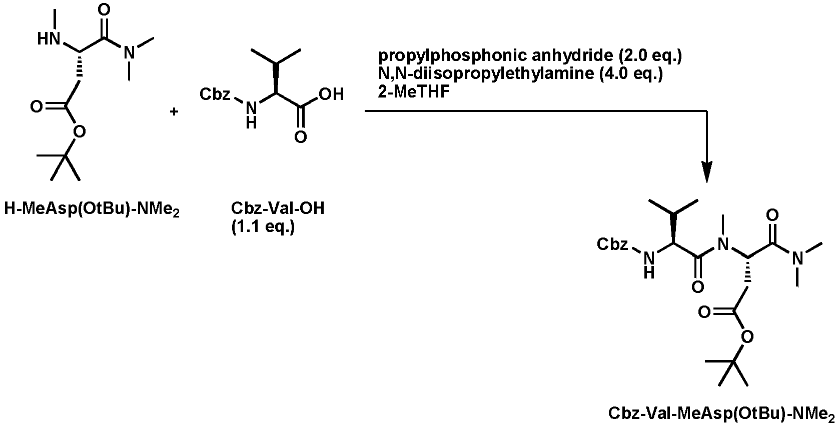

A solution of H-MeAsp(OtBu)-NMe2 obtained by the synthesis method described in Example 49 was concentrated under reduced pressure for the removal of the solvent to obtain 4.26 g of an oil. A solution was prepared by an addition of 2-methyltetrahydrofuran (43 mL), and Cbz-Val-OH (5.11 g, 20.35 mmol, 1.1 eq.) was added. The flask was cooled with an ice bath, and DIPEA (12.9 mL, 74.0 mmol, 4.0 eq.) was added. A solution of propylphosphonic acid anhydride in 2-methyltetrahydrofuran (1.6 M, 23 mL, 37.0 mmol, 2.0 eq.) was added dropwise over 10 minutes with a syringe. The internal temperature during the dropwise addition was kept at 8.0 to 19.0°C. Sampling was performed 1 hour after the completion of the dropwise addition, and the completion of the reaction was confirmed by HPLC analysis. A 5% aqueous sodium carbonate solution (34 mL) was added dropwise, then the mixture was stirred, then transferred to a separatory funnel, and allowed to stand still, and the aqueous layer was removed. A 5% aqueous sodium bisulfate solution (34 mL) was added, then the mixture was shaken well and then allowed to stand still, and the aqueous layer was removed. The washing with a 5% aqueous sodium bisulfate solution (34 mL) was repeated one more time. A 5% aqueous sodium carbonate solution (34 mL) was added, then the mixture was shaken well and then allowed to stand still, and the aqueous layer was removed. The obtained organic layer was concentrated under reduced pressure conditions to obtain 9.0 g of a crude product. The obtained crude product was purified by silica gel column chromatography to obtain 7.0 g of Cbz-Val-MeAsp(OtBu)-NMe₂.
Yield: 82%
HPLC purity: 100%
Measurement method: HPLC Method A
Retention time: 4.45 min
Mass spectrometry: m/z486.76 ([M+Na]⁺)

### (Synthesis of raw material 6) Synthesis of Cbz-Ala-EtPhe(4-Me)-Sar-OtBu

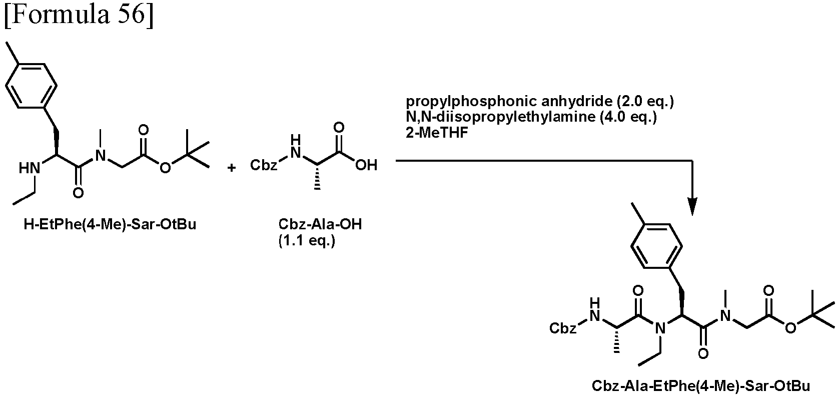

In a separatory funnel, H-EtPhe(4-Me)-Sar-OtBu hydrochloride (6.0 g, 16.18 mmol) obtained by the synthesis method described in Example 38 was suspended in 2-methyltetrahydrofuran (100 mL), then the suspension was washed with a 5% aqueous sodium carbonate solution (100 mL), and the aqueous layer was removed. The washing with a 5% aqueous sodium carbonate solution (100 mL) was repeated one more time. The obtained organic layer was concentrated under reduced pressure conditions to obtain 4.81 g of H-EtPhe(4-Me)-Sar-OtBu. A solution was prepared by the addition of 2-methyltetrahydrofuran (48 mL), and Cbz-Ala-OH (3.52 g, 15.79 mmol, 1.1 eq.) was added. DIPEA (10.0 mL, 57.4 mmol, 4.0 eq.) was added, and a solution of propylphosphonic acid anhydride in 2-methyltetrahydrofuran (1.6 M, 18 mL, 28.7 mmol, 2.0 eq.) was added dropwise over 10 minutes with a syringe. After completion of the dropwise addition, the mixture was stirred at room temperature for 3 hours. A 5% aqueous sodium carbonate solution (40 mL) was added dropwise, then the mixture was stirred, then transferred to a separatory funnel, and allowed to stand still, and the aqueous layer was removed. Washing with a 5% aqueous sodium bisulfate solution (40 mL) was carried out four times, and then washing with a 5% aqueous sodium carbonate solution (40 mL) was carried out twice. The obtained organic layer was concentrated under reduced pressure conditions to obtain 5.26 g of a crude product. The obtained crude product was purified by silica gel column chromatography to obtain 3.14 g of Cbz-Ala-EtPhe(4-Me)-Sar-OtBu.
Yield: 40%
HPLC purity: 99.5%
Measurement method: HPLC Method A
Retention time: 5.50 min
Mass spectrometry: m/z562.86 ([M+Na]⁺)

### (Synthesis of raw material 7) Cbz-Hph(3,5-F₂-4-CF₃)-Pro-cLeu-MeGcp-MeAsp(OtBu)-NMe₂

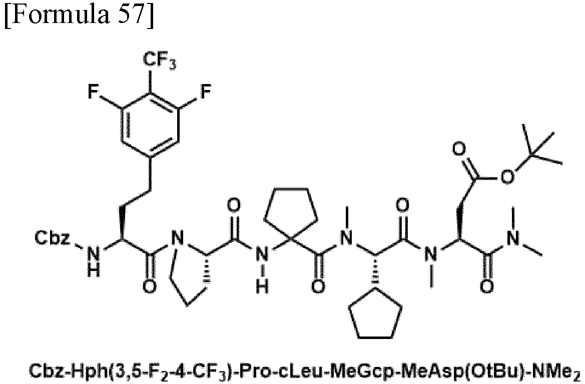

A solution of Cbz-Hph(3,5-F₂-4-CF₃)-Pro-cLeu-MeGcp-MeAsp(OtBu)-NMe₂ obtained by the synthesis method described in Example 59 was concentrated under reduced pressure for the removal of the solvent to obtain 6.1 g of oil. The obtained crude product was purified by silica gel column chromatography to obtain 4.4 g of Cbz-Hph(3,5-F₂-4-CF₃)-Pro-cLeu-MeGcp-MeAsp(OtBu)-NMe₂.
Yield: 88%
HPLC purity: 100%
Measurement method: HPLC Method A
Retention time: 6.00 min
Mass spectrometry: m/z1000.23 ([M+Na]⁺)

### (Example 37) Synthesis of Cbz-Phe(4-Me)-Sar-OtBu

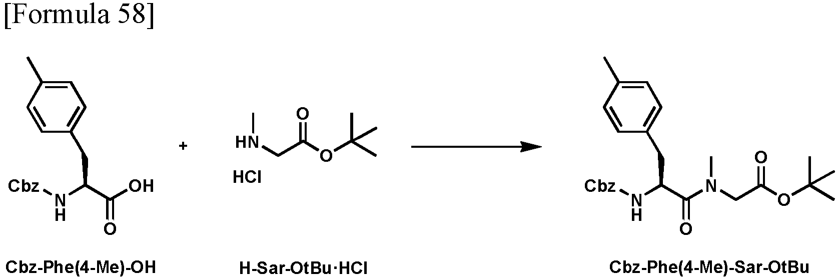

To a reaction vessel, Cbz-Phe(4-Me)-OH (17.22 g, 55.0 mmol), H-Sar-OtBu hydrochloride (11.06 g, 59.7 mmol), 2-methyltetrahydrofuran (141 g), and DIPEA (37.91 g, 293 mmol) were added at 25°C. A solution of propylphosphonic acid anhydride in 2-methyltetrahydrofuran (50.4 wt%, 75.31 g, 119 mmol) was added dropwise over 1 hour and 30 minutes. After completion of the dropwise addition, the mixture was stirred for 2 hours, then sampling was performed, and the completion of the reaction was confirmed by HPLC analysis. A 5% aqueous sodium carbonate solution (102 g) was added dropwise over 40 minutes. The mixture was stirred for 10 minutes and then allowed to stand still, and the aqueous layer was removed. The obtained organic layer was subjected to washing with a 5% aqueous sodium bisulfate monohydrate solution (102 g, twice), a 5% aqueous sodium carbonate solution (102 g), and a 5% aqueous sodium chloride solution (102 g, twice) and then concentrated under reduced pressure conditions to obtain a solution containing Cbz-Phe(4-Me)-Sar-OtBu (38.80 g).
HPLC purity: 99.87%
Measurement method: HPLC Method B
Retention time: 4.11 min
Mass spectrometry: m/z 441 ([M+H]⁺)

### (Example 38) Synthesis of H-EtPhe(4-Me)-Sar-OtBu hydrochloride

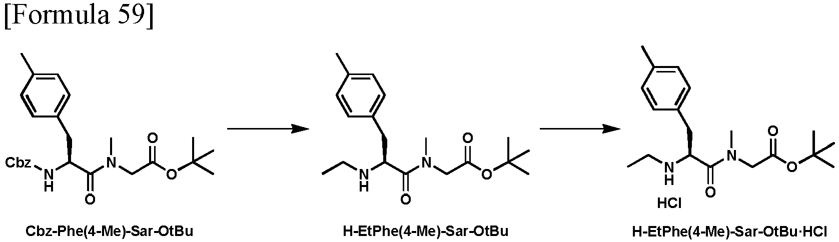

To a reaction vessel, 5% palladium carbon (55.31% wet, 2.60 g, 0.546 mmol, 1 mol% on Pd metal basis), a solution of Cbz-Phe(4-Me)-Sar-OtBu (38.80 g) obtained in Example 37, 2-methyltetrahydrofuran (179 g), acetonitrile (22.29 g, 543 mmol, 10.0 eq.), TsOH•H₂O (10.85 g, 57.0 mmol, 1.0 eq.), and water (1.03 g, 56.9 mmol) were added. Nitrogen replacement was performed at 25°C, then hydrogen replacement was performed, and the mixture was stirred for 2 hours under the hydrogen atmosphere (0.20 MPaG). Then, sampling was performed, and the consumption of Cbz-Phe(4-Me)-Sar-OtBu was confirmed by HPLC analysis. The gas in the reaction vessel was replaced with nitrogen, and 5% palladium carbon (55.31% wet, 15.50 g, 3.26 mmol, 6 mol% on Pd metal basis) was added. After the gas in the reaction vessel was replaced with hydrogen, the mixture was warmed to 33°C. After stirring at 33°C for 7 hours under the hydrogen atmosphere (0.20 MPaG), sampling was performed, and a reaction rate being 99% was confirmed by HPLC analysis. After the reaction mixture was filtered, the cake was washed a total of three times with 2-methyltetrahydrofuran (twice with 68 g and once with 51 g). A mixed solution of the filtrate and washing solution was subjected to washing twice with a 5% aqueous sodium carbonate solution (119 g), and then the obtained organic layer was concentrated under reduced pressure conditions. 2-Methyltetrahydrofuran (27 g) was added, then the mixture was concentrated into 34 mL under reduced pressure conditions, and then 2-methyltetrahydrofuran (12 g) was added. A solution of pyridine hydrochloride (6.27 g, 54.3 mmol, 1.0 eq.) dissolved in acetonitrile (19 g) was added dropwise to the obtained solution over 70 minutes to precipitate crystals of the H-EtPhe(4-Me)-Sar-OtBu hydrochloride. Acetonitrile (6.8 g) was added, and then crystals of H-EtPhe(4-Me)-Sar-OtBu hydrochloride (17.04 mg) obtained from another experiment conducted in a similar manner as described above were added. After stirring for 30 minutes, MTBE (73 g) was added dropwise over 60 minutes. After stirring for 60 minutes, MTBE (122 g) was added dropwise over 60 minutes. After stirring for 14 hours, the slurry was filtered. The obtained solid was washed with 2-methyltetrahydrofuran (68 g) and then washed with a mixed solution of 2-methyltetrahydrofuran (34 g) and MTBE (34 g). The obtained solid was dried under reduced pressure conditions to obtain H-EtPhe(4-Me)-Sar-OtBu hydrochloride (16.62 g).
Melting point of H-EtPhe(4-Me)-Sar-OtBu hydrochloride: 196°C
Yield: 82% (yield by 2 steps from Cbz-Phe(4-Me)-Sar-OtBu)
HPLC purity: 100%
Measurement method: HPLC Method B
Retention time: 2.51 min
Mass spectrometry: m/z 335 ([M+H]⁺)

### (Example 39) Synthesis of Cbz-Aze-EtPhe(4-Me)-Sar-OtBu

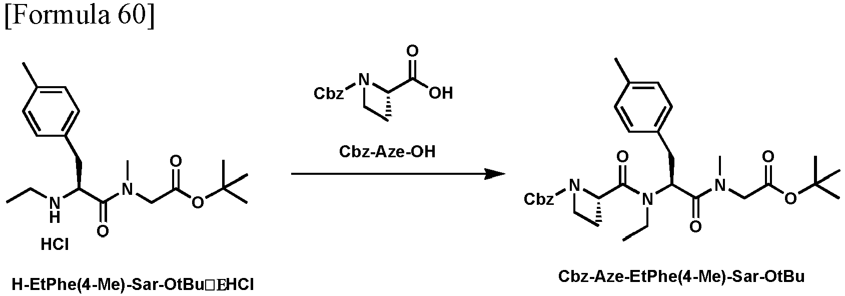

To a reaction vessel, Cbz-Aze-OH (12.94 g, 55.0 mmol), 2-methyltetrahydrofuran (132 g), H-EtPhe(4-Me)-Sar-OtBu hydrochloride (17.01 g, 45.9 mmol) synthesized in a similar manner as in Example 38, and DIPEA (47.41 g, 367 mmol) were added at room temperature. A solution of propylphosphonic acid anhydride in 2-methyltetrahydrofuran (50.4 wt%, 87.00 g, 137 mmol) was added at 25°C over 1 hour and 30 minutes. After completion of the addition, the mixture was stirred for 2 hours, then sampling was performed, and the completion of the reaction was confirmed by HPLC analysis. A 5% aqueous sodium carbonate solution (155 g) was added, then the mixture was stirred for 20 minutes and then allowed to stand still, and the aqueous layer was removed. The obtained organic layer was subjected to washing with a 4% aqueous sulfuric acid solution (156 g), a 10% aqueous potassium bisulfate solution (155 g), and a 5% aqueous sodium carbonate solution (155 g), then 2-methyltetrahydrofuran (43 g) was added, and the mixture was concentrated under reduced pressure conditions. This procedure was repeated three times. To the obtained residue, 2-methyltetrahydrofuran (29 g) was added to obtain a solution containing Cbz-Aze-EtPhe(4-Me)-Sar-OtBu (78.24 g).
HPLC purity: 98.22%
Measurement method: HPLC Method B
Retention time: 4.14 min
Mass spectrometry: m/z 552 ([M+H]⁺)

### (Example 40) Synthesis of H-Aze-EtPhe(4-Me)-Sar-OtBu

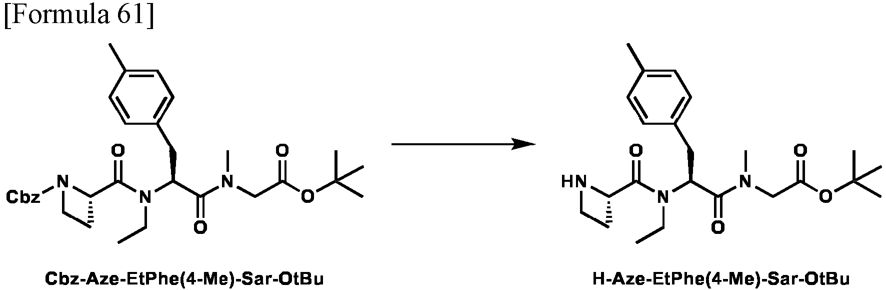

To a reaction vessel, 10% palladium carbon (55.65% wet, 7.76 g, 3.21 mmol, 7 mol% on Pd metal basis) and 2-methyltetrahydrofuran (39 g) were added. Nitrogen replacement was performed at 25°C, then hydrogen replacement was performed, and the mixture was stirred for 2 hours under the hydrogen atmosphere (0.40 MPaG). A solution of Cbz-Aze-EtPhe(4-Me)-Sar-OtBu (78.24 g) obtained in Example 39, 2-methyltetrahydrofuran (29 g), and water (1.71 g, 94.9 mmol) were added. The mixture was stirred for 3 hours under the hydrogen atmosphere (0.20 MPaG), then sampling was performed, and the completion of the reaction was confirmed by HPLC analysis. After the reaction mixture was filtered, the cake was washed twice with 2-methyltetrahydrofuran (61 g). A mixed solution of the filtrate and washing solution was concentrated under reduced pressure conditions, then 2-methyltetrahydrofuran (34 g) was added, and the mixture was concentrated under reduced pressure conditions. This procedure was repeated twice. To the obtained residue, 2-methyltetrahydrofuran (34 g) was added to obtain a solution containing H-Aze-EtPhe(4-Me)-Sar-OtBu (70.59 g).
HPLC purity: 96.53%
Measurement method: HPLC Method B
Retention time: 2.67 min
Mass spectrometry: m/z 418 ([M+H]⁺)

### (Example 41) Synthesis of Cbz-MeAla-Aze-EtPhe(4-Me)-Sar-OtBu

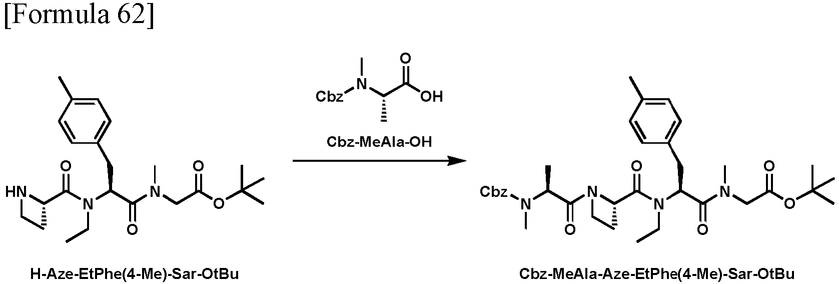

To a reaction vessel, a solution of H-Aze-EtPhe(4-Me)-Sar-OtBu (70.59 g) obtained in Example 40, Cbz-MeAla-OH (13.13 g, 55.0 mmol), 2-methyltetrahydrofuran (68 g), and N-methylmorpholine (11.60 g, 115 mmol) were added at 25°C. A solution of propylphosphonic acid anhydride in 2-methyltetrahydrofuran (50.4 wt%, 69.40 g, 110 mmol) was added at 25°C over 1 hour and 30 minutes. After completion of the addition, the mixture was stirred for 5 hours, then sampling was performed, and the completion of the reaction was confirmed by HPLC analysis. A 5% aqueous sodium carbonate solution (177 g) was added, immediately thereafter 1-methylimidazole (3.77 g, 45.9 mmol) was added, then the mixture was stirred for 2 hours and then allowed to stand still, and the aqueous layer was removed. The obtained organic layer was subjected to washing with a 4% aqueous sulfuric acid solution (138 g), a 10% aqueous potassium bisulfate solution (138 g), and a 5% aqueous sodium carbonate solution (138 g), then 2-methyltetrahydrofuran (51 g) was added, and the mixture was concentrated under reduced pressure conditions. This procedure was repeated three times. To the obtained residue, 2-methyltetrahydrofuran (27 g) was added to obtain a solution containing Cbz-MeAla-Aze-EtPhe(4-Me)-Sar-OtBu (83.89 g).
HPLC purity: 97.28%
Measurement method: HPLC Method B
Retention time: 4.07 min
Mass spectrometry: m/z 637 ([M+H]⁺)

### (Example 42) Synthesis of H-MeAla-Aze-EtPhe(4-Me)-Sar-OtBu

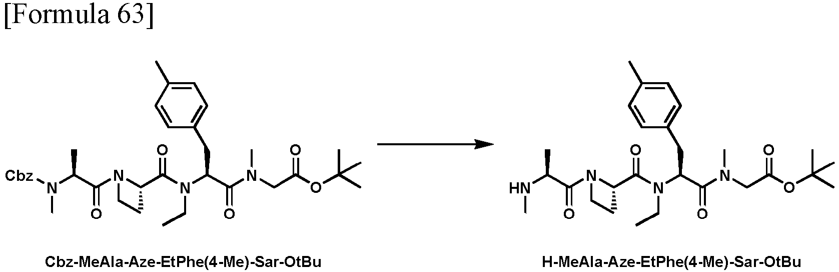

To a reaction vessel, 10% palladium carbon (55.65% wet, 5.54 g, 2.29 mmol, 5 mol% on Pd metal basis) and 2-methyltetrahydrofuran (39 g) were added. Nitrogen replacement was performed at 25°C, then hydrogen replacement was performed, and the mixture was stirred for 2 hours under the hydrogen atmosphere (0.40 MPaG). A solution of Cbz-MeAla-Aze-EtPhe(4-Me)-Sar-OtBu (83.89 g) obtained in Example 41, 2-methyltetrahydrofuran (27 g), and water (2.10 g, 117 mmol) were added. The mixture was stirred for 3 hours under the hydrogen atmosphere (0.20 MPaG), then sampling was performed, and the completion of the reaction was confirmed by HPLC analysis. After the reaction mixture was filtered, the cake was washed twice with 2-methyltetrahydrofuran (59 g). A mixed solution of the filtrate and washing solution was concentrated under reduced pressure conditions and then filtered. To the obtained filtrate, CPME (85 g) was added, and the mixture was concentrated into 68 mL under reduced pressure conditions. This procedure was repeated three times. To the obtained residue, CPME (15 g), MTBE (26 g), and n-heptane (27 g) were added while stirring at 40°C, and then crystals of H-MeAla-Aze-EtPhe(4-Me)-Sar-OtBu (16.66 mg) obtained in a similar manner to the process in Example 66 were added. After stirring at 40°C for 1 hour, the mixture was cooled to 20°C over 2 hours. After stirring at 20°C for 16 hours, n-heptane (241 g) was added over 1 hour. After stirring at 20°C for 4 hours, the mixture was cooled to 8°C over 2 hours. After stirring at 8°C for 16 hours, the slurry was filtered. The obtained solid was washed with n-heptane (66 g) and then dried under reduced pressure conditions to obtain crystals of H-MeAla-Aze-EtPhe(4-Me)-Sar-OtBu (18.28 g).
Melting point of H-MeAla-Aze-EtPhe(4-Me)-Sar-OtBu: 95°C
Yield: 79% (yield by 4 steps from H-EtPhe(4-Me)-Sar-OtBu)
HPLC purity: 99.76%
Measurement method: HPLC Method B
Retention time: 2.62 min
Mass spectrometry: m/z 503 ([M+H]⁺)

### (Example 43) Synthesis of Cbz-Ile-MeAla-Aze-EtPhe(4-Me)-Sar-OtBu

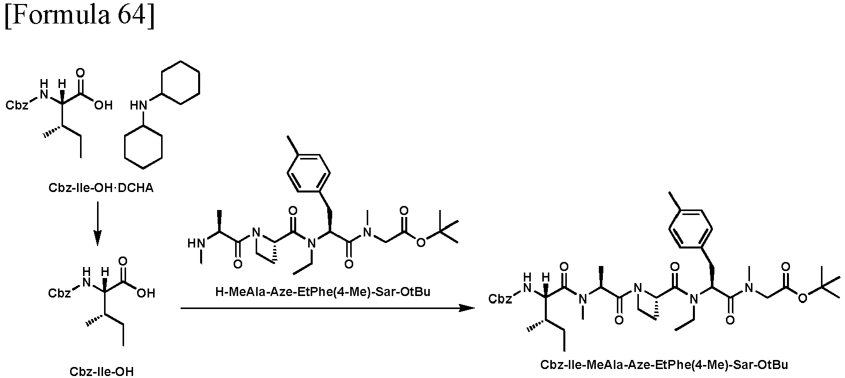

To a reaction vessel, toluene (92 g), a 5% aqueous sodium bisulfate monohydrate solution (100 g), and Cbz-Ile-OH dicyclohexylamine salt (21.43 g, 47.7 mmol) were added while stirring at 25°C. A 5% aqueous sodium bisulfate monohydrate solution (220 g) was added, then the mixture was stirred for 10 minutes and then allowed to stand still, and the aqueous layer was removed. The obtained organic layer was washed three times with a 5% aqueous sodium bisulfate monohydrate solution (320 g) and then washed twice with a 5% aqueous sodium chloride solution (220 g). The obtained organic layer was concentrated into 26 mL under reduced pressure conditions. To the obtained residue, H-MeAla-Aze-EtPhe(4-Me)-Sar-OtBu (20.2 g, 40.2 mmol) obtained by a similar method as in Example 42, 2-methyltetrahydrofuran (72 g), toluene (62 g), acetonitrile (22 g), and DIPEA (22.63 g, 175 mmol) were added while stirring at 25°C, and then HATU (22.69 g, 59.7 mmol) was added while stirring at 22°C. After completion of the addition, the mixture was stirred for 2 hours, then sampling was performed, and the completion of the reaction were confirmed by HPLC analysis. A 5% aqueous sodium carbonate solution (172 g) and 1-methylimidazole (3.27 g, 39.8 mmol) were added. The mixture was stirred at 22°C for 2 hours and then warmed to 25°C, and 2.5% ammonia water (172 g) was added. The mixture was stirred for 10 minutes and then allowed to stand still, and the aqueous layer was removed. The obtained organic layer was washed with 2.5% ammonia water (172 g), a 4% aqueous sulfuric acid solution (172 g), a 10% aqueous sodium bisulfate monohydrate solution (172 g), and a 3% aqueous dipotassium hydrogen phosphate solution (172 g) and then concentrated into 60 mL under reduced pressure conditions. Toluene (52 g) was added, and the mixture was concentrated into 60 mL under reduced pressure conditions. This procedure was repeated twice, and then the obtained residue was filtered. To the filtrate, toluene (66 g) was added, and then n-heptane (102 g) was added over 10 minutes while stirring at 22°C. Crystals of Cbz-Ile-MeAla-Aze-EtPhe(4-Me)-Sar-OtBu (285 mg) obtained in a similar manner to the process in Example 67 were added, and then the mixture was cooled to 18°C over 4 hours and further cooled to 10°C over 4 hours. After stirring at 10°C for 18 hours, n-heptane (102 g) was added over 3 hours. After completion of the addition, the mixture was further stirred at 10°C for 18 hours, and then the slurry was filtered. The obtained solid was washed twice with n-heptane (92 g) and then dried under reduced pressure conditions to obtain crystals of Cbz-Ile-MeAla-Aze-EtPhe(4-Me)-Sar-OtBu (28.08 g).
Melting point of Cbz-Ile-MeAla-Aze-EtPhe(4-Me)-Sar-OtBu: 70°C
Yield: 93%
HPLC purity: 99.84%
Measurement method: HPLC Method B
Retention time: 4.21 min
Mass spectrometry: m/z 750 ([M+H]⁺)

### (Example 44) Synthesis of Teoc-MeLeu-OPFP

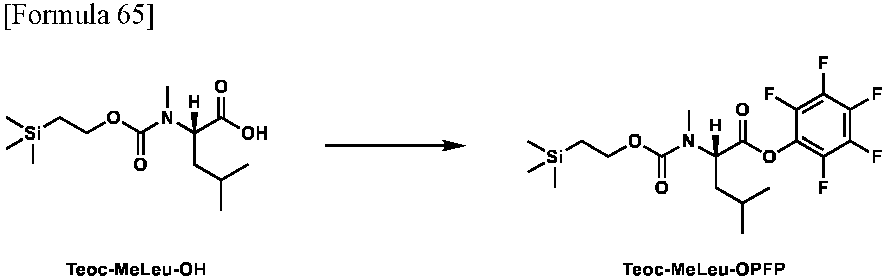

To a reaction vessel, Teoc-MeLeu-OH (19.34 g, 66.8 mmol), 1,3-dimethyl-2-imidazolidinone (132 g), and pentafluorophenol (15.36 g, 83.4 mmol) were added at 25°C. After cooling to 0°C while stirring, 1-(3-dimethylaminopropyl)-3-ethylcarbodiimide hydrochloride (19.34 g, 83.7 mmol) was added. The mixture was warmed to 25°C over 1 hour and further stirred at 25°C for 1 hour, then sampling was performed, and the completion of the reaction was confirmed by HPLC analysis. Isopropyl acetate (110 g) and a 0.5 M aqueous hydrochloric acid solution (126 g) were sequentially added. The mixture was stirred for 10 minutes and then allowed to stand still, and the aqueous layer was removed. The obtained organic layer was washed with a 0.5 M aqueous hydrochloric acid solution (126 g), and then 1,3-dimethyl-2-imidazolidinone (22 g) was added. The obtained organic layer was subjected to washing with a 5% aqueous potassium carbonate solution (126 g, twice) and a 10% aqueous sodium chloride solution (126 g) and then concentrated into 44.4 mL under reduced pressure conditions. To the obtained residue, isopropyl acetate (19 g) was added to obtain a solution containing Teoc-MeLeu-OPFP (67 mL).
HPLC purity: 97.95%
Measurement method: HPLC Method B
Retention time: 5.58 min
Mass spectrometry: m/z 428 ([M-CH2=CH2+H]⁺)

### (Example 45) Synthesis of Teoc-MeLeu-Ile-MeAla-Aze-EtPhe(4-Me)-Sar-OtBu

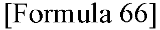

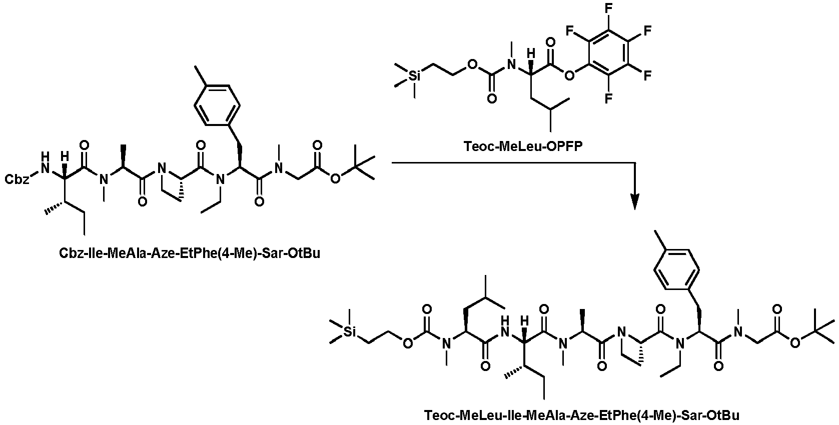

To a reaction vessel, Cbz-Ile-MeAla-Aze-EtPhe(4-Me)-Sar-OtBu (25.00 g, 33.3 mmol) obtained in a similar manner as in Example 43, a solution containing Teoc-MeLeu-OPFP (67 mL) obtained in Example 44, acetone (70 g), N-methylmorpholine (20.22 g, 200 mmol), and 10% palladium carbon (54.33% wet, 7.84 g, 3.34 mmol, 10 mol% on Pd metal basis) were sequentially added. While stirring at 25°C, nitrogen replacement was performed, and hydrogen replacement was performed. The mixture was stirred for 2 hours under the hydrogen atmosphere (0.18 MPaG), then sampling was performed, and the completion of the reaction was confirmed by HPLC analysis. After the reaction mixture was filtered, the cake was washed three times with acetone (29 g). A mixed solution of the filtrate and washing solution was concentrated into 120 mL under reduced pressure conditions. To the obtained residue, toluene (87 g), a 5% aqueous potassium carbonate solution (110 g), and 4-dimethylaminopyridine (4.07 g, 33.3 mmol) were sequentially added while stirring at 25°C. The mixture was stirred for 5 hours and then allowed to stand still, and the aqueous layer was removed. The obtained organic layer was subjected to washing with a 4% aqueous sulfuric acid solution (110 g), a 10% aqueous potassium bisulfate solution (110 g), and a 5% aqueous potassium carbonate solution (110 g, twice) and then concentrated under reduced pressure conditions to obtain a solution containing Teoc-MeLeu-Ile-MeAla-Aze-EtPhe(4-Me)-Sar-OtBu (47.5 mL).
HPLC purity: 98.61%
Measurement method: HPLC Method B
Retention time: 5.28 min
Mass spectrometry: m/z 888 ([M+H]⁺)

### (Example 46) Synthesis of H-MeLeu-Ile-MeAla-Aze-EtPhe(4-Me)-Sar-OtBu

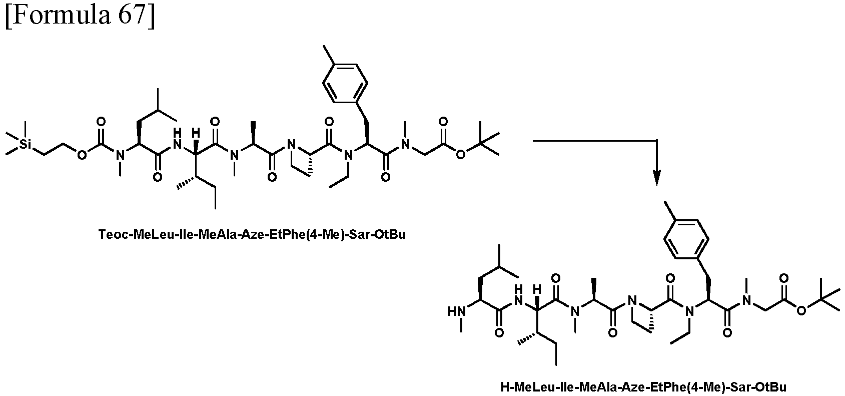

To a reaction vessel, a toluene solution containing Teoc-MeLeu-Ile-MeAla-Aze-EtPhe(4-Me)-Sar-OtBu (61.47 w/w%, 4.88 g, 3.38 mmol) obtained in a similar manner as in Example 45 and 2-methyltetrahydrofuran (8.9 mL) were added. The mixture was warmed to 45°C, and a solution of tetrabutylammonium fluoride in tetrahydrofuran (1.2 M, 7.04 mL, 8.45 mmmol) was added over 20 minutes. After completion of the addition, the mixture was stirred for 1 hour, then sampling was performed, and the completion of the reaction was confirmed by HPLC analysis. The mixture was cooled to 25°C, and isopropyl acetate (9.0 mL) was added. The reaction mixture was subjected to washing with a 5% aqueous sodium carbonate solution (9.13 g, three times) and a 5% aqueous sodium chloride solution (9.13 g), and then the obtained organic layer was concentrated under reduced pressure conditions. To the obtained residue, ethanol (12.5 mL) was added, and the mixture was concentrated. This procedure was repeated twice to obtain a solution containing H-MeLeu-Ile-MeAla-Aze-EtPhe(4-Me)-Sar-OtBu (6.9 mL).
HPLC purity: 98.17%
Measurement method: HPLC Method B
Retention time: 3.04 min
Mass spectrometry: m/z 743.4 ([M+H]⁺)

### (Example 47) Synthesis of H-MeLeu-Ile-MeAla-Aze-EtPhe(4-Me)-Sar-OtBu L-tartrate

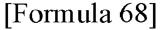

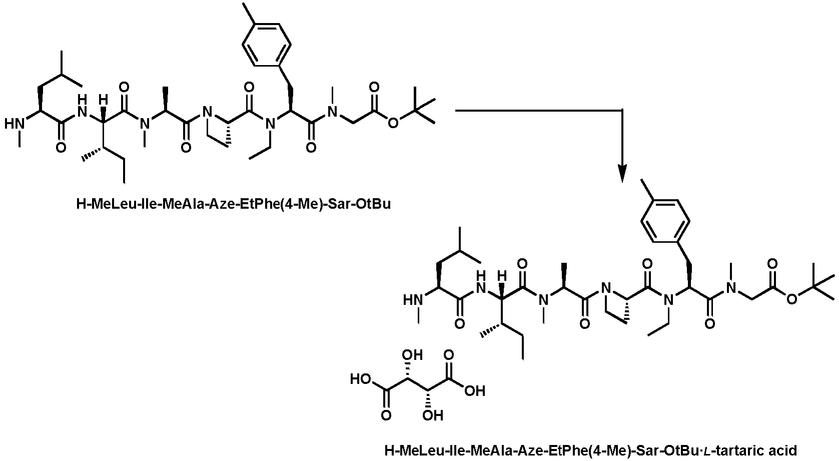

To a reaction vessel, a solution containing H-MeLeu-Ile-MeAla-Aze-EtPhe(4-Me)-Sar-OtBu (38.46 w/w%, 56.16 g, 29.1 mmol) obtained in a similar manner as in Example 46 and ethanol (8.0 mL) were added. L-tartaric acid (4.80 g, 32.0 mmol) was added at 22°C, and MTBE (207 mL) was added over 2 minutes. A slurry of crystals of H-MeLeu-Ile-MeAla-Aze-EtPhe(4-Me)-Sar-OtBu L-tartrate (108.0 mg) obtained in a similar manner to the process in Example 68 suspended in MTBE (1.62 mL) was added, and then MTBE (77 g) was added over 1 hour. After stirring for 18 hours, n-heptane (106 g) was added dropwise over 1 hour. The mixture was cooled over 1 hour such that the internal temperature became 10°C. After further stirring at 10°C for 27 hours, the slurry was filtered. The obtained solid was washed with MTBE (130 mL) and then dried under reduced pressure conditions to obtain H-MeLeu-Ile-MeAla-Aze-EtPhe(4-Me)-Sar-OtBu L-tartrate (26.13 g, content: 92.2 w/w%). The content and the yield were calculated by HPLC analysis using a preparation.
Melting point of H-MeLeu-Ile-MeAla-Aze-EtPhe(4-Me)-Sar-OtBu L-tartrate: 94°C
Yield: 88% (yield by 3 steps from H-Ile-MeAla-Aze-EtPhe(4-Me)-Sar-OtBu)
HPLC purity: 99.38%
Measurement method: HPLC Method B
Retention time: 3.01 min
Mass spectrometry: m/z 743.6 ([M+H]⁺)

### (Example 48) Synthesis of Cbz-MeAsp(OtBu)-NMe₂

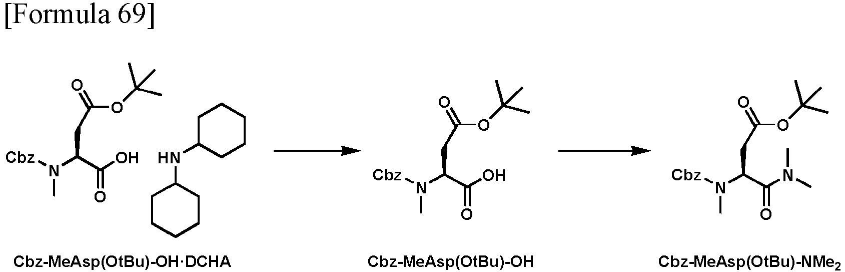

To a reaction vessel, Cbz-MeAsp(OtBu)-OH dicyclohexylamine salt (25.00 g, 48.2 mmol) and 2-methyltetrahydrofuran (126 g) were added at 25°C. After washing with a 10% aqueous sodium bisulfate monohydrate solution (150 g) was repeated twice, washing with a 5% aqueous sodium chloride solution (150 g) was performed. The obtained organic layer was concentrated under reduced pressure conditions. To the obtained residue, 2-methyltetrahydrofuran (95 g) was added, and the mixture was concentrated under reduced pressure conditions. This procedure was repeated twice. To the obtained residue (47.91 g), 2-methyltetrahydrofuran (95 g), acetonitrile (75 g), DIPEA (35.46 g, 274 mmol), and dimethylamine hydrochloride (7.88 g, 96.6 mmol) were added at 25°C. A solution of propylphosphonic acid anhydride in 2-methyltetrahydrofuran (50.4 wt%, 61.33 g, 97.1 mmol) was added dropwise over 1 hour and 30 minutes. After completion of the dropwise addition, the mixture was stirred for 1 hour, then sampling was performed, and the completion of the reaction was confirmed by HPLC analysis. A 2 M aqueous sodium hydroxide solution (150 g) was added. The mixture was stirred for 10 minutes and then allowed to stand still, and the aqueous layer was removed. The obtained organic layer was washed with a 2 M aqueous sodium hydroxide solution (150 g), a 13% aqueous sulfuric acid solution (150 g), a 10% aqueous sodium bisulfate monohydrate solution (150 g), and a 5% aqueous sodium carbonate solution (150 g) and then concentrated under reduced pressure conditions. 2-Methyltetrahydrofuran (125 g) was added, and the mixture was concentrated under reduced pressure conditions. This procedure was repeated twice to obtain a solution containing Cbz-MeAsp(OtBu)-NMe₂ (42.39 g).
HPLC purity: 99.85 %
Measurement method: HPLC Method C
Retention time: 3.37 min
Mass spectrometry: m/z 387 ([M+Na]⁺)

### (Example 49) Synthesis of H-MeAsp(OtBu)-NMe₂

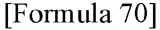

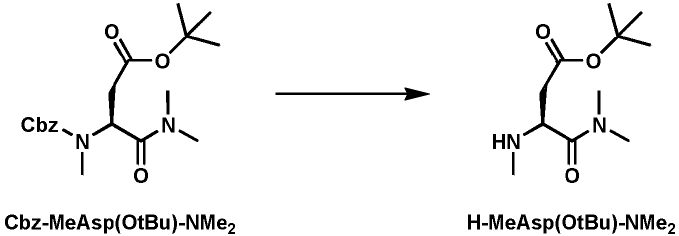

To a reaction vessel, 10% palladium carbon (54.33% wet, 3.39 g, 1.45 mmol, 3 mol% on Pd metal basis) and 2-methyltetrahydrofuran (75 g) were added. Nitrogen replacement was performed at 25°C, then hydrogen replacement was performed, and the mixture was stirred for 2 hours under the hydrogen atmosphere (0.40 MPaG). A solution of Cbz-MeAsp(OtBu)-NMe₂ (42.39 g) obtained in Example 48 and 2-methyltetrahydrofuran (22 g) were added. The mixture was stirred for 1 hour and 30 minutes under the hydrogen atmosphere (0.20 MPaG), then sampling was performed, and the completion of the reaction was confirmed by HPLC analysis. After the reaction mixture was filtered, the cake was washed twice with 2-methyltetrahydrofuran (75 g). A mixed solution of the filtrate and washing solution was concentrated under reduced pressure conditions to obtain a solution containing H-MeAsp(OtBu)-NMe₂ (30.76 g).
HPLC purity: 98.64%
Measurement method: HPLC Method C
Retention time: 1.44 min
Mass spectrometry: m/z 231 ([M+H]⁺)

### (Example 50) Synthesis of Cbz-MeGcp-MeAsp(OtBu)-NMe₂

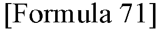

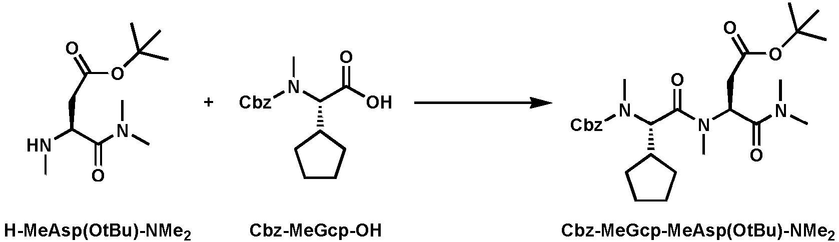

To a reaction vessel, a solution of H-MeAsp(OtBu)-NMe₂ (30.76 g) obtained in Example 49, Cbz-MeGcp-OH (16.96 g, 58.2 mmol), 2-methyltetrahydrofuran (40 g), acetonitrile (17 g), and DIPEA (27.74 g, 215 mmol) were added at 25°C. HATU (27.49 g, 72.3 mmol) was added over 10 minutes. After completion of the addition, the mixture was stirred for 3 hours, then sampling was performed, and the completion of the reaction was confirmed by HPLC analysis. Toluene (30 g), a 5% aqueous potassium carbonate solution (23 g), and 1-methylimidazole (3.97 g, 48.4 mmol) were added, and the mixture was stirred for 30 minutes. 2.5% ammonia water (88 g) and 2-methyltetrahydrofuran (25 g) were added. The mixture was stirred for 10 minutes and then allowed to stand still, and the aqueous layer was removed. The obtained organic layer was washed with 2.5% ammonia water (113 g), a 10% aqueous sodium bisulfate monohydrate solution (113 g, twice), and a 5% aqueous potassium carbonate solution (113 g) and then concentrated under reduced pressure conditions. 2-Methyltetrahydrofuran (42 g) was added, and the mixture was concentrated under reduced pressure conditions to obtain a solution containing Cbz-MeGcp-MeAsp(OtBu)-NMe₂ (52.78 g).
HPLC purity: 98.59%
Measurement method: HPLC Method C
Retention time: 4.10 min
Mass spectrometry: m/z526 ([M+Na]⁺)

### (Example 51) Synthesis of H-MeGcp-MeAsp(OtBu)-NMe₂

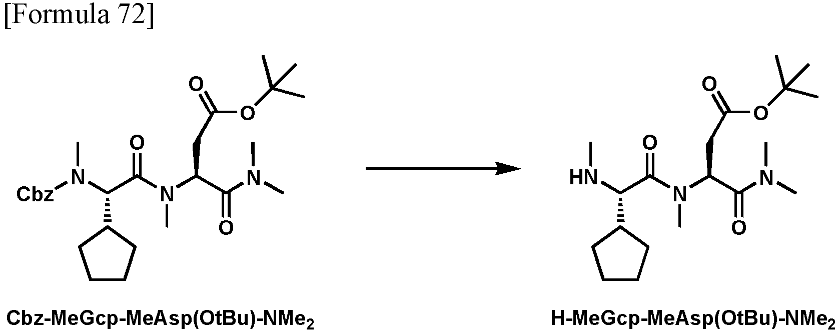

To a reaction vessel, 10% palladium carbon (54.33% wet, 3.39 g, 1.45 mmol, 3 mol% on Pd metal basis) and 2-methyltetrahydrofuran (75 g) were added. Nitrogen replacement was performed at 25°C, then hydrogen replacement was performed, and the mixture was stirred for 2 hours under the hydrogen atmosphere (0.40 MPaG). A solution of Cbz-MeGcp-MeAsp(OtBu)-NMe₂ (52.78 g) obtained in Example 50 and 2-methyltetrahydrofuran (15 g) were added, and then the mixture was warmed to 30°C. The mixture was stirred for 2 hours under the hydrogen atmosphere (0.20 MPaG), then sampling was performed, and the completion of the reaction was confirmed by HPLC analysis. After the reaction mixture was filtered, the cake was washed twice with 2-methyltetrahydrofuran (75 g). A mixed solution of the filtrate and washing solution was concentrated under reduced pressure conditions, then acetonitrile (75 g) was added, and the mixture was concentrated under reduced pressure conditions. This procedure was repeated twice to obtain a solution containing H-MeGcp-MeAsp(OtBu)-NMe₂ (42.5 mL).
HPLC purity: 96.86%
Measurement method: HPLC Method D
Retention time: 2.96 min
Mass spectrometry: m/z 370 ([M+H]⁺)

### (Example 52) Synthesis of H-MeGcp-MeAsp(OtBu)-NMe₂ hydrochloride

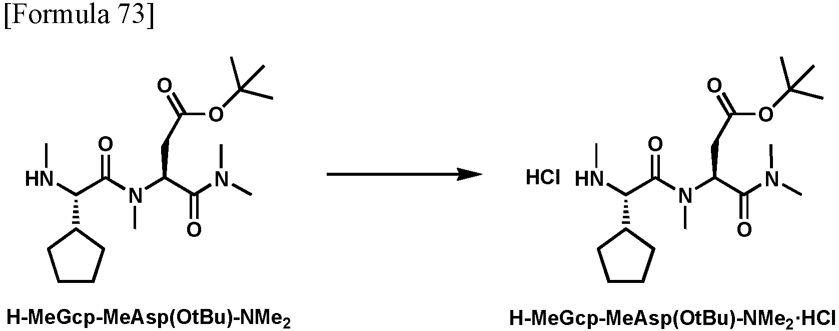

To a reaction vessel, a solution of H-MeGcp-MeAsp(OtBu)-NMe₂ (42.5 mL) obtained in Example 51 and acetonitrile (8.0 g) were added. MTBE (65 g) was added at 40°C, and then a solution of pyridine hydrochloride in acetonitrile (16.94 w/w%, 4.50 g) was added dropwise over 30 minutes. After stirring for 1 hour, a solution of pyridine hydrochloride in acetonitrile (16.94 w/w%, 31.34 g) was added dropwise over 3 hours and 30 minutes, and acetonitrile (14 g) was added. After stirring for 1 hour, the mixture was cooled to 10°C over 6 hours. After further stirring at 10°C for 11 hours, the slurry was filtered. The obtained solid was washed twice with MTBE (38 g) and then dried under reduced pressure conditions to obtain H-MeGcp-MeAsp(OtBu)-NMe₂ hydrochloride (15.68 g).
Melting point of H-MeGcp-MeAsp(OtBu)-NMe₂ hydrochloride: 227°C Yield: 80% (yield by 5 steps from Cbz-MeAsp(OtBu)-OH dicyclohexylamine salt)
HPLC purity: 99.62%
Measurement method: HPLC Method D
Retention time: 2.92 min
Mass spectrometry: m/z 370 ([M+H]⁺)

### (Example 53) Synthesis of Cbz-cLeu-MeGcp-MeAsp(OtBu)-NMe₂

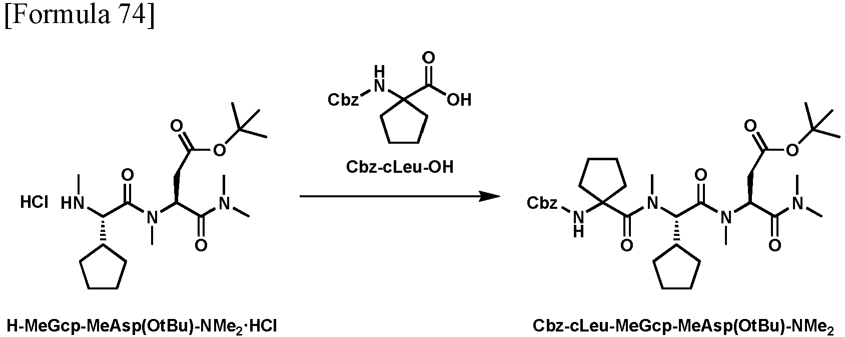

To a reaction vessel, H-MeGcp-MeAsp(OtBu)-NMe₂ hydrochloride (1.00 g, 2.46 mmol) synthesized in a similar manner as in Example 52 and acetonitrile (10 mL) were added. Subsequently, DIPEA (2.72 mL, 15.6 mmol), Cbz-cLeu-OH (1.74 g, 6.61 mmol), and HATU (2.75 g, 7.23 mmol) were sequentially added while stirring, and the mixture was warmed to 50°C. The mixture was stirred at 50°C for 6 hours, then sampling was performed, and the completion of the reaction was confirmed by HPLC analysis. 1-Methylimidazole (0.78 mL, 9.78 mmol) and water (3.99 mL) were added. After stirring at 50°C for 1 hour, the mixture was cooled to 25°C. After stirring at 25°C for 14 hours, the slurry was filtered. The obtained solid was washed with a combination of acetonitrile/water (8:3 (v/v), 5.33 mL) and then dried under reduced pressure conditions to obtain Cbz-cLeu-MeGcp-MeAsp(OtBu)-NMe₂ (1.27 g).
Melting point of Cbz-cLeu-MeGcp-MeAsp(OtBu)-NMe₂: 202°C
Yield: 84%
HPLC purity: 99.86%
Measurement method: HPLC Method D
Retention time: 6.70 min
Mass spectrometry: m/z 637 ([M+Na]⁺)

### (Example 54) Synthesis of H-cLeu-MeGcp-MeAsp(OtBu)-NMe₂

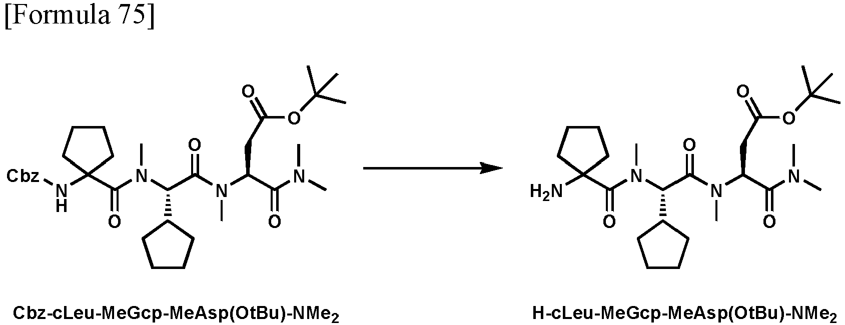

To a reaction vessel, 5% palladium carbon (50% wet, 0.85 g, 0.20 mmol, 3.5 mol% on Pd metal basis) and tetrahydrofuran (14.0 mL) were added. Nitrogen replacement was performed at 25°C, then hydrogen replacement was performed, and the mixture was stirred for 2 hours under the hydrogen atmosphere (0.40 MPaG). A solution of Cbz-cLeu-MeGcp-MeAsp(OtBu)-NMe₂ (3.51 g, 5.71 mmol) synthesized in a similar manner as in Example 53 dissolved in tetrahydrofuran (42 mL) was added. The mixture was stirred for 2 hours under the hydrogen atmosphere (0.20 MPaG), then sampling was performed, and the completion of the reaction was confirmed by HPLC analysis. After the reaction mixture was filtered, the cake was washed twice with tetrahydrofuran (14 mL). A mixed solution of the filtrate and washing solution was concentrated under reduced pressure conditions to obtain a solution containing H-cLeu-MeGcp-MeAsp(OtBu)-NMe₂ (7.37 g).
HPLC purity: 99.98%
Measurement method: HPLC Method C
Retention time: 2.42 min
Mass spectrometry: m/z 503 ([M+Na]⁺)

### (Example 55) Synthesis of Cbz-Pro-cLeu-MeGcp-MeAsp(OtBu)-NMe₂

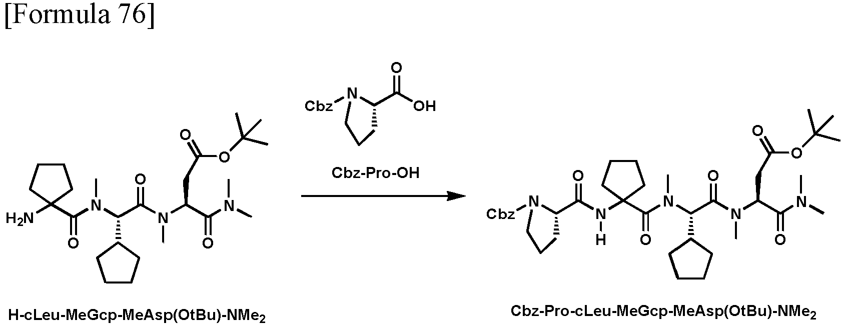

To a reaction vessel, a tetrahydrofuran solution (9.63 g) containing H-cLeu-MeGcp-MeAsp(OtBu)-NMe₂ (5.00 g, 10.4 mmol) obtained by a similar method as in Example 54, acetonitrile (25 mL), and Cbz-Pro-OH (3.37 g, 13.5 mmol) were added at room temperature. While stirring at 25°C, N-methylmorpholine (3.12 g, 30.8 mmol) and HATU (5.93 g, 15.6 mmol) were added. After completion of the addition, the mixture was stirred for 1 hour, then sampling was performed, and the completion of the reaction was confirmed by HPLC analysis. Toluene (40 mL) and 1-methylimidazole (0.90 g, 10.9 mmol) were added, and then a 5% aqueous potassium carbonate solution (10 mL) was added at 10°C. The mixture was warmed to 25°C, stirred for 30 minutes, and then cooled to 10°C, and 2.5% ammonia water (20 mL) was added. The mixture was warmed to 25°C, further stirred for 10 minutes, and then allowed to stand still, and the aqueous layer was removed. The obtained organic layer was washed with 2.5% ammonia water (30 mL), a 3% aqueous sulfuric acid solution (30 mL), a 10% aqueous potassium bisulfate solution (30 mL), and a 5% aqueous sodium carbonate solution (30 mL, twice) and then concentrated into 25 mL under reduced pressure conditions. Toluene (25 mL) was added, and the mixture was concentrated into 25 mL under reduced pressure conditions. To the obtained residue, tetrahydrofuran (15 mL) was added, and the mixture was stirred at 40 to 60°C to prepare a homogeneous solution. After cooling to 25°C, a slurry of crystals of Cbz-Pro-cLeu-MeGcp-MeAsp(OtBu)-NMe₂ (49.9 mg) obtained in a similar manner to the process in Example 69 suspended in a mixed solution of n-heptane (0.16 mL) and tetrahydrofuran (0.04 mL), and subsequently a mixed solution of n-heptane (0.32 mL) and tetrahydrofuran (0.08 mL) were added. After stirring at 25°C for 13 hours, n-heptane (5 mL) was added over 17 minutes. After completion of the addition, the mixture was stirred for 1 hour, and then n-heptane (5 mL) was added over 15 minutes. After further stirring for 1 hour, n-heptane (25 mL) was added over 16 minutes. After further stirring for 3 hours, the slurry was filtered. The obtained solid was washed twice with a mixed solution of n-heptane(10 mL) and tetrahydrofuran (2.5 mL) and then dried under reduced pressure conditions to obtain crystals of Cbz-Pro-cLeu-MeGcp-MeAsp(OtBu)-NMe₂ (6.51 g).
Melting point of Cbz-Pro-cLeu-MeGcp-MeAsp(OtBu)-NMe₂: 178°C
Yield: 88%
HPLC purity: 100%
Measurement method: HPLC Method D
Retention time: 6.54 min
Mass spectrometry: m/z 734 ([M+Na]⁺)

### (Example 56) Synthesis of H-Pro-cLeu-MeGcp-MeAsp(OtBu)-NMe₂

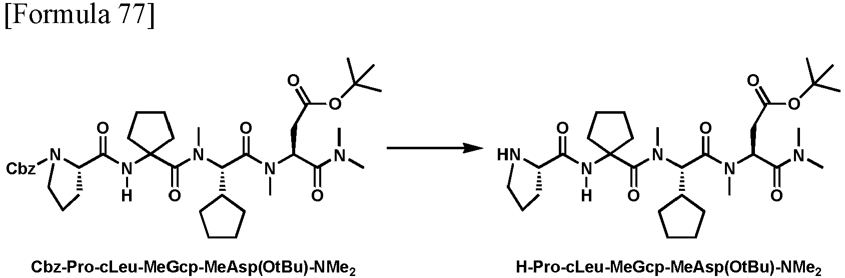

To a reaction vessel, 10% palladium carbon (54.33% wet, 1.36 g, 0.568 mmol, 2.2 mol% on Pd metal basis) and tetrahydrofuran (27 mL) were added. Nitrogen replacement was performed at 25°C, then hydrogen replacement was performed, and the mixture was stirred for 2 hours under the hydrogen atmosphere (0.40 MPaG). Cbz-Pro-cLeu-MeGcp-MeAsp(OtBu)-NMe₂ (18.03 g, 25.3 mmol) synthesized in a similar manner as in Example 55 and tetrahydrofuran (52 mL) were added. The mixture was stirred for 1 hour under the hydrogen atmosphere (0.18 MPaG), then sampling was performed, and the completion of the reaction was confirmed by HPLC analysis. After the reaction mixture was filtered, the cake was washed three times with 2-methyltetrahydrofuran (38 mL). A mixed solution of the filtrate and washing solution was concentrated into 81 mL under reduced pressure conditions, then 2-methyltetrahydrofuran (68 mL) was added, and the mixture was concentrated into 81 mL. This procedure was repeated three times. To the obtained residue, n-heptane (37 mL) was added while stirring at 45°C, and then a slurry of crystals of H-Pro-cLeu-MeGcp-MeAsp(OtBu)-NMe₂ (40.60 mg) obtained in a similar manner to the process in Example 70 suspended in n-heptane (0.75 mL), and n-heptane (0.75 mL) were sequentially added. After stirring at 45°C for 2 hours, n-heptane (22 g) was added over 15 minutes. After further stirring at 45°C for 18 hours, n-heptane (109 g) was added over 75 minutes. After further stirring at 45°C for 2 hours, the mixture was cooled to 22°C over 2 hours and further cooled to 10°C over 1 hour. After stirring at 10°C for 16 hours, the slurry was filtered. The obtained solid was sequentially washed with a mixed solvent of 2-methyltetrahydrofuran/heptane (1:9 (v/w), 47 g) and subsequently 2-methyltetrahydrofuran (68 mL) and then dried under reduced pressure conditions to obtain crystals of H-Pro-cLeu-MeGcp-MeAsp(OtBu)-NMe₂ (12.96 g).
Melting point of H-Pro-cLeu-MeGcp-MeAsp(OtBu)-NMe₂: 147°C
Yield: 95.8%
HPLC purity: 100%
Measurement method: HPLC Method C
Retention time: 2.73 min
Mass spectrometry: m/z 578.5 ([M+H]⁺)

### (Example 57-1) Synthesis of Boc-Hph(3,5-F₂-4-CF₃)-OBn

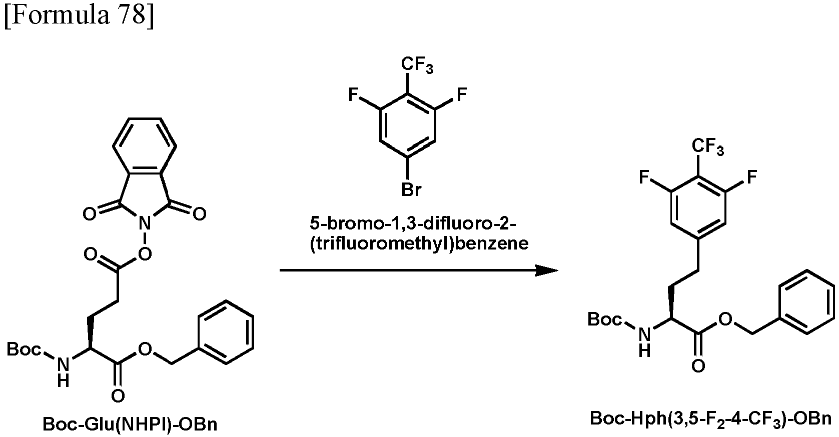

To a reaction vessel in which the gas was replaced with nitrogen, a slurry of nickel(II) bromide trihydrate (1.58 g, 5.80 mmol) suspended in 1,3-dimethyl-2-imidazolidinone (160 mL), and 4,4'-di-tert-butyl-2,2'-bipyridine (1.56 g, 5.80 mmol) were added. While stirring, Boc-Glu(NHPI)-OBn (40.0 g, 83.0 mmol) synthesized by the method described in International Publication No. WO 2020/189540 was added, and then 1,3-dimethyl-2-imidazolidinone (40 mL), 5-bromo-1,3-difluoro-2-(trifluoromethyl)-benzene (26.0 g, 99 mmol), and N-methylmorpholine (22.8 mL, 207 mmol) were sequentially added. After cooling to 10°C, active zinc (16.26 g, 249 mmol) was added. While TMSCI (21.0 mL, 166 mmol) was added dropwise over 1 hour, and the mixture was warmed to 25°C. Immediately after completion of the addition, sampling was performed, and the completion of the reaction was confirmed by HPLC analysis. A 15% aqueous ammonium chloride solution (416 g) was added at 0°C, and then the mixture was warmed to 25°C and stirred for 50 minutes. Toluene (200 mL) was added, then the slurry was filtered through celite, and then the cake was washed with toluene (200 mL). The obtained solution was stirred for 20 minutes and then allowed to stand still, and the aqueous layer was removed. To the obtained organic layer, a solution of disodium dihydrogen ethylenediaminetetraacetate dihydrate (31.4 g, 84.0 mmol) dissolved in a 0.1 M aqueous potassium hydroxide solution (600 mL) was added while stirring. The mixture was stirred for 3 hours and then allowed to stand still, and the aqueous layer was removed. The obtained organic layer was subjected to washing with a 10% aqueous sodium chloride solution (400 mL) and then concentrated under reduced pressure conditions to obtain a solution containing Boc-Hph(3,5-F₂-4-CF₃)-OBn (90.81 g).
HPLC purity: 78.47%
Measurement method: HPLC Method E
Retention time: 7.40 min
Mass spectrometry: m/z 374 ([M-Boc+2H]⁺)

### (Examples 57-1 to 57-3) Synthesis of Boc-Hph(3,5-F₂-4-CF₃)-OBn: Evaluation of effect of addition of basic compound

To a reaction vessel in which the gas was replaced with nitrogen, a slurry of nickel(II) bromide trihydrate (0.07 eq.) suspended in a solvent (4.0 v/w of Boc-Glu(NHPI)-OBn), and 4,4'-di-tert-butyl-2,2'-bipyridine (0.07 eq.) were added. While stirring, Boc-Glu(NHPI)-OBn (X g (see Table 38), 1.0 eq.) synthesized by the method described in International Publication No. WO 2020/189540 was added, and then a solvent (1.0 v/w of Boc-Glu(NHPI)-OBn), 5-bromo-1,3-difluoro-2-(trifluoromethyl)-benzene (1.2 eq.), and NMM (2.5 eq.) only in Example 57-1 were sequentially added. After cooling to 10°C, active zinc (3.0 eq.) was added. While TMSCl (Y eq. (see Table 38)) was added dropwise over 1 hour, the mixture was warmed to 25°C. After completion of the addition, sampling was performed Z hours later, and a reaction rate was confirmed. The reaction rate was calculated according to the following expression using the area value of Boc-Glu(NHPI)-OBn and the area value of Boc-Hph(3,5-F₂-4-CF₃)-OBn calculated by HPLC analysis. Reaction rate (%)= Area volume of Boc-Hph(3,5-F2-4-CF3)-OBn / Area value of (Boc-Glu(NHPI)-OBn + Area value of Boc-Hph(3,5-F2-4-CF3)-OBn) × 100

**[Table 38]**

| Example | X (g) | Solvent | Y (eq.) | Base | Z (h) | Reaction rate |
|---|---|---|---|---|---|---|
| 57-1 | 40 | DMI | 2.0 | NMM (2.5 eq.) | 0 | ≥99% |
| 57-2 | 5 | DMI | 3.0 | none | 0 | 75.3% |
| | | | | | 3 | 95.4% |
| 57-3 | 5 | DMA | 2.0 | none | 0 | ≥99% |

### (Example 58) Synthesis of Cbz-Hph(3,5-F₂-4-CF₃)-OH dicyclohexylamine salt

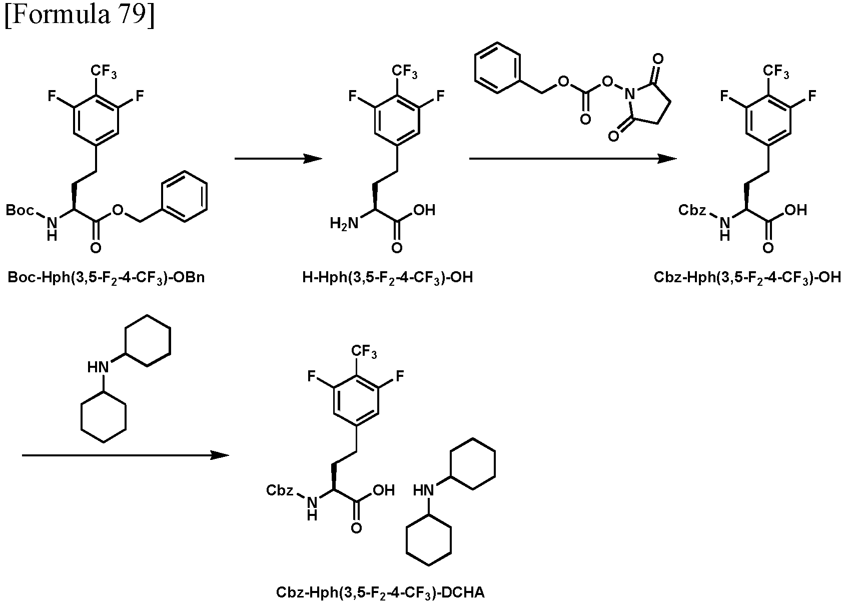

To a reaction vessel in which the gas was replaced with nitrogen, a solution of Boc-Hph(3,5-F₂-4-CF₃)-OBn (90.81 g) obtained in Example 57-1 and toluene (135 mL) were added, and then the mixture was cooled to 0°C. Trifluoromethanesulfonic acid (22.0 mL, 249 mmol) was added over 17 minutes, and then the mixture was warmed to 25°C. After completion of the addition, the mixture was stirred for 1 hour, then sampling was performed, and the completion of the reaction was confirmed by HPLC analysis. Water (40 mL) was added, then the mixture was stirred for 15 minutes, and then water (160 mL) was further added. The mixture was further stirred for 1 hour and then allowed to stand still, and the organic layer was removed to obtain a solution containing H-Hph(3,5-F₂-4-CF₃)-OH. The obtained H-Hph(3,5-F₂-4-CF₃)-OH was used in the next step without being purified.

To a solution containing H-Hph(3,5-F₂-4-CF₃)-OH obtained as described above, a 40% aqueous potassium phosphate solution (60 mL) was added while stirring. Acetonitrile (100 mL) and a 40% aqueous potassium phosphate solution (18 mL) were added, and then N-carbobenzoxyoxysuccinimide (16.53 g, 66.3 mmol) was added. After completion of the addition, the mixture was stirred for 2 hours, then sampling was performed, and the completion of the reaction was confirmed by HPLC analysis. A mixed solution consisting of n-heptane (80 mL) and MTBE (80 mL) was added, then the mixture was stirred for 10 minutes and then allowed to stand still, and the organic layer was removed. To the obtained aqueous layer, MTBE (200 mL), a 0.2 M aqueous sodium hydroxide solution (80 mL), and a 20% aqueous sodium chloride solution (80 mL) were added, then the mixture was stirred for 13 minutes and then allowed to stand still, and the aqueous layer was removed. The obtained organic layer was subjected to washing twice with a mixed solution of 0.2 M aqueous sodium hydroxide solution (80 mL) and a 20% aqueous sodium chloride solution (80 mL) and then further washing with a 0.2 M aqueous sodium hydroxide solution (80 mL) and a 1 M aqueous hydrochloric acid solution (600 mL). To the obtained organic layer, MTBE (80 mL) and a 10% aqueous sodium chloride solution (214 mL) were added, then the mixture was stirred for 10 minutes and then allowed to stand still, and the aqueous layer was removed. The obtained organic layer was concentrated under reduced pressure conditions. To the obtained residue, toluene (120 mL) was added, and the mixture was concentrated into 80 mL to obtain a solution containing Cbz-Hph(3,5-F₂-4-CF₃)-OH (116.07 g). The obtained Cbz-Hph(3,5-F₂-4-CF₃)-OH was used in the next step without being purified.

To a reaction vessel in which the gas was replaced with nitrogen, a solution of Cbz-Hph(3,5-F₂-4-CF₃)-OH in toluene (116.07 g) prepared as described above and toluene (188 mL) were added, and the mixture was warmed to 50°C. Dicyclohexylamine (22.4 mL, 112 mmol) and MTBE (94 mL) were added, and then Cbz-Hph(3,5-F₂-4-CF₃)-OH dicyclohexylamine salt (117 mg) was added. After completion of the addition, the mixture was stirred for 3 hours, and then n-heptane (94 mL) was added over 2 hours while stirring. After completion of the addition, the mixture was further stirred for 1 hour, and then n-heptane (188 mL) was added over 3 hours while stirring. After completion of the addition, the mixture was further stirred for 1 hour and then cooled to 20°C. After stirring at 20°C for 13 hours, the slurry was filtered. The cake was washed with a mixed solvent of MTBE/n-heptane (1:1 (v/v), 94 mL), and then the obtained wet powder was dried under reduced pressure conditions to obtain Cbz-Hph(3,5-F₂-4-CF₃)-OH dicyclohexylamine salt (31.7 g).
Melting point of Cbz-Hph(3,5-F₂-4-CF₃)-OH dicyclohexylamine salt: 154°C
Yield: 64% (yield by 4 steps from Boc-Glu(NHPI)-OBn)
HPLC purity: 99.70%
Measurement method: HPLC Method F
Retention time: 9.73 min
Mass spectrometry: m/z 418 ([M+H]⁺)

### (Example 59) Synthesis of Cbz-Hph(3,5-F₂-4-CF₃)-Pro-cLeu-MeGcp-MeAsp(OtBu)-NMe₂

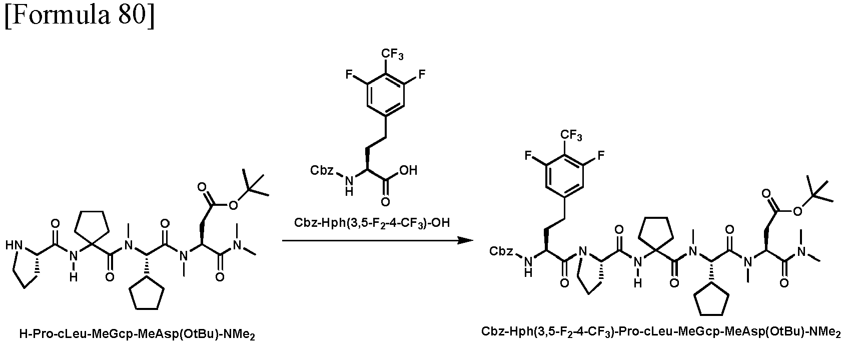

To a reaction vessel, Cbz-Hph(3,5-F₂-4-CF₃)-OH dicyclohexylamine salt (42.0 g, 70.2 mmol) synthesized in a similar manner as in Example 58 and 2-methyltetrahydrofuran (169 mL) were added. After washing with a 10% aqueous sodium bisulfate monohydrate solution (170 mL) was repeated twice, washing with a 5% aqueous sodium chloride solution (170 mL) was performed. The obtained organic layer was concentrated under reduced pressure conditions. The obtained residue was filtered, then 2-methyltetrahydrofuran (240 mL) was added, and the mixture was concentrated under reduced pressure conditions to obtain a solution (70.63 g, 41.5 w/w%) containing Cbz-Hph(3,5-F₂-4-CF₃)-OH (29.3 g, 70.2 mmol).

To another reaction vessel, a solution of Cbz-Hph(3,5-F₂-4-CF₃)-OH (41.5 w/w%, 62.7 g, 62.3 mmol) prepared as described above, H-Pro-cLeu-MeGcp-MeAsp(OtBu)-NMe₂ (30.0 g, 51.9 mmol) synthesized in a similar manner as in Example 56, 2-methyltetrahydrofuran (167 mL), and DIPEA (39.9 mL, 228 mmol) were added. While stirring at 10°C, a solution of propylphosphonic acid anhydride in 2-methyltetrahydrofuran (1.6 M, 78 mL, 125 mmol) was added, and then the mixture was warmed to 25°C. After completion of the addition, the mixture was stirred for 1 hour, then sampling was performed, and the completion of the reaction was confirmed by HPLC analysis. While stirring at 15°C, a 5% aqueous potassium carbonate solution (180 mL) and 1-methylimidazole (4.1 mL, 51.9 mmol) were added, and then the mixture was warmed to 25°C. The mixture was stirred for 50 minutes and then allowed to stand still, and the aqueous layer was removed. The obtained organic layer was sequentially subjected to washing with a 4% aqueous sulfuric acid solution (180 mL) and a 10% aqueous sodium bisulfate monohydrate solution (180 mL), and then n-heptane (108 mL), MTBE (72 mL), and acetonitrile (69 mL) were added. After washing with a 2.5% aqueous potassium carbonate solution (171 mL), 2-methyltetrahydrofuran (60 mL) and acetonitrile (102 mL) were added, and the mixture was subjected to washing with a 2.5% aqueous potassium carbonate solution (171 mL). To the obtained organic layer, 2-methyltetrahydrofuran (60 mL) and acetonitrile (102 mL) were added, and then the mixture was subjected to washing with a 2.5% aqueous potassium carbonate solution (171 mL) and then concentrated under reduced pressure conditions. To the obtained residue, isopropyl acetate (210 mL) was added, and the mixture was concentrated under reduced pressure conditions. This procedure was repeated twice to obtain a solution containing Cbz-Hph(3,5-F₂-4-CF₃)-Pro-cLeu-MeGcp-MeAsp(OtBu)-NMe₂ (84.49 g, 60.0 w/w%).
HPLC purity: 97.90%
Measurement method: HPLC Method C
Retention time: 4.63 min
Mass spectrometry: m/z 999 ([M+Na]⁺)

### (Example 60) Synthesis of Cbz-Hph(3,5-F₂-4-CF₃)-Pro-cLeu-MeGcp-MeAsp(OH)-NMe₂ diethylamine salt

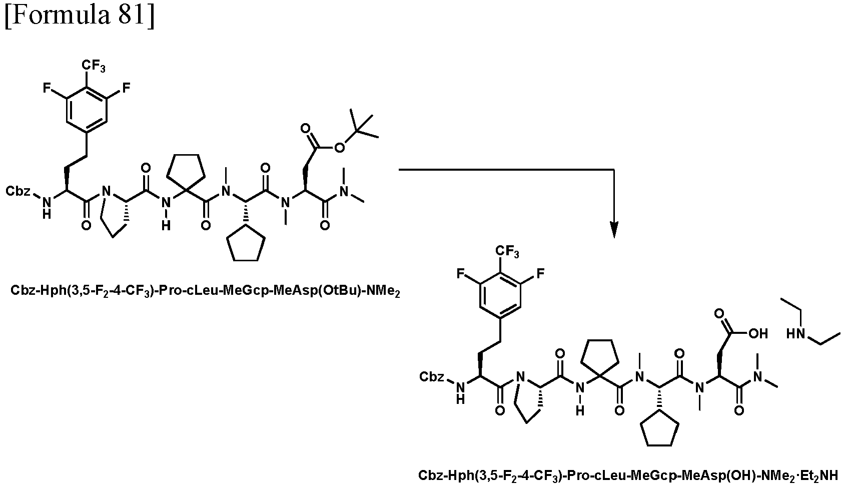

To a reaction vessel, a solution containing Cbz-Hph(3,5-F₂-4-CF₃)-Pro-cLeu-MeGcp-MeAsp(OtBu)-NMe₂ (60.0 w/w%, 42.25 g, 26.0 mmol) synthesized in Example 59, isopropyl acetate (108 mL), and 1,1,1,3,3,3-hexamethyldisilazane (13.6 mL, 65.0 mmol) were added, and then the mixture was cooled to 0°C. Trimethylsilyl trifluoromethanesulfonate (4.7 mL, 26.0 mmol) was added, and then the mixture was warmed to 20°C. After completion of the addition, the mixture was stirred for 3 hours, then sampling was performed, and the completion of the reaction was confirmed by HPLC analysis. After 2-methyltetrahydrofuran (128 mL) was added, the mixture was cooled to 0°C, then a 5% aqueous dipotassium hydrogen phosphate solution (254 mL) was added, and the mixture was warmed to 25°C. The mixture was stirred for 20 minutes and then allowed to stand still, and the aqueous layer was removed. The obtained organic layer was washed with a 5% aqueous sodium dihydrogen phosphate solution (254 mL), then diethylamine (10.8 mL, 104 mmol) was added, and the mixture was concentrated into 144 mL under reduced pressure conditions. To the obtained residue, isopropyl acetate (120 mL) and diethylamine (2.69 mL, 26.0 mmol) were added, and the mixture was concentrated into 144 mL under reduced pressure conditions. This procedure was repeated three times to prepare a slurry. Isopropyl acetate (120 mL) and diethylamine (2.69 mL, 26.0 mmol) were further added, then the mixture was concentrated into 108 mL under reduced pressure conditions, and then isopropyl acetate (38.2 mL) and diethylamine (2.18 mL, 21.1 mmol) were added while stirring at 25°C. After completion of the addition, the mixture was stirred for 4.5 hours, and then MTBE (192 mL) was added over 80 minutes. After completion of the addition, the mixture was stirred for 2 hours, and then the slurry was filtered. The obtained solid was washed twice with a mixed solution of isopropyl acetate/MTBE/diethylamine (1:5:0.06 (v/v), 72 mL) and then dried under reduced pressure conditions to obtain Cbz-Hph(3,5-F₂-4-CF₃)-Pro-cLeu-MeGcp-MeAsp(OH)-NMe₂ diethylamine salt (23.23 g, content: 92.0 w/w%).
Yield: 83% (2 steps from H-Pro-cLeu-MeGcp-MeAsp(OtBu)-NMe₂)
HPLC purity: 99.85%
Measurement method: HPLC Method C
Retention time: 4.18 min
Mass spectrometry: m/z 921 ([M+H]⁺)

### (Example 61) Synthesis of compound 11

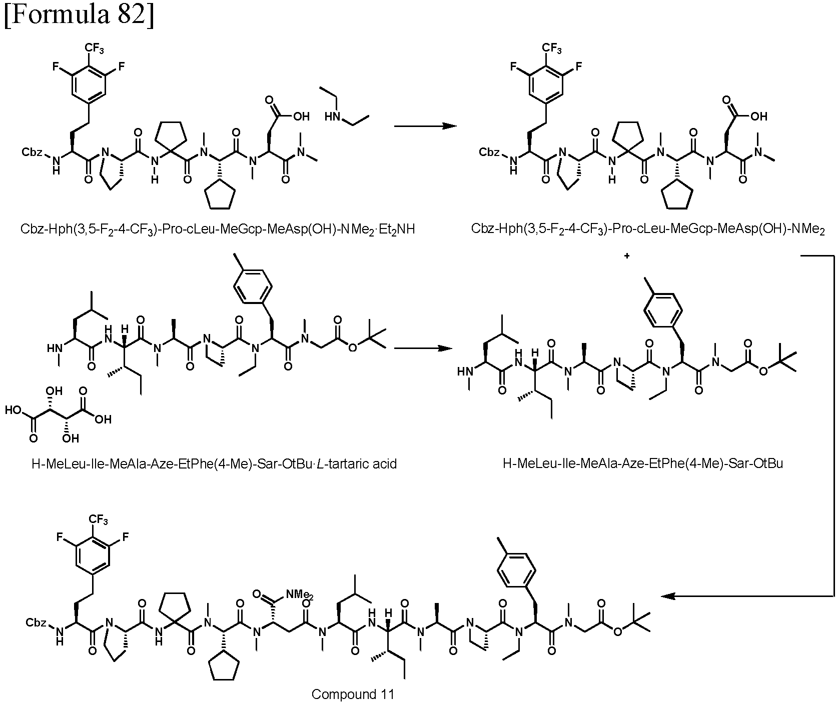

To a reaction vessel, Cbz-Hph(3,5-F₂-4-CF₃)-Pro-cLeu-MeGcp-MeAsp(OH)-NMe₂ diethylamine salt (92.0 w/w%, 20.78 g, 19.2 mmol) obtained in Example 60, acetonitrile (177 mL), dicyclohexylmethylamine (8.2 mL, 38.6 mmol), and DIPEA (10.1 mL, 58.0 mmol) were added, and the mixture was concentrated into 42.5 mL under reduced pressure conditions. To the obtained residue, acetonitrile (177 mL) and DIPEA (3.4 mL, 19.5 mmol) were added, and the mixture was concentrated into 42.5 mL under reduced pressure conditions. This procedure was repeated three times to obtain a solution (46.76 g, 37.9 w/w%) containing Cbz-Hph(3,5-F₂-4-CF₃)-Pro-cLeu-MeGcp-MeAsp(OH)-NMe₂ (17.70 g).

To another reaction vessel, H-MeLeu-Ile-MeAla-Aze-EtPhe(4-Me)-Sar-OtBu L-tartrate (93.8 w/w%, 18.66 g, 19.6 mmol) synthesized in a similar manner as in Example 47 and 2-methyltetrahydrofuran (87 mL) were added. While stirring at 25°C, a 5% aqueous sodium carbonate solution (87 mL) was added. The mixture was stirred for 10 minutes and then allowed to stand still, and the aqueous layer was removed. The obtained organic layer was subjected to washing with a 5% aqueous sodium carbonate solution (87 mL) and a 5% aqueous sodium chloride solution (87 mL) and then concentrated under reduced pressure conditions to obtain a solution (29.71 g, 49.0 w/w%) containing H-MeLeu-Ile-MeAla-Aze-EtPhe(4-Me)-Sar-OtBu (14.55 g).

To another reaction vessel, an acetonitrile solution containing Cbz-Hph(3,5-F₂-4-CF₃)-Pro-cLeu-MeGcp-MeAsp(OH)-NMe₂ (37.9 w/w%, 39.0 g, 16.0 mmol) prepared as described above, a 2-methyltetrahydrofuran solution containing H-MeLeu-Ile-MeAla-Aze-EtPhe(4-Me)-Sar-OtBu (49.0 w/w%, 26.7 g, 17.6 mmol) prepared as described above, 2-methyltetrahydrofuran (118 mL), and acetonitrile (13.5 mL) were added. After cooling to 10°C, DIPEA (5.5 mL, 31.6 mmol) and HATU (9.14 g, 24.1 mmol) were sequentially added while stirring, and the mixture was warmed to 25°C. After completion of the addition, the mixture was stirred for 3 hours, then sampling was performed, and the completion of the reaction was confirmed by HPLC analysis. 2-Methyltetrahydrofuran (44 mL) was added, then the mixture was cooled to 10°C, and then 10% ammonia water (100 mL) was added. The mixture was warmed to 25°C, stirred for 30 minutes, and then allowed to stand still, and the aqueous layer was removed. The obtained organic layer was subjected to washing with 10% ammonia water (100 mL), a 10% aqueous citric acid solution (100 mL), a 5% aqueous sodium carbonate solution (100 mL), and a 20% aqueous sodium chloride solution (100 mL) and then concentrated into 79 mL under reduced pressure conditions. To the obtained residue, 2-methyltetrahydrofuran (45 mL) was added, and the mixture was concentrated under reduced pressure conditions to obtain a solution containing compound 11 (69.84 g, content: 37.8 w/w%).
HPLC purity: 96.49%
Measurement method: HPLC Method G
Retention time: 10.27 min
Mass spectrometry: m/z 1668 ([M+Na]⁺)

### (Example 62) Synthesis of compound 12

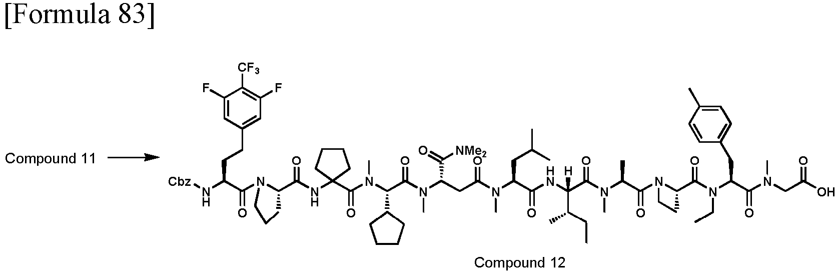

To a reaction vessel, a solution containing compound 11 (37.8 w/w%, 69.14 g, 15.9 mmol) obtained in Example 61 and 2-methyltetrahydrofuran (247 mL) were added, and then the mixture was cooled to 0°C. 1,1,1,3,3,3-Hexamethyldisilazane (22.6 mL, 108 mmol) and trimethylsilyl trifluoromethanesulfonate (12.0 mL, 66.4 mmol) were added, and then the mixture was warmed to 24°C. After completion of the addition, the mixture was stirred for 3 hours, then sampling was performed, and the completion of the reaction was confirmed by HPLC analysis. The mixture was cooled to 0°C, then a 5% aqueous dipotassium hydrogen phosphate solution (120 mL) was added, and then the mixture was warmed to 24°C. The mixture was stirred for 50 minutes and then allowed to stand still, and the aqueous layer was removed. The obtained organic layer was subjected to washing with a 5% aqueous ammonium chloride solution (131 mL, four times) and a 5% aqueous sodium carbonate solution (131 mL) and then concentrated under reduced pressure conditions to obtain a solution containing compound 12 (54.99 g, content: 45.8 w/w%).
HPLC purity: 95.41%
Measurement method: HPLC Method G
Retention time: 9.22 min
Mass spectrometry: m/z 1612 ([M+Na]⁺)

### (Example 63) Synthesis of compound 3

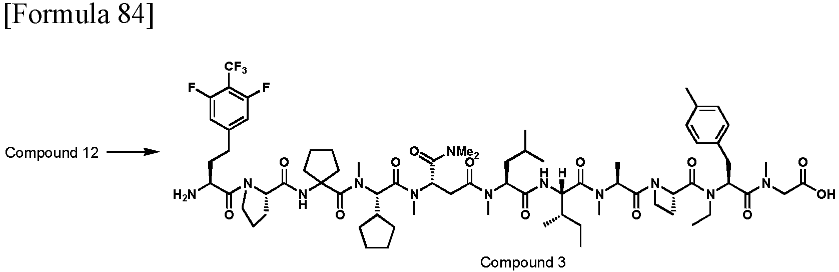

To a reaction vessel, 10% palladium carbon (54.13% wet, 3.97 g, 1.71 mmol, 11 mol% on Pd metal basis) and tetrahydrofuran (84 mL) were added. Nitrogen replacement was performed at 25°C, then hydrogen replacement was performed, and the mixture was stirred for 2 hours under the hydrogen atmosphere (0.35 MPaG). A solution containing compound 12 (45.8 w/w%, 53.95 g, 15.5 mmol) obtained in Example 62 and tetrahydrofuran (84 mL) were added. The mixture was stirred for 1 hour under the hydrogen atmosphere (0.20 MPaG), then sampling was performed, and the completion of the reaction was confirmed by HPLC analysis. After the reaction mixture was filtered, the cake was washed twice with tetrahydrofuran (57 mL). A mixed solution of the filtrate and washing solution was concentrated under reduced pressure conditions, then acetonitrile (226 mL) was added, and the mixture was concentrated into 90 mL under reduced pressure conditions. This procedure was repeated three times. After the obtained residue was filtered, tetrahydrofuran (47 mL) and toluene (110 mL) were added, and the mixture was concentrated under reduced pressure conditions. Tetrahydrofuran (224 mL) was added, and the mixture was concentrated into 134 mL under reduced pressure conditions to obtain a concentrate (120.80 g).

To a portion (107.90 g) of the obtained concentrate, tetrahydrofuran (20 mL) was added to prepare a solution containing compound 3. To another reaction vessel, n-heptane (240 mL) was added, and the solution containing compound 3 prepared in advance was added dropwise over 1 hour while stirring at 25°C. Tetrahydrofuran (10 mL) was added, then the mixture was stirred at 25°C for 1 hour, and then the slurry was filtered. The obtained solid was washed with n-heptane (100 mL) and then dried under reduced pressure conditions to obtain compound 3 (19.07 g, content: 97.06 w/w%). The content and the yield were calculated by HPLC analysis using a preparation.
Yield: 91.8% (yield by 3 steps from Cbz-Hph(3,5-F₂-4-CF₃)-Pro-cLeu-MeGcp-MeAsp(OH)-NMe₂ diethylamine salt)
HPLC purity: 97.56%
Measurement method: HPLC Method G
Retention time: 6.57 min
Mass spectrometry: m/z 1456 ([M+H]⁺)

### (Example 64) Synthesis of compound 4

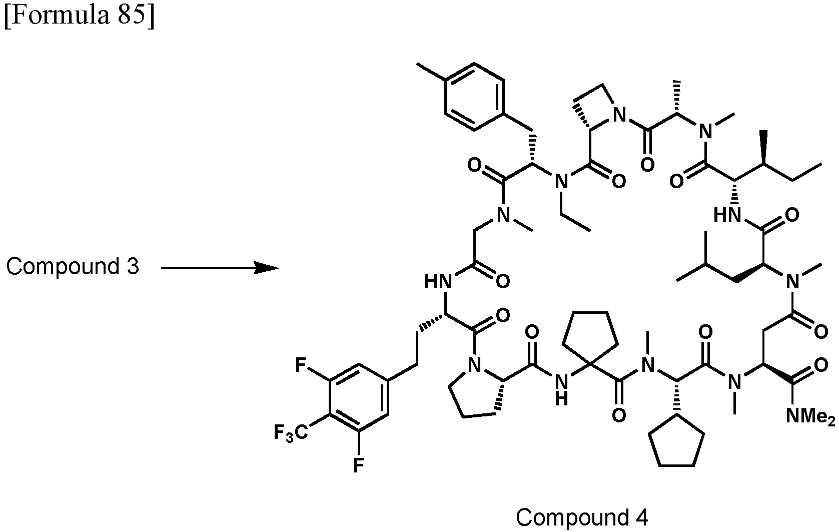

To a reaction vessel, HATU (2.36 g, 6.21 mmol) and acetonitrile (76 mL) were added. To another reaction vessel, compound 3 (9.50 g, 6.53 mmol) synthesized in a similar manner as in Example 63, DIPEA (2.62 mL, 15.0 mmol), and acetonitrile (152 mL) were added to prepare a solution, half the amount of which was added over 6 hours to the solution of HATU in acetonitrile prepared in advance, while stirring at 25°C. After completion of the addition, the mixture was allowed to stand still and stored for 13 hours, and then a solution of HATU (2.36 g, 6.21 mmol) dissolved in acetonitrile (11.8 mL), and acetonitrile (3.8 mL) were sequentially added. The remaining half of the acetonitrile solution containing compound 3 and DIPEA prepared in advance was added over 6 hours. After completion of the addition, the mixture was stirred for 30 minutes, then sampling was performed, and the completion of the reaction was confirmed by HPLC analysis. MTBE (124 mL), heptane (9.5 mL), and 2.5% ammonia water (95 g) were added. The mixture was stirred for 10 minutes and then allowed to stand still, and the aqueous layer was removed. The obtained organic layer was washed with a 4% aqueous sulfuric acid solution (133 g), a 5% aqueous dipotassium hydrogen phosphate solution (95 g), and a 0.5% aqueous sodium chloride solution (95 g, twice) and then dust was filtered, and the obtained filtrate was concentrated under reduced pressure conditions. Acetonitrile (66.5 mL) was added, and the mixture was concentrated under reduced pressure conditions. This procedure was repeated twice, then acetone (66.5 mL) was added, and the mixture was concentrated under reduced pressure conditions. This procedure was repeated six times. To the obtained residue, acetone (38 mL) was added to obtain a solution containing compound 4 (54.3 g).
HPLC purity: 90.22%
Measurement method: HPLC Method H
Retention time: 17.99 min
Mass spectrometry: m/z 1439 ([M+H]⁺)

### (Example 65) Synthesis of hydrate crystal (type C) of compound 4

To a reaction vessel, an acetone solution (33.33 g) containing compound 4 (6.00 g, 4.17 mmol) synthesized in a similar manner as in Example 64 and acetone (12.53 g) were added. The mixture was warmed to 40°C, and water (19.2 mL) was added over 10 minutes while stirring. Hydrate crystals (type C) of compound 4 (18 mg) were added to a glass vial and suspended in a mixed solution of acetone/water (5:4 (v/v), 0.24 mL), and then the suspension was added to the crystallization solution. A mixed solution of acetone/water (5:4 (v/v), 0.24 mL) was further added to the glass vial, and the obtained suspension was added to the crystallization solution. After stirring for 2 hours, water (4.8 mL) was added over 10 minutes. After further stirring for 3 hours, water (4.8 mL) was added over 10 minutes. After further stirring for 1 hour, the mixture was cooled to 25°C over 1 hour. After stirring at 25°C for 1 hour, the suspension was allowed to stand still and stored for 13 hours. After further stirring at 25°C for 2 hours, the suspension was filtered. The obtained wet powder was washed with a mixed solution of acetone (16.8 mL) and water (13.2 mL) and then washed with a mixed solution of methanol (15 mL) and water (15 mL). The obtained wet powder was further suspended in a mixed solution of methanol (15 mL) and water (15 mL) and allowed to stand still and stored for 14 hours, and then the suspension was filtered. The obtained wet powder was suspended in water (30 mL), allowed to stand still and stored for 2 hours, and then filtered. The suspension and washing with water mentioned above were carried out again, and then the obtained wet powder was dried under reduced pressure conditions to obtain hydrate crystals (type C) of compound 4 (4.97 g).
HPLC purity: 99.74 %
Measurement method: HPLC Method H
Retention time: 17.91 min
Mass spectrometry: m/z 1439 ([M+H]⁺)

### (Example 66) Crystallization of H-MeAla-Aze-EtPhe(4-Me)-Sar-OtBu

H-MeAla-Aze EtPhe(4-Me)-Sar-OtBu (14.82 g) in an amorphous state and CPME (21.7 g) were mixed to prepare CPME solution (containing 40.6 w/w%) of H-MeAla-Aze-EtPhe(4-Me)-Sar-OtBu. The prepared solution was allowed to stand at 5 °C for one day to obtain crystals of H-MeAla-Aze-EtPhe(4-Me)-Sar-OtBu.

### (Example 67) Crystallization of Cbz-Ile-MeAla-Aze-EtPhe(4-Me)-Sar-OtBu

A mixture of Cbz-Ile-MeAla-Aze-EtPhe(4-Me)-Sar-OtBu (10 mg) in an amorphous state and toluene/heptane (1:2 (v/v), 0.1 mL) was added to a vial, and the resulting mixture was shaken at room temperature for 2 days to obtain crystals of Cbz-Ile-MeAla-Aze-EtPhe(4-Me)-Sar-OtBu.

### (Example 68) Crystallization of H-MeLeu-Ile-MeAla-Aze-EtPhe (4-Me) -Sar-OtBu L-tartrate

H-MeLeu-Ile-MeAla-Aze-EtPhe(4-Me)-Sar-OtBu (600 mg, 0.808 mmol) in an amorphous state, L-tartaric acid (121 mg, 0.808 mmol) and methanol (6 mL) were added to a glass vial to prepare a solution. After 0.1 mL of the prepared solution was added to another glass vial, the solution was concentrated to dryness under reduced pressure to remove the solvent. n-Butyl acetate (0.02 mL) and glass beads were added to a vial and shaken at 25 °C for 7 days to obtain crystals of L-tartaric acid salt of H-MeLeu-Ile-MeAla-Aze-EtPhe(4-Me)-Sar-OtBu.

### (Example 69) Crystallization of Cbz-Pro-cLeu-MeGcp-MeAsp(OtBu)-NMe₂

CPME (5.19 g) was added to Cbz-Pro-cLeu-MeGcp-MeAsp(OtBu)-NMe₂ (1.94 mL) in an amorphous state, and the mixture was stirred at room temperature for 4 hours, and then the slurry was filtered. The obtained solid was washed twice with CPME (2.38 mL) and then dried under reduced pressure to obtain crystals of Cbz-Pro-cLeu-MeGcp-MeAsp(OtBu)-NMe₂.

### (Example 70) Crystallization of H-Pro-cLeu-MeGcp-MeAsp(OtBu)-NMe₂

H-Pro-cLeu-MeGcp-MeAsp(OtBu)-NMe₂ (10 mg) in an amorphous state and tetrahydrofuran (0.02 mL) were added to a vial and then shaken at room temperature for 6 days. To the resulting solution was added heptane (0.04 mL), and the mixture was further shaken at room temperature for 6 days to obtain crystals of H-Pro-cLeu-MeGcp-MeAsp(OtBu)-NMe₂.

### Industrial Applicability

According to the present invention, medicaments and intermediates containing an N-monoalkylamino acid or a peptide containing the N-monoalkylamino acid and methods for producing the same are provided.

## Claims

1. A method for producing an N-monoalkylamino acid or an ester thereof or a peptide containing the N-monoalkylamino acid or an ester thereof, the method comprising:
an alkylation step of mixing a starting amino acid or an ester thereof or a peptide containing the starting amino acid or an ester thereof, a C₁-C₆ primary alkylating agent or a substituted methyl halide, and a catalyst in a solvent in the presence of hydrogen,
wherein the alkylation step is carried out at a pressure of 1 atm or more, and produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.

2. A method for producing an N-monoalkylamino acid or an ester thereof or a peptide containing the N-monoalkylamino acid or an ester thereof, the method comprising:
an alkylation step of mixing a starting amino acid or an ester thereof or a peptide containing the starting amino acid or an ester thereof, a C₁-C₆ primary alkylating agent or a substituted methyl halide, a hydride reducing agent, and a catalyst in a solvent,
wherein the alkylation step produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.

3. The method according to claim 2, wherein the hydride reducing agent is trialkylsilane.

4. The method according to any one of claims 1 to 3, wherein the C₁-C₆ primary alkylating agent is C₁-C₅ alkyl nitrile or C₁-C₅ alkyl aldehyde.

5. The method according to any one of claims 1 to 4, wherein the substituted methyl halide is one member selected from the group consisting of methoxymethyl chloride (MOM-Cl), ethoxymethyl chloride (EOM-Cl), 2-methoxyethoxymethyl chloride (MEM-Cl), and 2-(trimethylsilyl)ethoxymethyl chloride (SEM-Cl).

6. The method according to any one of claims 1 to 5, wherein the catalyst is a heterogeneous hydrogenation catalyst containing a transition metal.

7. The method according to claim 6, wherein the heterogeneous hydrogenation catalyst is a catalyst containing a transition metal selected from the group consisting of Pd, Rh, and Pt.

8. The method according to any one of claims 1 to 7, wherein the solvent includes at least one solvent selected from the group consisting of an ether-based solvent, an alcohol-based solvent, and an ester-based solvent.

9. The method according to any one of claims 1 to 8, wherein the solvent is selected from the group consisting of an ether-based solvent, an alcohol-based solvent, an ester-based solvent, and a combination thereof.

10. A method for producing a peptide or an ester thereof, comprising the steps of:
(a) obtaining an N-monoalkylamino acid or an ester thereof or a peptide containing the N-monoalkylamino acid or an ester thereof in accordance with the method according to any one of claims 1 to 9; and
(b) optionally extending one or more amino acids or peptides to the N-monoalkylamino acid or an ester thereof or a peptide containing the N-monoalkylamino acid or an ester thereof by a bond-forming reaction to obtain a peptide or an ester thereof.

11. The method according to any one of claims 1 to 10, further comprising contacting a reaction mixture in the alkylation step with additional hydrogen.

12. The method according to any one of claims 1 to 11, wherein the amino group of the starting amino acid or an ester thereof or a peptide containing the starting amino acid or an ester thereof is attached to a protecting group that can be removed under a hydrogenolysis condition.

13. The method according to claim 12, wherein the protecting group is an arylmethyloxycarbonyl group.

14. The method according to claim 12 or 13, wherein the removal of the protecting group is carried out in the presence of an additive selected from the group consisting of p-toluenesulfonic acid, methanesulfonic acid, sodium bisulfate, triethylamine hydrochloride, and propylphosphonic acid.

15. The method according to claim 12 or 13, wherein a reaction mixture in the alkylation step further contains a base.

16. The method according to any one of claims 12 to 15, wherein the removal of the protecting group and the alkylation step are carried out in one-pot.

17. The method according to any one of claims 1 to 11, wherein
the amino group of the starting amino acid or an ester thereof or a peptide containing the starting amino acid or an ester thereof is a primary amino group, and
a reaction mixture in the alkylation step further contains a base.

18. The method according to claim 15 or 17, wherein the base is a tertiary amine.

19. A method for producing a peptide having a cyclic portion composed of at least 4 amino acids or an ester thereof, the method comprising the steps of:
obtaining an N-monoalkylamino acid or an ester thereof or a peptide containing the N-monoalkylamino acid or an ester thereof in accordance with the method according to any one of claims 1 to 18;
optionally extending one or more amino acids by a bond-forming reaction to obtain a peptide chain; and
cyclizing with a group at the C-terminus and a group at the N-terminus of the peptide chain to form the cyclic portion.

20. The method according to claim 19, wherein
the cyclic portion is composed of at least 8 amino acids, and
the peptide having the cyclic portion or an ester thereof is a peptide composed of 8 to 15 amino acids or an ester thereof.

21. The method according to claim 20, wherein the peptide having a cyclic portion is a peptide having a cyclic portion represented by the following formula: 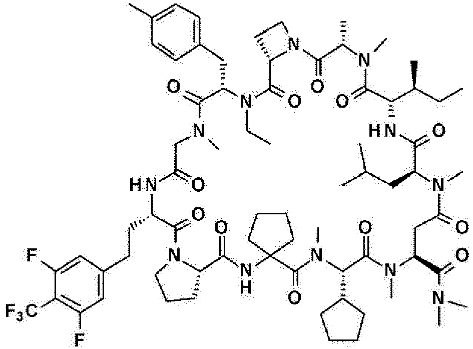 or a salt thereof or a solvate thereof.

22. The method according to claim 21, wherein the peptide having a cyclic portion or a salt thereof or a solvate thereof is a solvate of the peptide having a cyclic portion.

23. The method according to claim 22, wherein the solvate of the peptide having a cyclic portion is a hydrate of the peptide having a cyclic portion.

24. A method of suppressing a production of a dialkylated compound in an N-monoalkylation reaction of a starting amino acid or an ester thereof or a peptide containing the starting amino acid or an ester thereof, wherein the dialkylated compound is a compound in which an amino group of the starting amino acid or an ester thereof or a peptide containing the starting amino acid or an ester thereof is dialkylated, the method comprising:
an alkylation step of mixing a starting amino acid or an ester thereof or a peptide containing the starting amino acid or an ester thereof, a C₁-C₆ primary alkylating agent or a substituted methyl halide, and a catalyst in a solvent in the presence of hydrogen,
wherein the alkylation step is carried out at a pressure of 1 atm or more, and produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.

25. A method of suppressing a production of a dialkylated compound in an N-monoalkylation reaction of a starting amino acid or an ester thereof or a peptide containing the starting amino acid or an ester thereof, wherein the dialkylated compound is a compound in which an amino group of the starting amino acid or an ester thereof or a peptide containing the starting amino acid or an ester thereof is dialkylated, the method comprising:
an alkylation step of mixing a starting amino acid or an ester thereof or a peptide containing the starting amino acid or an ester thereof, a C₁-C₆ primary alkylating agent or a substituted methyl halide, a hydride reducing agent, and a catalyst in a solvent,
wherein the alkylation step produces an N-monoalkylamino acid or an ester thereof or a peptide containing the N-monoalkylamino acid or an ester thereof in which a primary alkyl group corresponding to the C₁-C₆ primary alkylating agent or the substituted methyl halide is attached to an amino group of the starting amino acid or an ester thereof.

26. The method according to claim 24 or 25, further comprising the steps of:
extending a peptide chain of an N-monoalkylamino acid or an ester thereof or a peptide containing the N-monoalkylamino acid or an ester thereof by a bond-forming reaction; and
treating the extended peptide with an aqueous acid solution to remove the dialkylated compound.
